(19) Europäisches Patentamt
European Patent Office
Office européen des brevets

(11) EP 4 447 936 B1

(12) EUROPEAN PATENT SPECIFICATION

(45) Date of publication and mention of the grant of the patent:
**19.11.2025 Bulletin 2025/47**

(21) Application number: **22840588.2**

(22) Date of filing: **16.12.2022**

(51) International Patent Classification (IPC):
***A61K 9/107*** *(2006.01)* ***A61K 47/22*** *(2006.01)*
***A61K 47/34*** *(2017.01)* ***A61K 47/55*** *(2017.01)*
***A61K 47/64*** *(2017.01)* ***A61K 47/69*** *(2017.01)*
***C12N 15/88*** *(2006.01)*

(52) Cooperative Patent Classification (CPC):
**A61K 9/1075; A61K 47/22; A61K 47/34; A61K 47/551; A61K 47/64; A61K 47/6935; C12N 15/88**

(86) International application number:
**PCT/EP2022/086403**

(87) International publication number:
**WO 2023/111290 (22.06.2023 Gazette 2023/25)**

(54) **POLYSARCOSINE-VITAMIN E DERIVATIVES**

POLYSARCOSIN-VITAMIN-E-DERIVATE

DÉRIVÉS DE POLYSARCOSINE-VITAMINE E

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.12.2021 EP 21383149**

(43) Date of publication of application:
**23.10.2024 Bulletin 2024/43**

(73) Proprietor: **Polypeptide Therapeutic Solutions, S.L.**
**46980 Paterna (ES)**

(72) Inventors:
- **MORELLÓ BOLUMAR, Daniel**
**46980 PATERNA (ES)**
- **MIRAVET MARTÍ, Rafael**
**46980 PATERNA (ES)**
- **ALONSO DE CASTRO, Silvia**
**46980 PATERNA (ES)**
- **VICENT DOCÓN, María Jesús**
**46012 VALENCIA (ES)**
- **NEBOT CARDA, Vicent Josep**
**46980 PATERNA (ES)**

(74) Representative: **ZBM Patents - Zea, Barlocci & Markvardsen**
**Rambla de Catalunya, 123**
**08008 Barcelona (ES)**

(56) References cited:
**WO-A1-2004/026856 WO-A1-2019/081455**

- **ALEXANDER BIRKE ET AL: "Polysarcosine-containing copolymers: Synthesis, characterization, self-assembly, and applications", PROGRESS IN POLYMER SCIENCE, vol. 81, 1 June 2018 (2018-06-01), GB, pages 163 - 208, XP055573544, ISSN: 0079-6700, DOI: 10.1016/j.progpolymsci.2018.01.002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 447 936 B1

## Description

This application claims the benefit of the European Patent Application 21383149.8 filed on 17.12.2021.

## Technical Field

The present invention relates to polysarcosine-vitamin E derivatives, and to conjugates comprising them wherein the polysarcosine-vitamin E derivatives are covalently attached to other moieties such as e.g., diagnostic agents or therapeutic agents. It also relates to compositions comprising the polysarcosine-vitamin E derivatives and/or their conjugates as well as to self-assembled particles formed from them which may encapsulate a variety of agents, and to their uses in medicinal, cosmetic or food applications.

## Background Art

The vitamin E family is a group of fat-soluble compounds which includes tocopherols and tocotrienols. Both tocopherols and tocotrienols include a chroman ring and a 16-carbon side chain in their structure. The difference between them lies in that while the carbon side chain of the tocopherols is saturated, the side chain of the tocotrienols is unsaturated. Each tocopherol and tocotrienol exists in four different forms: α, β, γ, δ, which differ in the number and position of methyl groups on the ring as shown below. The most common and biologically active form is α-tocopherol, which is often referred to as vitamin E.

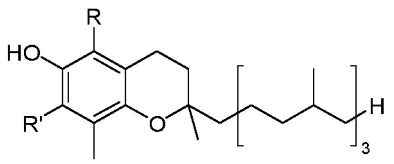

Tocopherol

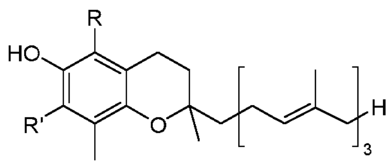

Tocotrienol

α: R, R' = $CH_3$
β: R = $CH_3$; R' = H
γ: R' = $CH_3$; R = H
δ: R, R' = H

Vitamin E is an essential food ingredient. It has been identified as an essential factor for reproduction. It has antioxidant, anti-thrombolytic and other therapeutic effects. The antioxidant properties of vitamin E derivatives are linked with the 6-hydroxy-chroman ring of their chemical structure.

Low blood levels of Vitamin E are associated with increased risk of development of degenerative diseases.

This has prompted researchers to study the ability of vitamin E to prevent chronic diseases related to oxidative stress, including cardiovascular and neurodegenerative diseases, atherosclerosis, and cancer. Further, the involvement of vitamin E in cell signaling and gene expression has also been reported.

Additionally, there is an emerging interest in the use of vitamin E derivatives as drug delivery systems to increase cell permeability and access to the desired targets, as well as for medical imaging as contrast agents or fluorescent probes.

Vitamin E tends to be an unstable molecule. Besides, its poor water solubility has greatly limited its

application. In order to improve its shelf life, vitamin E has been supplied in the form of derivatives in which the 6-hydroxy moiety attached to the chroman ring is esterified with an acetate or a succinate moiety to render vitamin E acetate or vitamin E succinate derivatives.

α-tocopherylsuccinate was the first reported vitamin E derivative for which activity against growth inhibition of cancer cells was demonstrated. However, this derivative is highly hydrophobic and toxic. Efforts to overcome the poor water solubility of vitamin E led to the preparation of tocopheryl polyethylene glycol succinate (TPGS) also called tocophersolan, which is obtained by esterification of vitamin E succinate with poly(ethylene glycol) (PEG). TPGS has the following chemical structure:

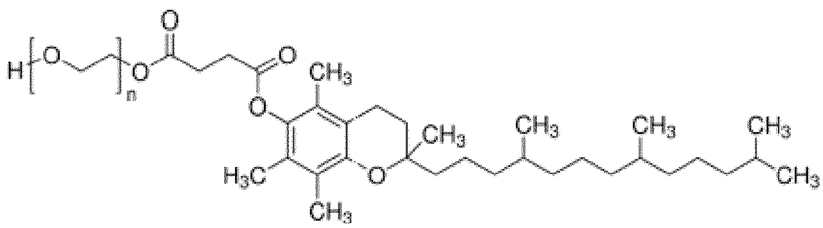

TPGS has been approved by the US FDA as a nutritional supplement and drug delivery vehicle. It can be used as solubilizer, absorption and permeation enhancer, emulsifier as well as surface stabilizer in drug delivery. TPGS may act as

anticancer agent against many cancer types. In this regard, conjugates of TPGS with e.g., doxorubicin, paclitaxel or cisplatin have been described. Additionally, TPGS-based polymers can improve the drug encapsulation efficiency, intracellular uptake, and therapeutic effect.

Despite the above, TPGS has some drawbacks associated to the inclusion of PEG in its structure. PEG is widely used in drug delivery and nanotechnology because of its stealth properties and biocompatibility, which allows particulate delivery systems and biomaterials to evade the immune system, thereby having a prolonged circulation time within the biological system. However, it has been reported that PEG may cause an unexpected immunogenic response known as the accelerated blood clearance (ABC) phenomenon, resulting in the increased clearance and reduced efficacy of PEG-conjugated substances. Furthermore, PEG conjugates show poor degradability. Birke et al. (Progress in Polymer Science 81 (2018) 163-208) disclose polysarcosine-containing co-polymers and their potential in drug delivery.

Therefore, there is still the need to develop alternative vitamin E derivatives which overcome the problems of the prior art.

**Summary of Invention**

The inventors have developed new vitamin E derivatives based on polysarcosine (PSar), which, unlike PEG-containing derivatives such as TPGS, are biodegradable, non-immunogenic, and show excellent shelf-life. The polysarcosine-vitamin E derivatives of the invention are amphiphilic. They comprise a hydrophilic polar head portion, the PSar-based moiety, and a hydrophobic part, the vitamin E-based moiety. As hybrid compounds, they have the properties of both components. Thus, on the one hand, they show excellent

solubility in water and organic solvents thanks to the PSar-based moiety, and, on the other hand, they have antioxidant properties due to the Vitamin E-based moiety.

As illustrated in the examples below, the polysarcosine-vitamin E derivatives of the invention may form self-assembled particles in solvents, such as micelles, and encapsulate molecules such as active agents or imaging contrast agents. The polysarcosine-vitamin E derivatives showed higher accumulation of the delivered agent in the epidermis layer in comparison to analogue TPGS, which demonstrates that PSar-based materials may be advantageous over TPGS for skin delivery applications, while avoiding the disadvantages associated with PEG.

Therefore, a first aspect of the invention relates to a polymer comprising a repeating sarcosine-based unit which is covalently attached directly or through a linker to a vitamin E-based moiety, or a pharmaceutically, nutraceutically, or cosmetically acceptable salt thereof, or any stereoisomer or mixtures of stereoisomers, either of the polymer or of any of its salts; wherein the repeating sarcosine-based unit has the formula (XXIII)

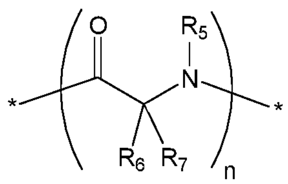

(XXIII)

wherein $R_5$ is selected from the group consisting of methyl, $-CH_2F$, $-CHF_2$, and $-CF_3$; $R_6$ and $R_7$ are independently selected from the group consisting of hydrogen and fluorine; and n is an integer from 5 to 500; and the vitamin E-based moiety has the formula (XXIV):

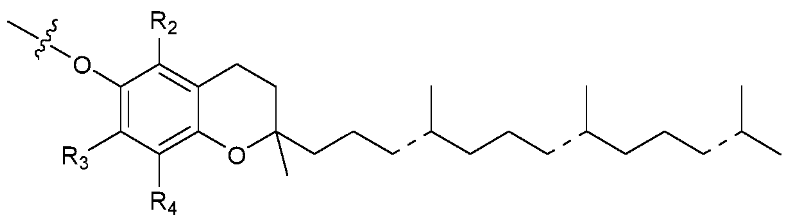

(XXIV)

wherein $R_2$, $R_3$ and $R_4$ are independently selected from the group consisting of hydrogen, fluorine, methyl, $-CH_2F$, $-CHF_2$, and $-CF_3$; and each of the dashed bonds ----- is independently a single bond or, alternatively, a double bond. In the polymer of the invention the repeating sarcosine-based unit is covalently attached directly or through a linker to the vitamin E-based

moiety by any of the attachment points indicated herein with an "*". The vitamin E-based moiety is covalently attached directly or through a linker to the repeating sarcosine-based unit by the attachment point indicated herein with a wavy line " " that intersects a bond.

More particularly, the polymer comprising a repeating sarcosine-based unit which is covalently attached directly or through a linker to a vitamin E-based moiety is a polymer of formula (I),

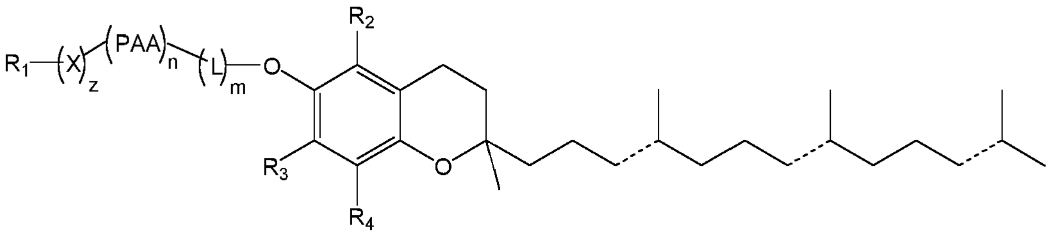

(I)

wherein:

PAA is a repeating sarcosine-based unit selected from formula (XXI) and formula (XXII):

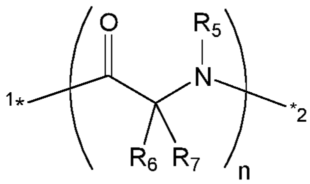

(XXI)

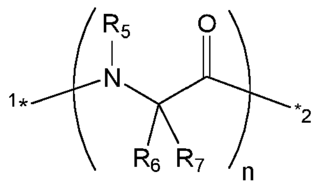

(XXII)

wherein "*1" denotes the attaching point of the repeating sarcosine-based unit PAA to X or $R_1$ (when z is 0), and "*2" denotes the attaching point of the repeating sarcosine-based unit PAA to the linker L or the oxygen atom of the vitamin E-based moiety (when m is 0);

m is 0 or 1, with the condition that when the repeating sarcosine-based unit PAA has the formula (XXI), then m is 1;

z is 0 or 1, with the condition that when the repeating sarcosine-based unit PAA has the formula (XXI), then z is 1, and when the repeating sarcosine-based unit PAA has the formula (XXII), then z is 0;

$R_1$ is selected from the group consisting of $-(C_1-C_{12})$alkyl, $-(C_1-C_{12})$alkylphenyl, $-(C_1-C_6)$alkyl-O-$(C_1-C_6)$alkyl, -CO-$(C_1-C_{12})$alkyl, -CO-$(C_1-C_{12})$alkylphenyl, and -CO-$(C_1-C_6)$alkyl-O-$(C_1-C_6)$alkyl; wherein $R_1$ is optionally substituted with one or more substituents selected from the group consisting of halogen, -OH, $-NR_aR_b$, -SH, $-NHNH_2$, $-COOR_c$, $-CF_3$, and $-OCF_3$;

$R_2$, $R_3$ and $R_4$ are independently selected from the group consisting of hydrogen, fluorine, methyl, $-CH_2F$, $-CHF_2$, and $-CF_3$;

$R_5$ is selected from the group consisting of methyl, $-CH_2F$, $-CHF_2$, and $-CF_3$;

$R_6$ and $R_7$ are independently selected from the group consisting of hydrogen and fluorine;

n is an integer from 5 to 500;

X is -O- or -NH-; and

L is a biradical chain which comprises one or more moieties selected from the group consisting of -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -COO-, $-C(=CH_2)-$, -C(=NH)-, -CONH-, -NHCO-, -NH(CO)NH-, -S-, -S-S-, -SO-, $-SO_2-$, $-SO_2NH_2-$ and -phenylene-, wherein L is

optionally substituted with one or more substituents selected from the group consisting of halogen, -OH, $-NR_aR_b$, -SH, $-NHNH_2$, $-COOR_c$, $-CF_3$, $-OCF_3$,

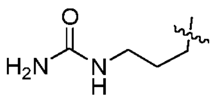 ; 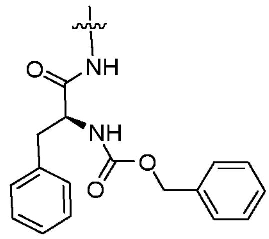 ; 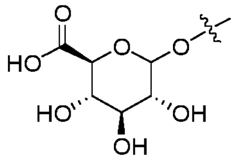

wherein when L comprises a $-CH_2-$ moiety, the two hydrogen atoms attached to the carbon atom are optionally replaced by the ring:

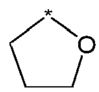

when PAA is a repeating sarcosine-based unit of formula (XXI), L is attached to the nitrogen atom of the PAA by a chemically feasible bond which is selected from the group consisting of an amide bond, a carbamate bond, a urea bond, and an amine bond, and when PAA is a repeating sarcosine-based unit of formula (XXII) and m is 1, L is attached to the carbonyl group of the PAA by a chemically feasible bond which is selected from the group consisting of an amide bond and an ester bond;

L is attached to the oxygen atom of the vitamin E-based moiety by a chemically feasible bond which is selected from the group consisting of an ester bond, a carbamate bond and an ether bond; and

each of the dashed bonds - - - - - is independently a single bond or, alternatively, a double bond; and

$R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl.

The polysarcosine-vitamin E derivatives of the invention may form covalent conjugates with other molecules. Thus, another aspect of the invention relates to a conjugate of formula (II), or a pharmaceutically, nutraceutically, or cosmetically acceptable salt thereof, or any stereoisomer or mixtures of stereoisomers, either of the conjugate of formula (II) or of any of its salts

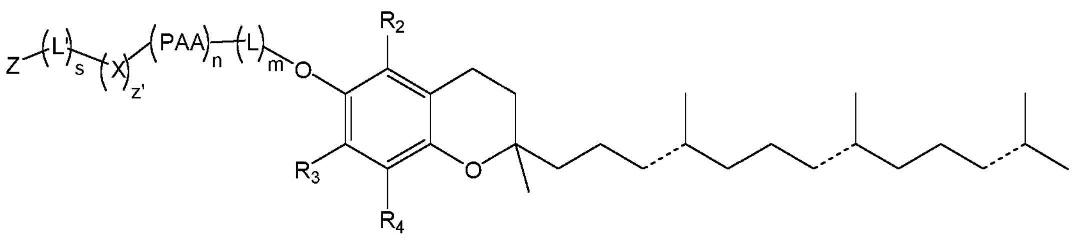

(II)

wherein:

m, PAA, X, n, L, $R_2$, $R_3$, $R_4$, and the dashed bonds are as defined herein;

s is 0 or 1;

z' is 0 or 1, with the condition that when the repeating sarcosine-based unit PAA has the formula (XXII), then z' is 0;

Z is an agent selected from the group consisting of a nutraceutically active agent, a pharmaceutically active agent, a

cell-targeting agent, a diagnostically active agent, and a cosmetically active agent;

L' is a biradical chain which comprises one or more moieties selected from the group consisting of -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -COO-, $-C(=CH_2)-$, -C(=NH)-, -CONH-, -NHCO-, -NH(CO)NH-, -S-, -S-S-, -SO-, $-SO_2-$, $-SO_2NH_2-$ and -phenylene-, wherein L' is optionally substituted with one or more substituents selected from the group consisting of halogen, -OH, $-NR_aR_b$, -SH, $-NHNH_2$, $-COOR_c$, $-CF_3$, $-OCF_3$,

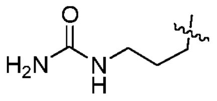 ; 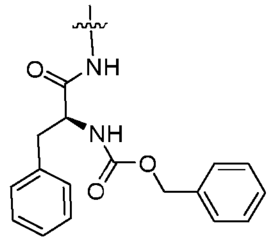 ; 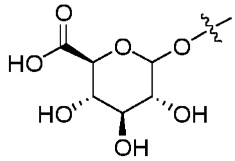

and $R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl; and

wherein when L' comprises a $-CH_2-$ moiety, the two hydrogen atoms attached to the carbon atom are optionally replaced by the ring:

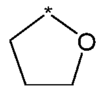

when s is 0, Z is directly attached to X or the PAA sarcosine repeating unit by a chemically feasible bond which is selected from the group consisting of an amide bond, a carbamate bond, an amine bond, a urea bond, an ether bond, and an ester bond;

when s is 1 L' is attached to Z by a chemically feasible bond which is selected from the group consisting of an amide bond, a carbamate bond, an amine bond, a urea bond, an ether bond, and an ester bond, and L' is attached to X or the PAA sarcosine repeating unit by a chemically feasible bond which is selected from the group consisting of an amide bond, a carbamate bond, an amine bond, a urea bond, an ether bond, and an ester bond.

As mentioned above, thanks to their amphiphilic nature, the polysarcosine-vitamin E derivatives of the invention may form self-assembled particles in solution such as for example micelles or lipid nanoparticles which may be used to encapsulate and deliver molecules of different nature. Accordingly, another aspect of the invention relates to a self-assembled particle comprising:

(a) the polymer as defined herein; or alternatively
(b) the conjugate of formula (II) as defined herein; or alternatively
(c) a combination of the polymer as defined herein and the conjugate of formula (II),

and optionally an agent encapsulated within the self-assembled particle which is selected from the group consisting of a nutraceutically active agent, a pharmaceutically active agent, a cell-targeting agent, a diagnostically active agent, and a cosmetically active agent.

The polysarcosine-vitamin E derivatives of the invention as well as the conjugates of formula (II) as defined herein may be formulated in a variety of compositions such as nutraceutical, pharmaceutical, diagnostic, or cosmetic compositions. Thus, another aspect of the invention relates to a composition comprising an effective amount of:

(a) the polymer as defined herein, or alternatively,
(b) the conjugate of formula (II) as defined herein, or alternatively,
(c) a combination of the polymer as defined herein and the conjugate of formula (II), or alternatively,
(d) the self-assembled particles as defined herein,

together with one or more acceptable excipients or carriers.

The polysarcosine-vitamin E derivatives of the invention, their conjugates, self-assembled particles, and compositions thereof may be used in medicinal, cosmetic or food applications. Thus, another aspect of the invention relates to

(i) the polymer as defined herein, or alternatively,
(ii) the conjugate of formula (II) as defined herein, or alternatively,
(iii) the self-assembled particles as defined herein, or alternatively,
(iv) the pharmaceutical composition as defined herein,

for use in medicine, wherein the encapsulated agent in the self-assembled particles, when present, is a pharmaceutically active agent or a cell-targeting agent as defined herein, and in the conjugate of formula (II), when present, Z is a pharmaceutically active agent or a cell-targeting agent.

Another aspect of the invention relates to

(i) the conjugate of formula (II) as defined herein, or alternatively,
(ii) the self-assembled particle as defined herein, with the condition that when the self-assembled particle comprises the polymer as defined herein in the absence of a conjugate of formula (II), an encapsulated agent is present within the self-assembled particle; or alternatively,
(iii) the diagnostic composition as defined herein;

for use in diagnostics, wherein the encapsulated agent in the self-assembled particle, when present, is a diagnostically active agent, and in the conjugate of formula (II), when present, Z is a diagnostically active agent.

According to another aspect, the invention relates to the use of the polymer as defined herein as a carrier, as a solubilizer, as absorption and permeation enhancer, as emulsifier or as surface stabilizer.

**Brief Description of Drawings**

FIG. 1 shows the cell viability by MTS assay of Vit E (dotted line), TPGS (dashed line), and TPSS (solid line) in HaCaT cells (A) in Fibroblast cells (B) after 72 hours of treatment (n = 3).

FIG. 2 shows Dil fluorescence intensity quantification (pixels) by ImageJ of confocal microscopy images of Dil-labelled micelles of TPGS and TPSS at 10 mg/mL after 8h of permeation using Franz Diffusion Cells. The original magnification displayed in each image was 40x. The asterisks indicate statistically significant differences after ANOVA analyses followed Bonferroni's post hoc tests. Mean ± SEM.

**Detailed description of the invention**

All terms as used herein in this application, unless otherwise stated, shall be understood in their ordinary meaning as known in the art. Other more specific definitions for certain terms as used in the present application are as set forth below and are intended to apply throughout the description and claims.

The term "about" or "around" as used herein refers to a range of values ± 10% of a specified value. For example, the expression "about 10" or "around 10" includes ± 10% of 10, i.e., from 9 to 11.

As used herein, the indefinite articles "a" and "an" are synonymous with "at least one" or "one or more." Unless indicated otherwise, definite articles used herein, such as "the" also include the plural of the noun.

The term $(C_1\text{-}C_x)$alkyl refers to a saturated linear or branched hydrocarbon chain which contains from 1 to x carbon atoms and only single bonds. Examples of alkyl groups may include without limitation methyl, ethyl, n-propyl, isopropyl, n-butyl, sec-butyl, tert-butyl, pentyl, neopentyl, n-hexyl, decyl and undecyl. The term $(C_2\text{-}C_x)$alkenyl refers to an unsaturated branched or linear hydrocarbon chain which comprises from 2 to x carbon atoms and at least one or more double bonds. Examples of alkenyl groups may include without limitation ethenyl (i.e. vinyl), allyl, propenyl, butenyl, pentenyl and hexenyl.

The expression "biradical chain which comprises one or more moieties" as defined herein is intended to encompass the biradicals as defined herein. The biradicals may be optionally substituted as indicated along the document.

The term "$-(C_1\text{-}C_6)$alkyl-O-$(C_1\text{-}C_6)$alkyl" refers to an alkyl chain as defined above wherein one of the hydrogen atoms has been substituted by a -O-$(C_1\text{-}C_6)$alkyl moiety, i.e., al alkoxy group. Examples of this radical include, without limitation, 2-methoxyethyl, 3-methoxy propyl, 4-methoxy butyl, 2-ethoxyethyl, 3-ethoxy propyl, and 4-ethoxy butyl.

The term "$-(C_1\text{-}C_{12})$alkylphenyl" refers to an alkyl chain as defined above wherein one of the hydrogen atoms has

been substituted by a phenyl moiety. Examples of this radical include, without limitation, benzyl, 2-phenetyl, and 3-phenylpropyl.

The term "-phenyl($C_1$-$C_6$)alkyl" refers to an phenyl radical wherein one of the hydrogen atoms has been substituted by an alkyl group as previously defined. Examples of this radical include, without limitation, (2-methyl)phenyl, (4-methyl)phenyl, and (3-ethyl)phenyl.

The terms "alkylene" or "phenylene" refer to bivalent radicals.

The term "($C_6$-$C_{12}$)aryl" refers to an aromatic carbocyclic mono- or bicyclic ring system comprising 6 to 12 carbon ring atoms. Examples of aryl moieties include, without limitation, phenyl and naphthyl.

The term "halogen" or "halo" as used herein means fluorine, chlorine, bromine, and iodine, preferably fluorine, chlorine, and bromine, more preferably fluorine and chlorine.

The expression "substituted with one or more" means that a group can be substituted with one or more, preferably with 1, 2, 3, 4, 5 or 6 substituents, provided that this group has enough positions susceptible of being substituted.

The term "chemically feasible" as used herein refers to the connectivity of atoms that satisfies the chemical valency of each atom and is expected to be stable by a skilled person.

As used herein, a wavy line "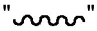" that intersects a bond in any chemical structure disclosed herein and the "*" represent the points of attachment to the remainder of the molecule. The wavy lines and the asterisks are used interchangeably.

Polymers comprising sarcosine and vitamin E-based moieties

A first aspect of the invention refers to a polymer comprising a repeating sarcosine-based unit of formula (XXIII) which is covalently attached directly or through a linker to a vitamin E-based moiety of formula (XXIV), or a pharmaceutically, nutraceutically, or cosmetically acceptable salt thereof, or any stereoisomer or mixtures of stereoisomers, either of the polymer or of any of its salts.

In the polymers of the invention the repeating polysarcosine-based unit may be placed in any order. Thus, the repeating sarcosine-based unit of formula (XXIII) may be attached directly or through a linker to the vitamin E-based moiety either by the carbonyl group or by the amino group.

This different orientation of the repeating polysarcosine-based moiety does not influence the amphiphilic behaviour of the polymer described herein, and consequently the activity displayed is maintained.

There is no limitation on the type of salt of the polymers of the invention that can be used, provided that these are pharmaceutically, nutraceutically, or cosmetically acceptable when they are used for pharmaceutically, nutraceutical or cosmetic purposes, respectively. The term "pharmaceutically, nutraceutically, or cosmetically acceptable salts", embraces non-toxic salts commonly used. The preparation of pharmaceutically, nutraceutically, or cosmetically acceptable salts of the polymers of the invention can be carried out by methods well-known in the art. Generally, such salts can be prepared by reacting the free acid or base form of a polymer of the invention with a stoichiometric amount of an appropriate base or acid, respectively, in a suitable solvent such as water, an organic solvent or a mixture of them. The polymers of the invention and their salts may differ in some physical properties, but they are equivalent for the purposes of the present invention.

Some polymers of the invention can have chiral centres that can give rise to various stereoisomers. As used herein, the term "stereoisomer" refers to all isomers of individual polymers that differ only in the orientation of their atoms in space. The term stereoisomer includes mirror image isomers (enantiomers), mixtures of mirror image isomers (racemates, racemic mixtures), geometric (cis/trans or syn/anti or E/Z) isomers, and isomers of polymers with more than one chiral center that are not mirror images of one another (diastereoisomers). The present invention relates to each of these stereoisomers and also mixtures thereof. Diastereoisomers and enantiomers can be separated by conventional techniques such as chromatography or fractional crystallization. Optical isomers may be obtained using enantiospecific synthesis. Alternatively, optical isomers can be resolved by conventional techniques to give optically pure isomers. The resolution can be carried out on any chiral synthetic intermediates or on the polymers of the invention.

In all embodiments of the invention referring to the polymers comprising a repeating sarcosine-based unit and a vitamin E-based moiety, the pharmaceutically, nutraceutically, or cosmetically acceptable salts thereof and the stereoisomers or mixtures of stereoisomers, either of any of the polymers or of any of their

pharmaceutically, nutraceutically, or cosmetically acceptable salts are always contemplated even if they are not specifically mentioned.

The polymers of the invention may be in crystalline form either as free solvation polymers or as solvates (e.g., hydrates). It is intended that all these forms are within the scope of the present invention. Methods of solvation are generally known within the art. For the purposes of the invention, the solvated forms with pharmaceutically, nutraceutically, or cosmetically acceptable solvents such as water, ethanol and the like are equivalent to the unsolvated form.

Except as otherwise specified, the polymers of the present disclosure also include polymers that differ only in the presence of one or more isotope-enriched atoms. Examples of isotope-enriched atoms, without limitation, are deuterium,

tritium, $^{13}C$ or $^{14}C$, or a nitrogen atom enriched in $^{15}N$ or a fluorine atom enriched in $^{18}F$.

The polymers of the invention are structures containing a polysarcosine-based moiety as a repeating unit. These polymers are characterized by a molecular weight (which can be average molecular weight (MW) or number average molecular weight (Mn)), a degree of polymerization, and a polydispersity index. The molecular weight can be measured by methods well-known in the art such as size exclusion chromatography (SEC) (also referred to as gel permeation chromatography (GPC)) matrix assisted laser desorption ionization-time-of-flight mass spectrometry (MALDI-TOF MS) or $^{1}H$-NMR spectroscopy, for example. Some of these methods are indicated in more detail in the examples below.

As used herein, the term "repeating unit", refers to the monomeric sarcosine-based unit and is defined by brackets depicted around it. The number on the lower right of the brackets (represented by letter "n") refers to the degree of polymerization (DP) for each monomeric unit as a statistical number. The DP is calculated by dividing the molecular weight of the polymer given by SEC-MALS technique by the molecular weight of the monomer unit. The DP value is reported as the central value of a gaussian distribution which comprises polymers of variable DP (depending on intrinsic polydispersity). The DP value is subject to a reasonable uncertainty, due to the ring-opening polymerization mechanism, which, in the context of the present invention, may be considered within the range $\pm$20%, preferably $\pm$15%, more preferably $\pm$10%, even more preferably $\pm$5%, being particularly preferred $\pm$2%.

The term "polydispersity index" (PDI) is used as a measure of broadness of molecular weight distribution. The larger the PDI, the broader the molecular weight. PDI of a polymer is calculated as the ratio of weight average (MW) by number average (Mn) molecular weight.

$$\text{Polydispersity index} = \frac{Mw}{Mn}$$

In one embodiment, optionally in combination with one or more features of the various embodiments described above or below, the polymer of the invention comprising a repeating sarcosine-based unit and a vitamin E-based moiety has the formula (I),

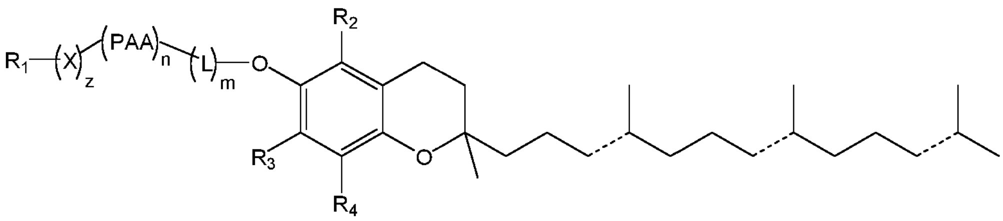

(I)

wherein $R_1$-$R_4$, X, PAA, L, m, n and z are as defined herein.

The polymers of formula (I) include a polysarcosine-based moiety PAA (drawn in the left part of the formula) and a vitamin E-based moiety (drawn in the right part of the formula) which are covalently bonded directly or through a linker L.

In one embodiment, optionally in combination with one or more features of the various embodiments described above or below, in the polymer comprising a repeating sarcosine-based unit and a vitamin E-based moiety, and in particular in the polymer of formula (I), the degree of polymerization of the polymer, or n, is from 5 to 400, or from 5 to 300, or from 5 to 200, or from 5 to 100, or from 5 to 50, or from 5 to 40, or from 5 to 35, or from 5 to 25, and more particularly is about 6, or 15, or 20, in particular measured by SEC or $^{1}H$-NMR spectroscopy as indicated in the examples.

According to another embodiment, optionally in combination with one or more features of the various embodiments described above or below, the average molecular weight (Mw) of the polymer comprising a repeating sarcosine-based unit and a vitamin E-based moiety, and in particular of the polymer of formula (I), is from 700 to 50000 Da, more particularly from 1000 to 20000 Da, and even more particularly from 1000 to 5000 Da or from 1000 to 3000 Da, measured by SEC as disclosed in the examples.

In another embodiment, optionally in combination with one or more features of the various embodiments described above or below, the polydispersity index of the polymer comprising a repeating sarcosine-based unit and a vitamin E-based moiety, and in particular of the polymer of formula (I), is from 1.01 to 1.80, or from 1.01 to 1.50, or from 1.01 to 1.30, or from 1.01 to 1.20, or from 1.01 to 1.10 measured by SEC as disclosed in the examples.

In the polymer comprising a repeating sarcosine-based unit and a vitamin E-based moiety, and in particular in the polymer of formula (I), $R_5$ is selected from the group consisting of methyl, $-CH_2F$, $-CHF_2$, and $-CF_3$. According to one embodiment, optionally in combination with one or more features of the various embodiments described above or below,

$R_5$ is methyl.

In the polymer comprising a repeating sarcosine-based unit and a vitamin E-based moiety, and in particular in the polymer of formula (I), $R_6$ and $R_7$ are independently selected from the group consisting of hydrogen and fluorine. According to one embodiment, optionally in combination with one or more features of the various embodiments described above or below, $R_6$ and $R_7$ are hydrogen.

In the polymer comprising a repeating sarcosine-based unit and a vitamin E-based moiety, and in particular in the polymer of formula (I), $R_2$, $R_3$ and $R_4$ are independently selected from the group consisting of hydrogen, fluorine, methyl, $-CH_2F$, $-CHF_2$, and $-CF_3$. According to one embodiment, optionally in combination with one or more features of the various embodiments described above or below, $R_2$, $R_3$ and $R_4$ are independently selected from the group consisting of hydrogen and methyl.

According to one embodiment, the polymer comprising a repeating sarcosine-based unit and a vitamin E-based moiety, and in particular in the polymer of formula (I), includes a vitamin E part which may encompass any of the 8 possible different forms of vitamin E (α, β, γ, δ tocopherols and α, β, γ, δ tocotrienols).

Thus, in one embodiment, optionally in combination with one or more features of the various embodiments described above or below, in the polymer of the invention, and in particular in the polymer of formula (I), each of the dashed bonds of the alkyl chain attached to the chroman ring is a single bond, and $R_2$, $R_3$ and $R_4$ are selected from the following meanings: (i) $R_2$, $R_3$ and $R_4$ are methyl, (ii) $R_2$, $R_4$ are methyl and $R_3$ is hydrogen, (iii) $R_3$ and $R_4$ are methyl and $R_2$ is hydrogen, and (iv) $R_4$ is methyl and $R_2$ and $R_3$ are hydrogen. In a more particular embodiment, each of the dashed bonds of the alkyl chain attached to the chroman ring is a single bond and $R_2$, $R_3$ and $R_4$ are methyl. Even more particularly, the vitamin E-based moiety of the polymer of the invention, and in particular in the polymer of formula (I), has the formula (XXIV'):

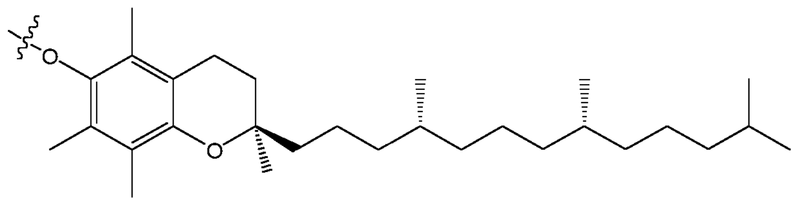

(XXIV')

In another embodiment, optionally in combination with one or more features of the various embodiments described above or below, in the polymer of formula (I), each of the dashed bonds of the alkyl chain attached to the chroman ring is a double bond, and $R_2$, $R_3$ and $R_4$ are selected from the following meanings: (i) $R_2$, $R_3$ and $R_4$ are methyl, (ii) $R_2$, $R_4$ are methyl and $R_3$ is hydrogen, (iii) $R_3$ and $R_4$ are methyl and $R_2$ is hydrogen, and (iv) $R_4$ is methyl and $R_2$ and $R_3$ are hydrogen. In a more particular embodiment, each of the dashed bonds of the alkyl chain attached to the chroman ring is a double bond and $R_2$, $R_3$ and $R_4$ are methyl.

In the polymer of formula (I), $R_1$ is selected from the group consisting of $-(C_1-C_{12})$alkyl, $-(C_1-C_{12})$alkylphenyl, $-(C_1-C_6)$alkyl-O-$(C_1-C_6)$alkyl, -CO-$(C_1-C_{12})$alkyl, -CO-$(C_1-C_{12})$alkylphenyl, and -CO-$(C_1-C_6)$alkyl-O-$(C_1-C_6)$alkyl; wherein the alkyl groups may be optionally substituted as defined herein. More particularly, when PAA is a repeating sarcosine-based unit of formula (XXI), and z is 1, $R_1$ is selected from the group consisting of $-(C_1-C_{12})$alkyl, $-(C_1-C_{12})$alkylphenyl, and $-(C_1-C_6)$alkyl-O-$(C_1-C_6)$alkyl; and when PAA is a repeating sarcosine-based unit of formula (XXII), and z is 0, $R_1$ is selected from the group consisting of -CO-$(C_1-C_{12})$alkyl, -CO-$(C_1-C_{12})$alkylphenyl, and -CO-$(C_1-C_6)$alkyl-O-$(C_1-C_6)$alkyl; wherein all the alkyl groups may be optionally substituted as defined herein.

In one embodiment, optionally in combination with one or more features of the various embodiments described above or below, in the polymer of formula (I), particularly when PAA is a repeating sarcosine-based unit of formula (XXI), and z is 1, $R_1$ is selected from the group consisting of $-(C_1-C_6)$alkyl, $-(C_1-C_3)$alkyl-O-$(C_1-C_3)$alkyl, and $-(C_1-C_6)$alkylphenyl; and wherein the alkyl groups may be optionally substituted as defined herein. More particularly, $R_1$ is selected from the group consisting of isopropyl, 2-methoxyethyl, n-propyl, n-butyl, neopentyl, benzyl, and 2-(2-methoxyethoxy)ethyl, and even more particularly $R_1$ is isopropyl.

In another embodiment, optionally in combination with one or more features of the various embodiments described above or below, in the polymer of formula (I), particularly when PAA is a repeating sarcosine-based unit of formula (XXII), and z is 0, $R_1$ is selected from the group consisting of -CO-$(C_1-C_6)$alkyl, -CO-$(C_1-C_6)$alkyl-O-$C_1-C_3)$alkyl, and -CO-$(C_1-C_6)$alkylphenyl; and wherein the alkyl groups may be optionally substituted as defined herein. More particularly, $R_1$ is acetyl.

In the polymers of the invention the repeating polysarcosine-based unit may be placed in any order. Thus, in the polymers of formula (I), the repeating sarcosine-based unit PAA is selected from formula (XXI) and formula (XXII):

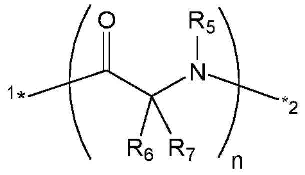

(XXI)

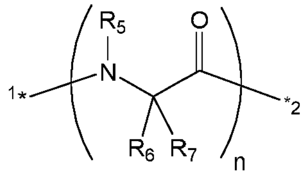

(XXII)

wherein $R_5$, $R_6$, $R_7$ and n are as defined herein, "*1" denotes the attaching point of the repeating sarcosine-based unit PAA to X or $R_1$, and "*2" denotes the attaching point of the repeating sarcosine-based unit PAA to the linker L or the oxygen atom of the vitamin E-based moiety.

This different orientation of the repeating polysarcosine-based moiety does not influence the amphiphilic behaviour of the polymer described herein, and consequently the activity displayed is maintained.

In the polymers of formula (I), the linker L is a biradical chain which comprises one or more moieties selected from the group consisting of -CH=CH-, -C≡C-, $-CH_2-$, -N=CH-, -CH=NH-, -NH-, -NH-NH-, -NH-N=CH-, -O-, $-O(CH_2)O-$, -CO-, -O(CO)-, -COO-, $-C(=CH_2)-$, -C(=NH)-, -CONH-, -NHCO-, -NH(CO)NH-, -S-, -S-S-, -SO-, $-SO_2-$, $-SO_2NH_2-$ and -phenylene-, wherein L is optionally substituted as defined herein.

As a biradical, one end of L is attached to the polysarcosine-based moiety, and the other end of L is attached to the oxygen atom of the vitamin E-based moiety. When the repeating sarcosine-based unit PAA has the formula (XXI), L is attached to the nitrogen atom of the PAA by a chemically feasible bond which is selected from the group consisting of an amide bond, a carbamate bond, a urea bond, and an amine bond, more particularly the bond being formed between a -CO-, -COO-, -(CO)NH-, or $-CH_2-$ moiety on the left side of the linker L and the nitrogen atom of the polysarcosine-based moiety. When the repeating sarcosine-based unit has the formula (XXII), and m is 1, L is attached to the carbonyl group of the PAA by a chemically feasible bond which is selected from the group consisting of an amide bond and an ester bond, more particularly the bond being formed between a -(O)- or -(N)- moiety on the left side of the linker L and the carbonyl moiety of the polysarcosine-based moiety.

Further, L is attached to the oxygen atom of the vitamin E-based moiety by a chemically feasible bond which is selected from the group consisting of an ester bond, a carbamate bond and an ether bond, more particularly the bond being formed between a -CO-, -N(CO)-, or $-CH_2-$ moiety on the right side of the linker L and the oxygen of the vitamin E derivative.

In one embodiment, optionally in combination with one or more features of the various embodiments described above or below, in the polymer of formula (I), L is a biradical chain which comprises from 1-50 moieties, more particularly from 2 to 30 or from 2 to 20 or from 2 to 15 moieties as defined above, provided that L can be attached to the rest of the molecule by chemically feasible bonds.

According to one embodiment, optionally in combination with one or more features of the various embodiments described above or below, in the polymer of formula (I), PAA is a repeating sarcosine-based unit of formula (XXI), z is 1, and m is 1, i.e., the polymer of formula (I) is a polymer of formula (Ia),

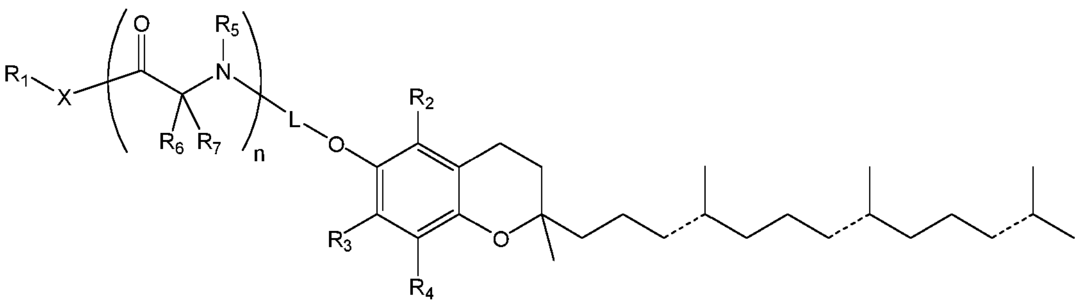

(Ia)

wherein $R_1$-$R_7$, X, L, and n are as defined herein, and L is attached to the nitrogen atom of the sarcosine-based unit by a chemically feasible bond which is selected from the group consisting of an amide bond, a carbamate bond, a urea bond, and an amine bond. More particularly, in the polymer of formula (Ia), the sarcosine-based unit is attached to X by a chemically feasible bond which is selected from the group consisting of an ester bond, and an amide bond.

In the polymers of formula (Ia), X is -O- or -NH-. In one more particular embodiment, optionally in combination with one or more features of the various embodiments described above or below, in the polymer of formula (Ia), X is -O-. In another more particular embodiment, optionally in combination with one or more features of the various embodiments

described above or below, in the polymer of formula (Ia), X is -NH-.

In a more particular embodiment, the polymer of formula (Ia) is a polymer of formula (Ia'),

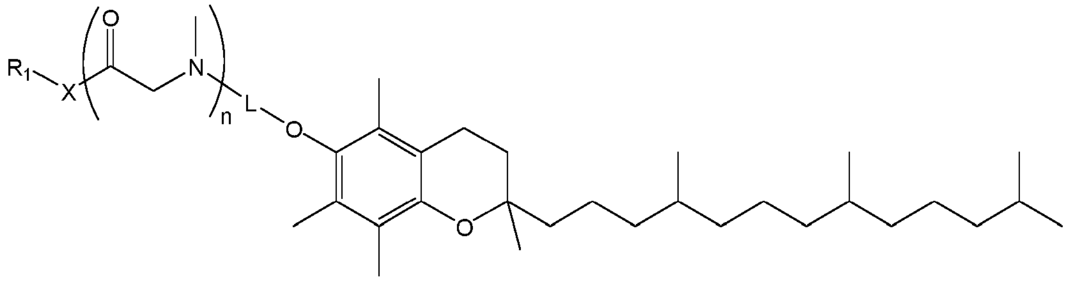

(Ia')

wherein n, $R_1$, X, and L are as defined herein, and even more particularly the polymer of formula (Ia) is a polymer of formula (Ia"):

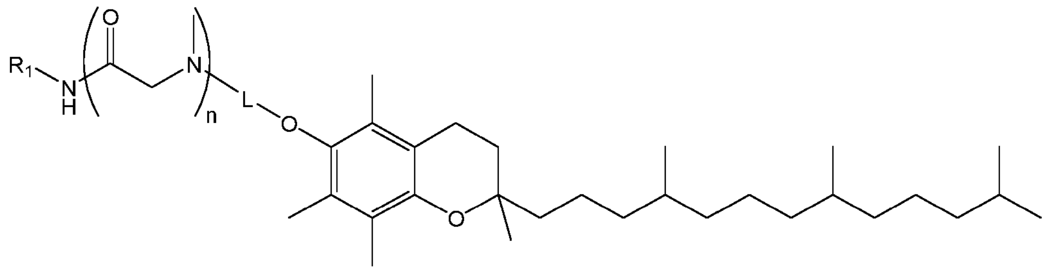

(Ia")

wherein $R_1$, and n is as defined herein.

According to a particular embodiment, optionally in combination with any of the embodiments provided above or below, in the polymers of formula (Ia) the linker L is a biradical selected from the group consisting of -$(C_1$-$C_{12})$alkylene- optionally substituted with one or more substituents selected from the group consisting of halogen, -OH, -$NR_aR_b$, -SH, -$NHNH_2$, -$COOR_c$, -$CF_3$, and -$OCF_3$, wherein $R_a$, $R_b$ and $R_c$ are radicals independently selected from the group consisting of H, -phenyl, -$(C_1$-$C_6)$alkyl, -$(C_2$-$C_6)$alkenyl, -$(C_1$-$C_6)$alkylphenyl, and -phenyl$(C_1$-$C_6)$alkyl; -$(C_1$-$C_6)$alkyl-CO-; -CO-$(C_1$-$C_6)$alkyl-; and a biradical of formula (III), (IV), (V), (VI), (VII), (VIII), (IX), (X), (XI) (XII) (XIII), (XIV), (XV), (XVI), (XVII), (XVIII) or (XIX),

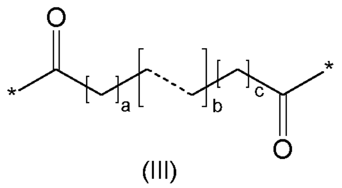

(III) ;

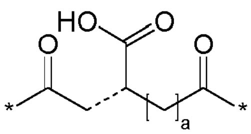

(IV) ;

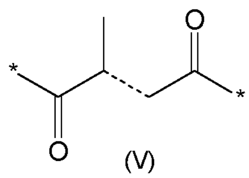

(V) ;

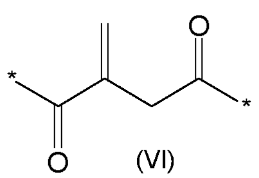

(VI) ;

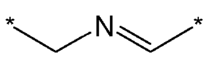

(VII) ;

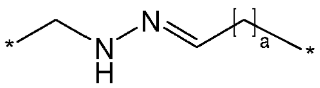

(VIII) ;

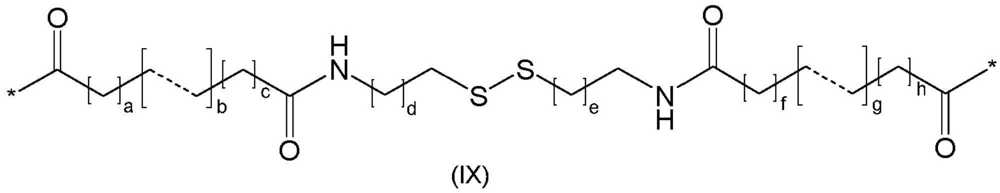 ;

(IX)

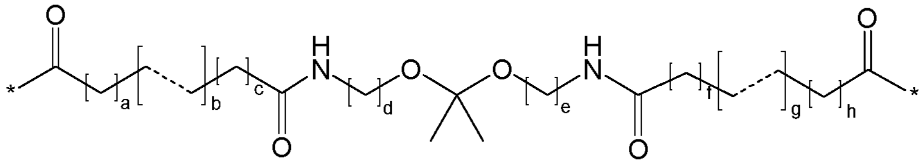 ;

(X)

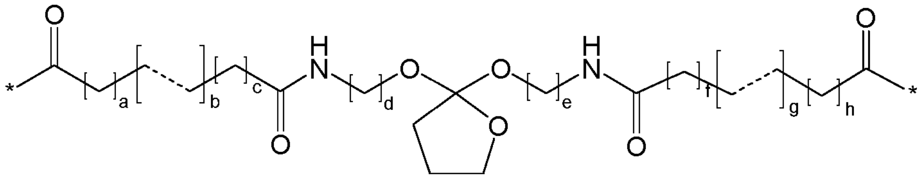 ;

(XI)

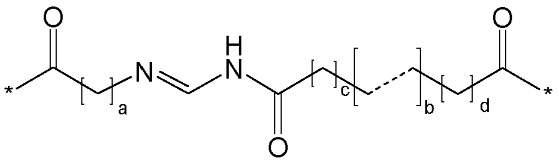 ; 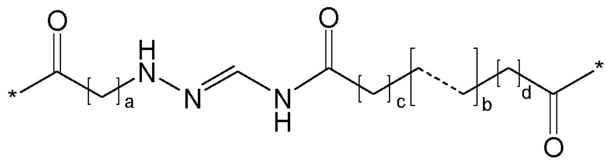 ;

(XII) (XIII)

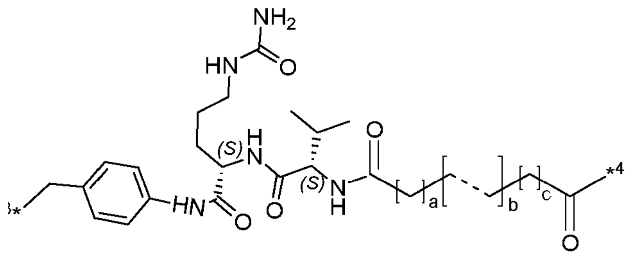 ; 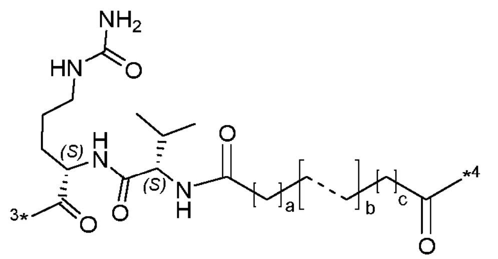 ;

(XIV) (XV)

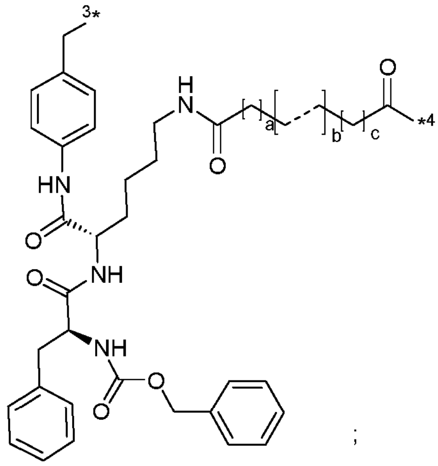

;

(XVI)

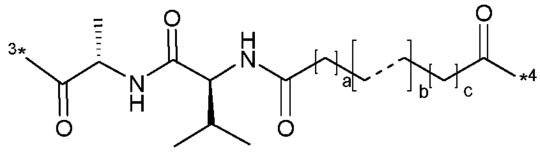

;

(XVII)

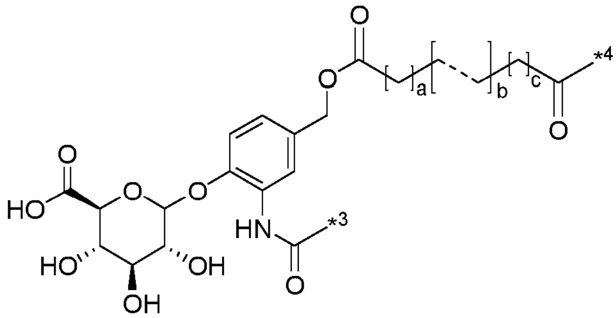

;

(XVIII)

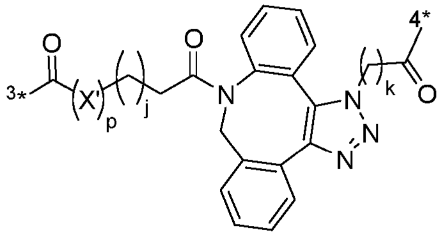

(XIX)

wherein the "*" denote the attaching points of the biradical L to the nitrogen atom of the repeating sarcosine-based unit or the oxygen atom of the vitamin E-based moiety, "*3" denotes the attaching point of the biradical L to the nitrogen atom of the repeating sarcosine-based unit, and "*4" denotes the attaching point of the biradical to the oxygen atom of the vitamin E-based moiety,

a, c, d, e, f, h, j, and k are independently an integer from 0 to 10, particularly from 0 to 6; b and g are independently an integer from 0 to 1; with the condition that when b is 0, then at least one of a or c is other than 0, and when g is 0, then at least one of f or h is other than 0;

X' is -O- or -NH-;

p is 0 or 1;

each of the dashed bonds - - - - - is independently a single bond or, alternatively, a double bond.

When drawn, the attachments points are indicated herein with an "*". As long as the specific attachment is not specified, it means that the linker may be attached to the rest of the molecule interchangeably in any direction.

In one embodiment, optionally in combination with one or more features of the various embodiments described above or below, in the polymer of formula (Ia), L is a -($C_1$-$C_{12}$)alkylene-, more particularly a -($C_1$-$C_6$)alkyl-, and is optionally substituted as defined herein.

In another embodiment, optionally in combination with one or more features of the various embodiments described above or below, in the polymer of formula (Ia), L is -($C_1$-$C_6$)alkyl-CO- or -CO-($C_1$-$C_6$)alkyl- and is optionally substituted as defined herein.

In another embodiment, optionally in combination with one or more features of the various embodiments described above or below, in the polymer of formula (Ia), L is a biradical of formula (III), more particularly a biradical of formula (III) wherein b is 0, and a + c are selected from 2, 3, 4, 5, 6, 7, 8, 9, 10, 11, and 12, and even more particularly, L is a biradical of formula (III) wherein b is 0 and a and c are 1, i.e. a biradical of formula (IIIa).

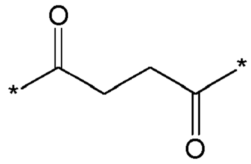

(IIIa)

In another embodiment, optionally in combination with one or more features of the various embodiments described above or below, in the polymer of formula (Ia), L is a biradical selected from the formulas (IV), (V), and (VI), more particularly, in the biradical of formula (IV), a is 1 and the dashed bond is a double bond, i.e., the biradical of formula (IV) has the formula (IVa), and in the biradical of formula (V), the dashed bond is more particularly a double bond, i.e., the biradical of formula (V) has the formula (Va).

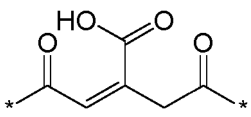 ; 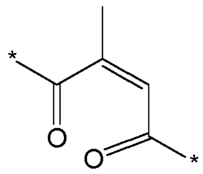

(IVa) (Va)

In another embodiment, optionally in combination with one or more features of the various embodiments described above or below, in the polymer of formula (Ia), L is a biradical selected from the formulas (VII), and (VIII), more particularly, in the biradical of formula (VIII), a is 1.

In another embodiment, optionally in combination with one or more features of the various embodiments described above or below, in the polymer of formula (Ia), L is a biradical of formula (IX), more particularly a biradical of formula (IX) wherein b and g are 0; and a, c, d, e, f and h are 1, i.e., a biradical of formula (IXa).

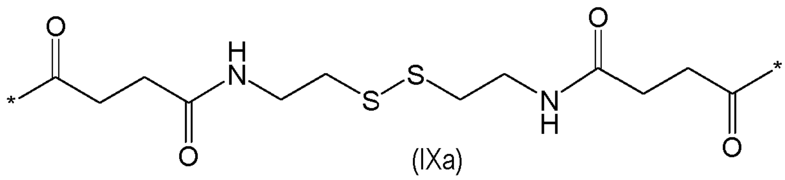

(IXa)

In another embodiment, optionally in combination with one or more features of the various embodiments described above or below, in the polymer of formula (Ia), L is a biradical selected from the formulas (X) and (XI), more particularly in the biradicals of formula (X) and (XI), b and g are 0; and a, c, d, e, f and h are 1, i.e. the biradicals of formula (X), and (XI) have the formulas (Xa), and (XIa), respectively.

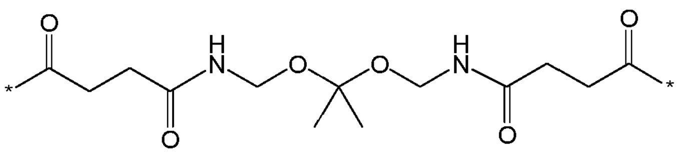 ;

(Xa)

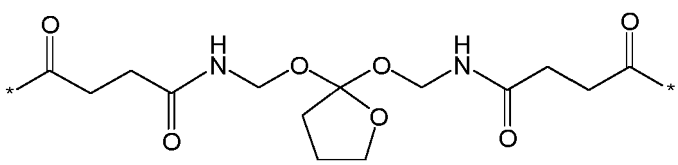

(XIa)

In another embodiment, optionally in combination with one or more features of the various embodiments

described above or below, in the polymer of formula (Ia), L is a biradical selected from the formulas (XII) and (XIII), more particularly in the biradicals of formula (XII) and (XIII), b is 0; and a, c and d are 1.

In another embodiment, optionally in combination with one or more features of the various embodiments described above or below, in the polymer of formula (Ia), L is a biradical selected from the formulas (XIV), (XV), (XVI), (XVII), and (XVIII), more particularly in the biradicals of formula (XIV), (XV), (XVI), (XVII), and (XVIII), b is 0; and a, and c are 1.

In another embodiment, optionally in combination with one or more features of the various embodiments described above or below, in the polymer of formula (Ia), L is a biradical of formula (XIX), wherein p is 0, and j and k are 1. In another embodiment, optionally in combination with one or more features of the various embodiments described above or below, in the polymer of formula (Ia), L is a biradical of formula (XIX), wherein p is 1, X' is -NH-, and j and k are 1. In another embodiment, optionally in combination with one or more features of the various embodiments described above or below, in the polymer of formula (Ia), L is a biradical selected of formula (XIX), wherein p is 1, X' is -O-, and j and k are 1.

According to a more particular embodiment, optionally in combination with one or more features of the various embodiments described above or below, in the polymer of formula (Ia), L is a biradical of formula (III) selected from the group consisting of:

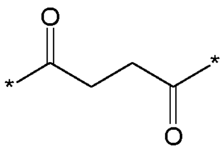 ; 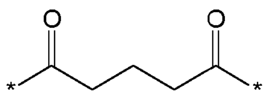 ; 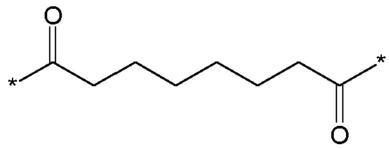 ;

According to another embodiment, optionally in combination with one or more features of the various embodiments described above or below, in the polymer of formula (I), PAA is a repeating sarcosine-based unit of formula (XXII), and z is 0, i.e., the polymer of formula (I) is a polymer of formula (Ib),

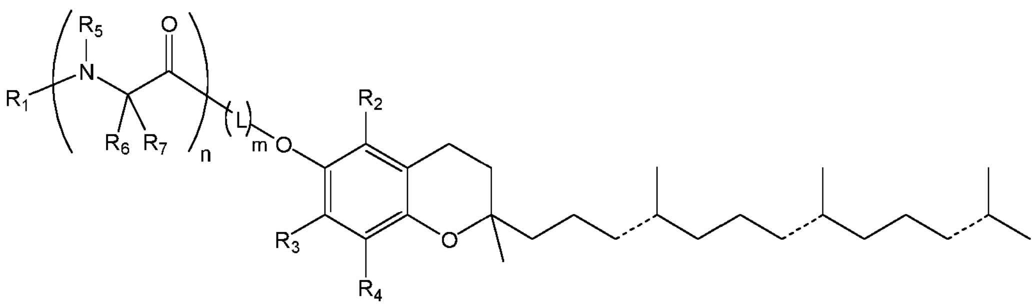

(Ib)

wherein $R_1$-$R_7$, L, m, and n are as defined herein, and when m is 1, L is attached to the carbonyl group of the PAA by a chemically feasible bond which is selected from the group consisting of an amide bond and an ester bond; and when m is 0, the PAA is attached to the oxygen atom of the vitamin E-based moiety by an ester bond. More particularly, in the polymer of formula (Ib), the PAA is attached to $R_1$ by an amine bond.

In one particular embodiment, optionally in combination with any of the embodiments provided above or below, in the polymers of formula (Ib), m is 0.

According to a particular embodiment, optionally in combination with any of the embodiments provided above or below, in the polymers of formula (Ib) m is 1, and the linker L is a biradical selected from the group consisting of -X'-($C_1$-$C_{12}$)alkylene- optionally substituted with one or more substituents selected from the group consisting of halogen, -OH, -$NR_aR_b$, -SH, -$NHNH_2$, -$COOR_c$, -$CF_3$, and -$OCF_3$, wherein $R_a$, $R_b$ and $R_c$ are radicals independently selected from the group consisting of H, -phenyl, -($C_1$-$C_6$)alkyl, -($C_2$-$C_6$)alkenyl, -($C_1$-$C_6$)alkylphenyl, and -phenyl($C_1$-$C_6$)alkyl; -X'-($C_1$-$C_6$)alkyl-CO-; and a biradical of formula (III'), (IV'), (V'), (VI'), (VII), (VIII), (IX'), (X'), (XI'), (XII') (XIII'), (XIV'), (XV'), (XVI'), (XVII'), (XVIII'), or (XIX'),

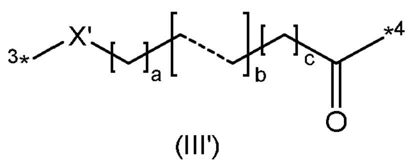 ; 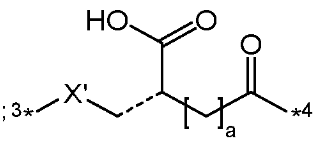 ; 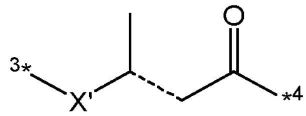 ;

(III') (IV') (V')

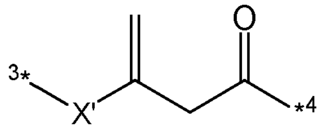 ; 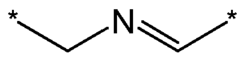 ; 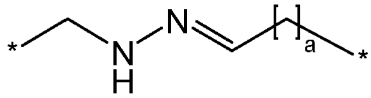 ;

(VI') (VII) (VIII)

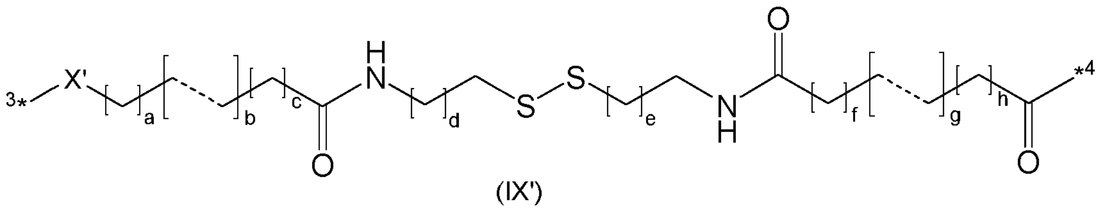 ;

(IX')

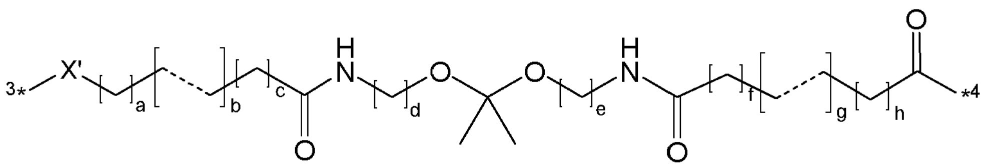 ;

(X')

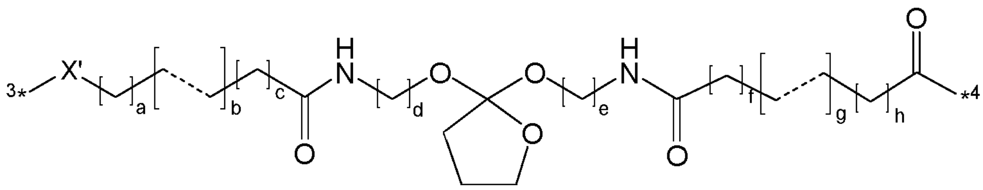 ;

(XI')

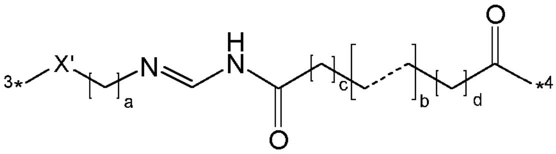 ; 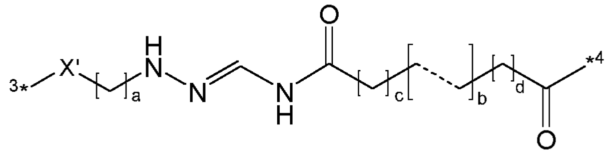

(XII') (XIII')

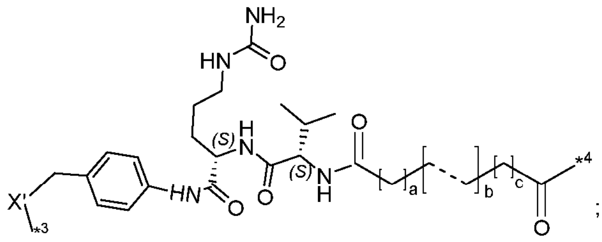

(XIV') ;

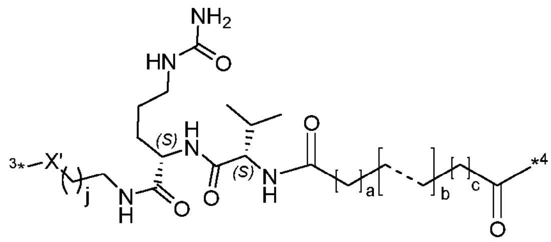

(XV')

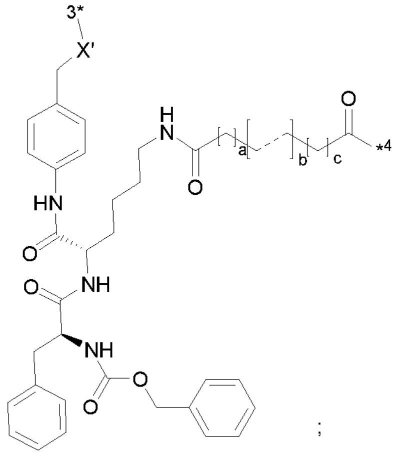

;

(XVI')

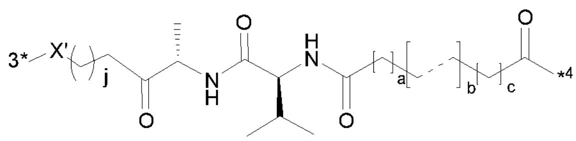

;

(XVII')

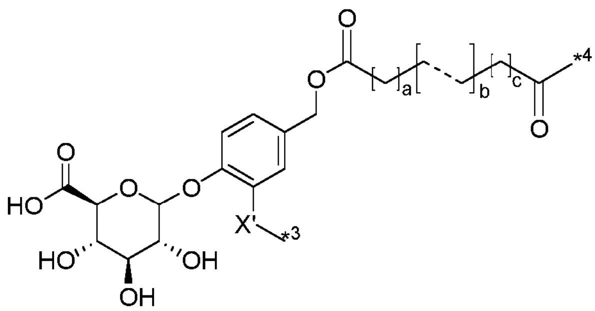

;

(XVIII')

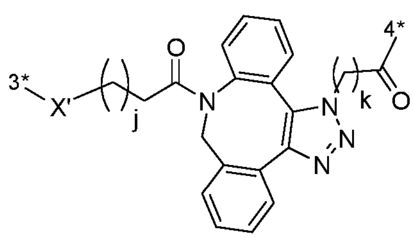

(XIX')

wherein the "*" denote the attaching points of the biradical L to the carbonyl group of the repeating sarcosine-based unit or the oxygen atom of the vitamin E-based moiety, "*3" denotes the attaching point of the biradical L to the carbonyl group, and "*4" denotes the attaching point of the biradical to the oxygen atom of the vitamin E-based moiety,

a, c, d, e, f, h, j, and k are independently an integer from 0 to 10, particularly from 0 to 6; b and g are independently an integer from 0 to 1; with the condition that when b is 0, then at least one of a or c is other than 0, and when g is 0, then at least one of f or h is other than 0;

X' is -O- or -NH-;

each of the dashed bonds - - - - - is independently a single bond or, alternatively, a double bond.

When drawn, the attachments points are indicated herein with an "*". As long as the specific attachment is not specified it means that the linker may be attached to the rest of the molecule interchangeably.

In one embodiment, optionally in combination with one or more features of the various embodiments described above or below, in the polymer of formula (Ib), L is a -X'-($C_1$-$C_{12}$)alkylene-, more particularly a - X'-($C_1$-$C_6$)alkyl-, and is optionally substituted as defined herein.

In another embodiment, optionally in combination with one or more features of the various embodiments

described above or below, in the polymer of formula (Ib), L is -X'-($C_1$-$C_6$)alkyl-CO- or - X'-($C_1$-$C_6$)alkyl- and is optionally substituted as defined herein.

In another embodiment, optionally in combination with one or more features of the various embodiments described above or below, in the polymer of formula (Ib), L is a biradical of formula (III'), more particularly a biradical of formula (III') wherein b is 0, and a + c are selected from 2, 3, 4, 5, 6, 7, 8, 9, 10, 11, and 12, and even more particularly, L is a biradical of formula (III') wherein b is 0, a and c are 1, and X' is -NH-, i.e. a biradical of formula (IIIa').

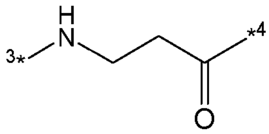

(IIIa')

In another embodiment, optionally in combination with one or more features of the various embodiments described above or below, in the polymer of formula (Ib), L is a biradical selected from the formulas (IV'), (V'), and (VI'), more particularly, X' is -NH-, and even more particularly, in the biradical of formula (IV'), a is 1 and the dashed bond is a double bond, i.e., the biradical of formula (IV') has the formula (IVa'), and in the biradical of formula (V'), the dashed bond is more particularly a double bond, and X' is -NH-, i.e., the biradical of formula (V') has the formula (Va').

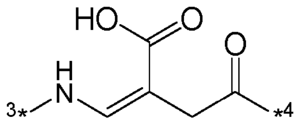

;

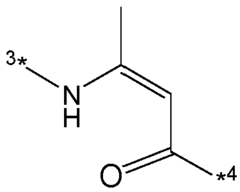

(IVa') (Va')

In another embodiment, optionally in combination with one or more features of the various embodiments described above or below, in the polymer of formula (Ib), L is a biradical selected from the formulas (VII), and (VIII), more particularly, in the biradical of formula (VIII), a is 1.

In another embodiment, optionally in combination with one or more features of the various embodiments described above or below, in the polymer of formula (Ib), L is a biradical of formula (IX'), more particularly a biradical of formula (IX') wherein b and g are 0; a, c, d, e, f and h are 1, and X' is -NH-, i.e. a biradical of formula (IXa').

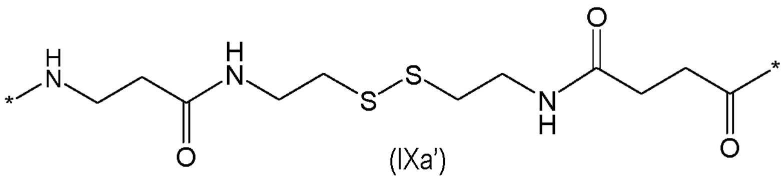

(IXa')

In another embodiment, optionally in combination with one or more features of the various embodiments described above or below, in the polymer of formula (Ib), L is a biradical selected from the formulas (X') and (XI'), more particularly in the biradicals of formula (X') and (XI'), b and g are 0; a, c, d, e, f and h are 1, and X' is -NH-, i.e. the biradicals of formula (X'), and (XI') have the formulas (Xa'), and (XIa'), respectively.

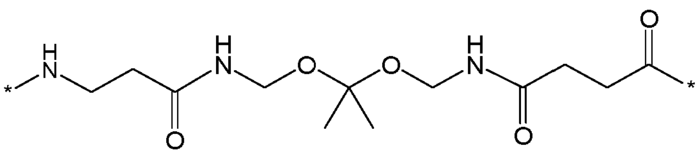

;

(Xa')

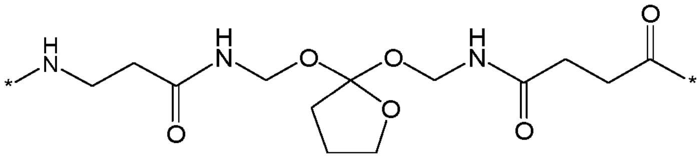

(XIa')

In another embodiment, optionally in combination with one or more features of the various embodiments described above or below, in the polymer of formula (Ib), L is a biradical selected from the formulas (XII') and (XIII'), more particularly in the biradicals of formula (XII') and (XIII'), b is 0; a, c and d are 1; and X' is -NH-.

In another embodiment, optionally in combination with one or more features of the various embodiments described above or below, in the polymer of formula (Ib), L is a biradical selected from the formulas (XIV'), (XV'), (XVI'), (XVII'), and (XVIII'), more particularly in the biradicals of formula (XIV'), (XV'), (XVI'), (XVII'), and (XVIII'), b is 0; a, and c are 1; and X' is -NH-.

In another embodiment, optionally in combination with one or more features of the various embodiments described above or below, in the polymer of formula (Ib), L is a biradical selected from the formulas (XIX'), wherein X' is -NH-, and j and k are 1. In another embodiment, optionally in combination with one or more features of the various embodiments described above or below, in the polymer of formula (Ib), L is a biradical selected from the formulas (XIX'), wherein X' is -O-, and j and k are 1.

According to a more particular embodiment, optionally in combination with one or more features of the various embodiments described above or below, in the polymer of formula (Ib), L is a biradical of formula (III') selected from the group consisting of:

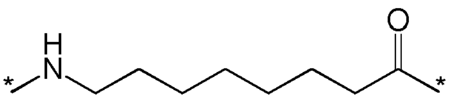 ; 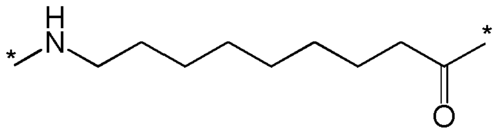 ;

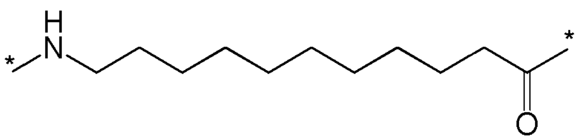 ; 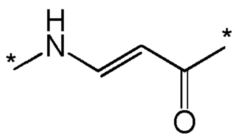 ;

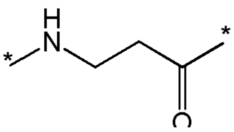 ; 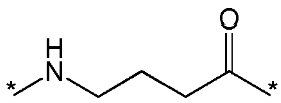 ; 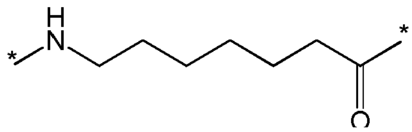 ;

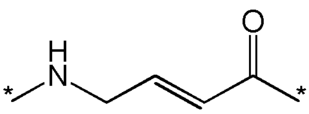 ; 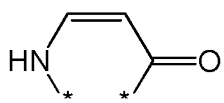

Processes for the preparation of the polymers of formula (I) are also part of the disclosure (not claimed). The polymers of formula (I) may be prepared by synthetic processes which are well-known in the art.

For example, the polymers of formula (I) wherein the repeating sarcosine-based unit PAA has the formula (XXI) (i.e., polymers of formula (Ia)) may be prepared by reacting a polymer of formula (A) with a polymer of formula (B), as shown in the scheme below:

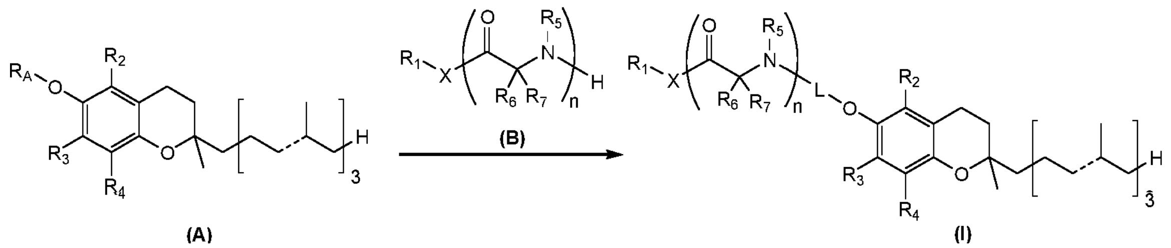


(A) (I)

wherein $R_A$ is selected from $-L_1-(CO)-Y$ or $-L_2-O-H$, or -L-LG, wherein $-L_1-(CO)-$ is a linker L which ends with a -CO- moiety, $-L_2-O-$ is a linker L which ends with a -O- moiety, and Y is OH or a halogen atom, LG is a leaving group, and n, X, $R_1$, $R_2$, $R_3$, $R_4$, $R_5$, $R_6$, $R_7$, and L are as defined herein.

When $R_A$ is $-L_1-(CO)-Y$ and Y is OH, an amide bond is formed between compounds (A) and (B). This reaction may be carried out in the presence of an activating agent such as: 4-(4,6-dimethoxy-1,3,5-triazin-2-yl)-4-methylmorpholinium tetrafluoroborate ($DMTMMBF_4$), 1-ethyl-3-(3-dimethylaminopropyl)carbodiimide hydrochloride (EDC.HCl) or hydroxybenzo-triazole (HOBt), preferably in the presence of a base, such as triethylamine (TEA), N-methylmorpholine (NMM), or N, N-diisopropylethylamine (DIPEA), in a suitable solvent, such as dimethylformamide, dichloromethane or chloroform, at a temperature comprised from room temperature to the temperature of the boiling point of the solvent, preferably at room temperature.

When $R_A$ is $-L_1-(CO)-Y$ and Y is a halogen atom, the reaction may be carried out in the presence of a base, such as triethylamine, in a suitable solvent, such as tetrahydrofuran, at a temperature comprised from room temperature to the reflux temperature of the solvent. The compound of formula (A) when X is a halogen atom may be prepared from the corresponding carboxylic acid by methods well-known in the art.

When $R_A$ is $-L_2-OH$, the reaction may be carried out in the presence of a carbonyl source such as 1,1'-carbonyldiimidazole (CDI), N,N'-disuccinimidyl carbonate (DSC) or bis(trichloromethyl) carbonate (BTC), in a suitable solvent such as acetonitrile or 1,4-dioxane, at a temperature comprised from room temperature to the reflux temperature of the solvent.

When $R_A$ is -L-LG, the reaction may be carried out in the presence of a base such as triethylamine (TEA), N-methylmorpholine (NMM), or N, N-diisopropylethylamine (DIPEA), in a suitable solvent, such as acetonitrile, tetrahydrofuran, dimethylformamide, dichloromethane or chloroform, at a temperature comprised from room temperature to the temperature of the boiling point of the solvent, preferably at room temperature. Non-limiting examples of leaving groups include halogen, $-OSO_2R$, and -OCOR, wherein R is selected from the group consisting of $(C_1-C_4)$alkyl optionally substituted with one or more halogen atoms, and $(C_6-C_{12})$aryl optionally substituted with $(C_1-C_4)$alkyl, or with $(C_1-C_4)$alkyl optionally substituted with one or more halogen atoms. More particularly, LG is selected from halogen, $-OSO_2CH_3$ (-OMs), $-OSO_2C_7H_7$ (-OTs), $-OSO_2CF_3$(-OTf), $-OCOCH_3$(-OAc), -OCOPh (-OBz), and $-OCOCF_3$(-TFA).

Compounds of formula (A) wherein $R_A$ is $-L_1-(CO)-Y$ and Y is OH, i.e., compounds of formula ($A_1$), are commercially available or may be prepared by reacting a compound of formula (C), which is a vitamin E derivative, with a compound of formula (D):

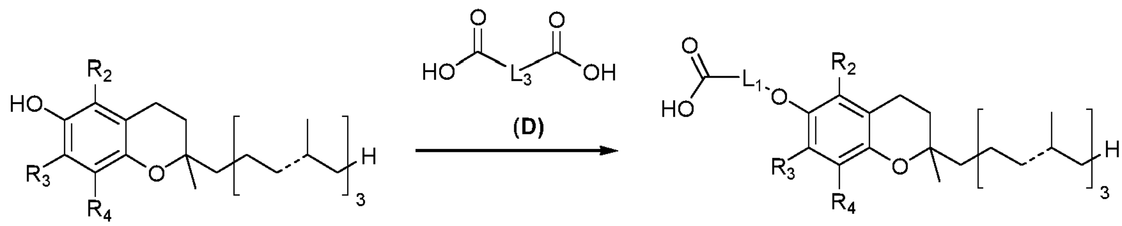


(C) ($A_1$)

wherein $R_2$, $R_3$, and $R_4$ are as defined herein, and $L_3$ is a linker L which ends with two -COOH moieties. This reaction may be carried out under analogue conditions as the ones previously disclosed for the formation of ester bonds.

Compounds of formula (A) wherein $R_A$ is -L-LG, i.e. compounds of formula ($A_2$), may be prepared by reacting a compound of formula (C), with a compound of formula (E),

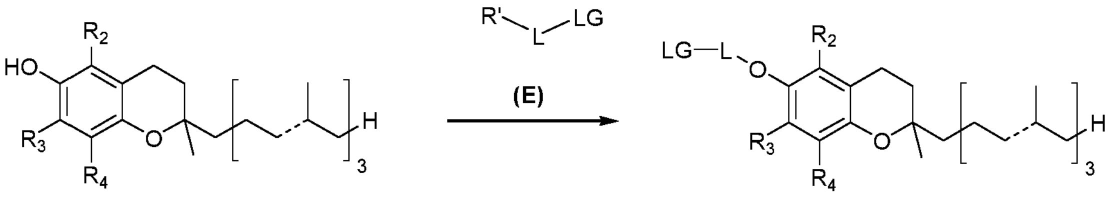


(C) (A₂)

wherein R' is -COOH or an anhydride or ester derivative thereof, or a leaving group, under analogue conditions as the ones previously disclosed for the formation of ether bonds.

Compounds of formula (B) may be prepared by ring opening polymerization (ROP) of N-carboxy anhydrides (NCAs), in particular 3-alkyloxazolidine-2,5-diones (compounds of formula (F)). Amines or alcohols of formula (G) are used as initiators in order to obtain a linear poly sarcosine polymer ($R_1$-X-$PSar_n$). The reaction is carried out in a suitable solvent such as dimethylformamide, at a temperature comprised from 4 °C to room temperature, preferably at about 10 °C.

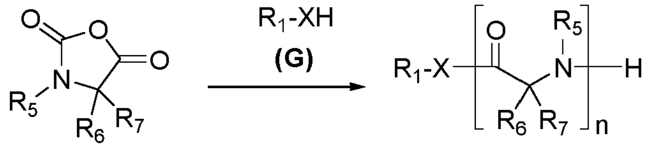


(F) (B)

The ratio monomer (NCA)/initiator (alcohol/amine) allow to control the degree of polymerization (DP). Polymers of formula (I) wherein the repeating sarcosine-based unit PAA has the formula (XXII) (i,e., polymers of formula (Ib)) may be prepared by synthetic processes which are well-known in the art. For example, a compound of formula (XXII) wherein m is 1, may be prepared by ring opening polymerization (ROP) of a N-carboxy anhydride of formula (F) by using an amine or alcohol of formula (H) as initiator:

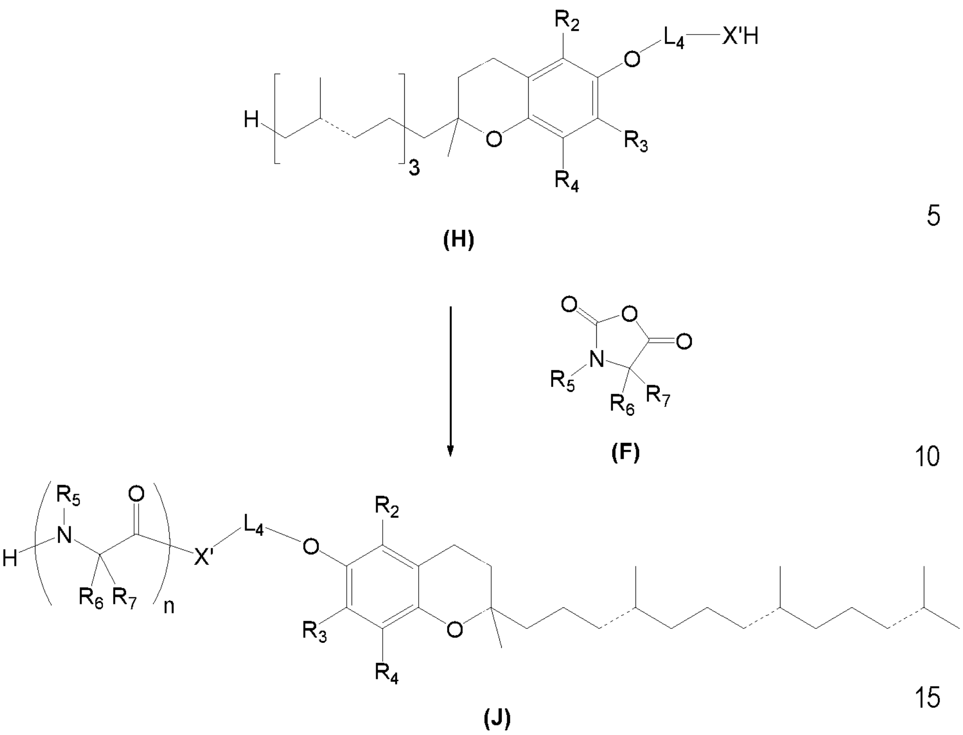
(H)
(F)

(J)

wherein -$L_4$-X'- is a linker L which ends with a X' moiety, wherein X' is -NH- or -O-.

When m is 0, the compound of formula (XXII) may be prepared by ring opening polymerization (ROP) of a N-carboxy anhydride of formula (F) by using an amine or alcohol of formula (K) as initiator:

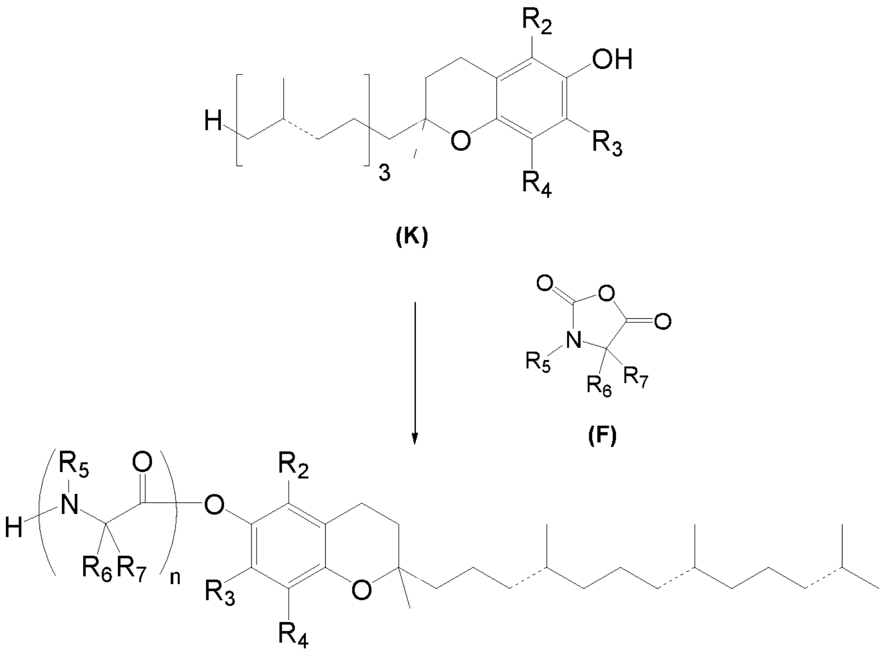

(K)

(F)

(J')

The obtained intermediates of formulas (J) and (J') may be reacted with a compound of formula R1-LG, wherein LG is a leaving group, to give rise to compounds of formula (I).

Conjugates of formula (II)

Using appropriate surface functionality, the polymers of the present invention may be further decorated with other molecules that can actively target cells and aid in cellular entry, resulting in a conjugate which has improved cell-specific delivery.

Accordingly, the present invention also relates to conjugates of the polysarcosine vitamin E derivatives of the invention with other molecules which are attached to them by covalent bonds, either directly or through linkers. Thus, the invention relates to a conjugate of formula (II) or a pharmaceutically, nutraceutically, or cosmetically acceptable salt thereof, or any stereoisomer or mixtures of stereoisomers, either of the conjugate of formula (II) or of any of its salts,

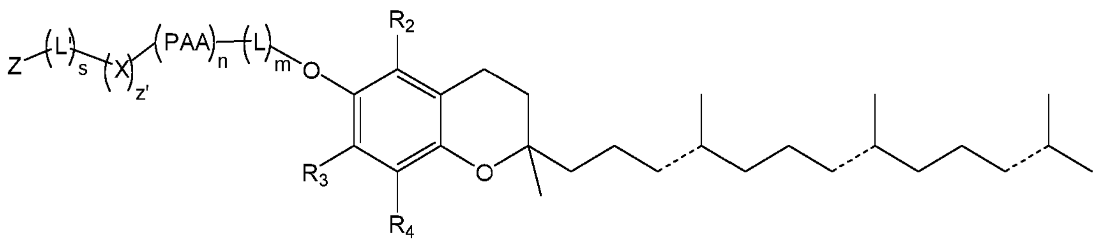

(II)

wherein m, PAA, X, n, L, $R_2$, $R_3$, $R_4$, and the dashed bonds are as defined herein;

Z is an agent selected from the group consisting of a nutraceutically active agent, a pharmaceutically active agent, a cell-targeting agent, a diagnostically active agent, and a cosmetically active agent;

s is 0 or 1; and

z' is 0 or 1, with the condition that when the repeating sarcosine-based unit PAA has the formula (XXII), then z' is 0;

L' is a biradical chain which comprises one or more moieties selected from the group consisting of -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -COO-, $-C(=CH_2)-$,

-C(=NH)-, -CONH-, -NHCO-, -NH(CO)NH-, -S-, -S-S-, -SO-, $-SO_2-$, $-SO_2NH_2-$ and -phenylene-, wherein L' is optionally substituted as defined herein.

According to one embodiment, optionally in combination with one or more features of the various embodiments described above or below, in the conjugate of formula (II), PAA is a repeating sarcosine-based unit of formula (XXI), i.e. the conjugate of formula (II) is a conjugate of formula (IIa),

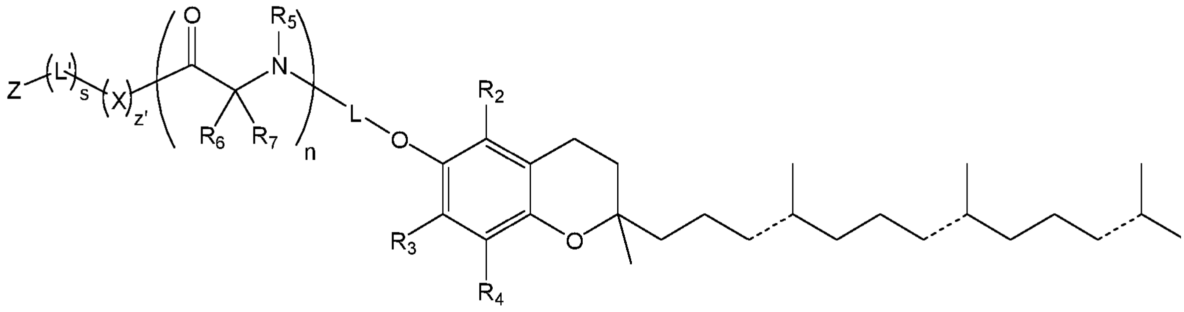

(IIa)

wherein n, X, L, L', Z, z', s, $R_2$-$R_7$, and the dashed bonds are as defined herein.

According to another embodiment, optionally in combination with one or more features of the various embodiments described above or below, in the conjugate of formula (II), PAA is a repeating sarcosine-based unit of formula (XXII), i.e. the conjugate of formula (II) is a conjugate of formula (IIb),

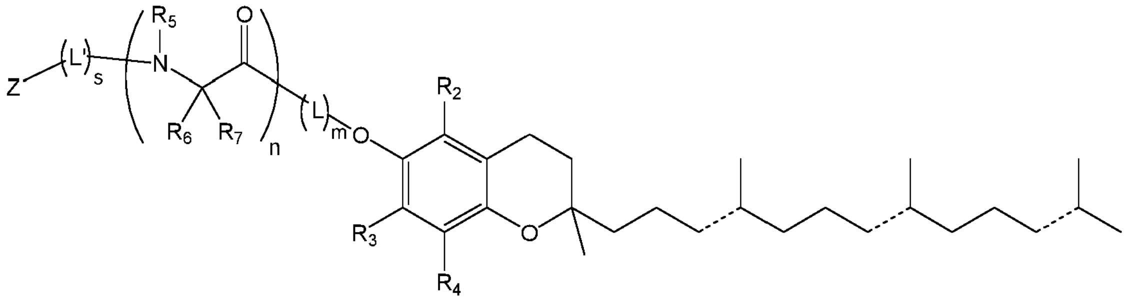

(IIb)

wherein n, L, L', Z, s, m, $R_2$-$R_7$, and the dashed bonds are as defined herein.

In one embodiment, optionally in combination with any of the embodiments provided above or below, Z is a cell-targeting agent. As used herein, the term "cell-targeting agent" refers to any molecule, macromolecule, or biomacromolecule, such as a nutraceutically active agent, a pharmaceutically active agent, a diagnostically active agent, and a cosmetically active agent, which selectively binds to receptors that are expressed or over-expressed on specific cell types. Cell-targeting groups are well known in the art.

Examples of cell-targeting groups include, but are not limited to, galactosamine, folate, a Her-2 binding peptide, TLR agonists, β-D-Glucose, Asn-Gly-Arg peptide, angiopep2, folic acid, aptamers (A-9, A10, Anti-gp120, TTA1, sgc8, Anti MUC-1, AS1411), primaquine, zidovudine, superoxide dismutase, prednisolone, platinum, cisplatin, sulphamethoxazole, amoxicillin, etoposide, mesalzine, doxorubicin, paclitaxel, 5-amino salicylic acid, denosumab, docetaxel, calcitonin, proanthocyanidin, methotrexate, camptothecin, galactose, glycyrrhetinic acid, lactose, hyaluornic acid, octeotride, lactobionic acid, β-galactosyl moiety, arabino-galactan, chitosan, azo-based poly-phosphazene, azo group and 4-amino-benzyl-carbamate, succinate, 4,4'-dihydroxyazo benzene-3-carboxilic acid, cyclic RGD penta-peptide, Aspartic acid octapeptide, alendronate, transferrin, bisphosphonate adendronate, mono sialoganglioside GM1, gluthatione, E-selectin thioaptamer, poloxamer-407, a urokinase-type plasminogen activator receptor (uPAR) antagonist, a CXCR4 chemokine receptor antagonist, a GRP78 peptide antagonist, an RGD peptide, an RGD cyclic peptide, a luteinizing hormone-releasing hormone (LHRH) antagonist peptide, an aminopeptidase targeting peptide, a brain homing peptide, a kidney homing peptide, a heart homing peptide, a gut homing peptide, an integrin homing peptide, an angiogenic tumor endothelium homing peptide, an ovary homing peptide, a uterus homing peptide, a sperm homing peptide, a microglia

homing peptide, a synovium homing peptide, a urothelium homing peptide, a prostate homing peptide, a lung homing peptide (e.g. RCPLSHSLICY), laminin receptor binding peptide (e.g. YIGSR) a skin homing peptide, a retina homing peptide, a pancreas homing peptide, a liver homing peptide, a lymph node homing peptide, an adrenal gland homing peptide, a thyroid homing peptide, a bladder homing peptide, a breast homing peptide, a neuroblastoma homing peptide, a lymphoma homing peptide, a muscle homing peptide, a wound vasculature homing peptide, an adipose tissue homing peptide, a virus binding peptide, or a fusogenic peptide.

According to another embodiment, optionally in combination with one or more features of the various embodiments described above or below, in the conjugate of formula (II), Z is anticancer drug. Non-limiting examples of anticancer drugs include, without limitation, cisplatin, paclitaxel, 5-fluoracil, gemcitabine, Herceptin, mitoxantrone, cantharidin, docetaxel, anthracycline drugs (doxorubicin, epirrubicin, daunorubicin, ildarubicin), irinotecan, or camptothecin.

In accordance with a particular embodiment, optionally in combination with any of the embodiments provided above or below, Z is selected from the group consisting of low molecular weight drugs, peptides, antibodies, hormones, enzymes, nucleic acids, proteins, and combinations thereof.

As used herein, the term "nucleic acid" refers to DNA or RNA. In a particular embodiment, optionally in combination with any of the embodiments provided above or below, the nucleic acid is an DNA/RNA hybrid, a short interfering RNA (siRNA), a microRNA (miRNA), a single guide RNA (sgRNA), a donorDNA, a self-amplyfing/replicating RNA, a circularRNA (oRNA), a plasmid DNA (pDNA), a closed-linear DNA (clDNA), a short hairpin RNA (shRNA), messenger RNA (mRNA), and antisense RNA (aRNA), a messenger RNA (mRNA), a CRISPR guide RNA, an antisense nucleic acid, a decoy nucleic acid, an aptamer, and a ribozyme to name a few, and encompasses both the nucleotide sequence and any structural embodiments thereof, such as double stranded, single stranded, helical, hairpin, etc, and may contain modified or unmodified bases. When distinct nucleic acids are provided, they may be all DNA molecules or all RNA molecules or may be mixtures of DNA and RNA molecules or molecules comprising an association of DNA and RNA strands.

The nucleic acid may be a poly- or oligonucleotide, such as oligo- or poly-double stranded RNA, oligo- or poly-double stranded DNA, oligo- or poly-single stranded RNA, oligo- or poly-single stranded DNA. Each of the nucleotides contained in the nucleic acid may be a naturally occurring nucleotide or a chemically-modified non-naturally occurring nucleotide. The strand length of the nucleic acid is not particularly limited, and the nucleic acid may have a short strand ranging from 10 to 200 bases, preferably from 20 to 180 bases, preferably from 25 to 100 bases, preferably from 30 to 50 bases; or the nucleic acid may have a relatively long strand of from 200 to 20000 bases, more preferably of from 250 to about 15000 bases.

In accordance with a particular embodiment, optionally in combination with any of the embodiments provided above or below, the nucleic acid is closed-linear DNA (clDNA), i.e., molecules wherein the double stranded region is flanked and protected by two single stranded loops thereby generating dumbbell-shaped molecules.

In a more particular embodiment, optionally in combination with any of the embodiments provided above or below, the clDNA consists of a stem region comprising a double stranded DNA sequence of interest covalently closed at both ends by hairpin loops, the clDNA comprising at least two modified nucleotides.

As used herein, the term "closed linear DNA" or "clDNA" refers to a single stranded covalently closed DNA molecule that forms a "dumbbell" or "doggy-bone" shaped structure under conditions allowing nucleotide hybridization. Therefore, although the clDNA is formed by a single stranded DNA molecule, the formation of the "dumbbell" structure by the hybridization of two complementary sequences within the same molecule generates a structure consisting on a double-stranded middle segment flanked by two single-stranded loops. The skilled in the art know how to generate clDNA from open or closed double stranded DNA using routine molecular biology techniques. For instance, those skilled in the art knows that a clDNA can be generated by attaching hairpin DNA adaptors -for instance, by the action of a ligase- to both ends of an open double stranded DNA. "Hairpin DNA adaptor" refers to a single stranded DNA that forms a stem-loop structure by the hybridization of two complementary sequences, wherein the stem region formed is closed at one end by a single stranded loop and is open at the other
end.
A "modified nucleotide" is any nucleotide (e.g., adenosine, guanosine, cytidine, uracil, and thymidine) that has been chemically modified -by modification of the base, the sugar, or the phosphate group- or that incorporates a non-natural moiety in its structure. Thus, the modified nucleotide may be naturally or non-naturally occurring depending on the modification

In the context of the present disclosure the expression, "diagnostically active agent", also referred to as "labeling or imaging agent", refers to any substance that is used as a label, or that enhances specific structures in any imaging technique. An imaging agent, hence, includes optical imaging agent, magnetic resonance imaging agent, radioisotope, and contrast agent. Imaging or labelling agents are well known in the art. Particular examples or imaging or labelling agents are gases such as sterilized air, oxygen, argon, nitrogen, fluor, perfluorocarbons, carbon dioxide, nitrogen dioxide, sulfur hexafluoride, xenon and helium; commercially available agents used in positron emission tomography (PET), computer assisted tomography (CAT), single photon emission computerized tomography, x-ray, fluoroscopy, and magnetic

resonance imaging (MRI). Examples of suitable materials for use as contrast agents in MRI include the gadolinium chelates currently available, such as diethylene triamine pentaacetic acid (DTPA) and gadopentotate dimeglumine, as well as iron, magnesium, manganese, copper and chromium. Examples of materials useful for CAT and x-rays include iodine-based materials for intravenous administration, such as ionic monomers typified by diatrizoate and iothalamate, non-ionic monomers such as iopamidol, isohexol, and ioversol, non-ionic dimers, such as iotrol and iodixanol, and ionic dimers, for example, ioxagalte. Other useful materials include barium for oral use and non-soluble salts such as zinc acetate. In some molecules, an imaging agent is a dye. In some molecules, an imaging agent is a fluorescent moiety. In some molecules, a fluorescent moiety is selected from: a fluorescent protein, a fluorescent peptide, a fluorescent dye, a fluorescent material or a combination thereof. Examples of fluorescent dyes include, but are not limited to, xanthenes (e.g., rhodamines, rhodols and fluoresceins, and their derivatives); bimanes; coumarins and their derivatives (e.g., umbelliferone and aminomethyl coumarins); aromatic amines (e.g., dansyl; squarate dyes); benzofurans; fluorescent cyanines; indocarbocyanines; carbazoles; dicyanomethylene pyranes; polymethine; oxabenzanthrane; xanthene; pyrylium; carbostyl; perylene; acridone; quinacridone; rubrene; anthracene; coronene; phenanthrecene; pyrene; butadiene; stilbene; porphyrin; pthalocyanine; lanthanide metal chelate complexes; rare-earth metal chelate complexes; and derivatives of such dyes. Examples of fluorescein dyes include, but are not limited to, 5-carboxyfluorescein, fluorescein-5-isothiocyanate, fluorescein-6-isothiocyanate and 6-carboxyfluorescein. Examples of rhodamine dyes include, but are not limited to, tetramethylrhodamine-6-isothiocyanate, 5-carboxytetramethylrhodamine, 5-carboxy rhodol derivatives, tetramethyl and tetraethyl rhodamine, diphenyldimethyl and diphenyldiethyl rhodamine, dinaphthyl rhodamine, rhodamine 101 sulfonyl chloride (sold under the tradename of TEXAS RED(R)). Examples of cyanine dyes include, but are not limited to, Cy3, Cy3B, Cy3.5, Cy5, Cy5.5, Cy7, IRDYE680, Alexa Fluor 750, IRDye800CW, ICG. Examples of fluorescent peptides include GFP (Green Fluorescent Protein) or derivatives of GFP (e.g., EBFP, EBFP2, Azurite, mKalama1, ECFP, Cerulean, CyPet, YFP, Citrine, Venus, YPet). Fluorescent labels are detected by any suitable method. For example, a fluorescent label may be detected by exciting the fluorochrome with the appropriate wavelength of light and detecting the resulting fluorescence, e.g., by microscopy, visual inspection, via photographic film, by the use of electronic detectors such as charge coupled devices (CCDs), photomultipliers, etc. In some molecules, the imaging agent is labeled with a positron-emitting isotope (e.g.,18F) for positron emission tomography (PET), gamma-ray isotope (e.g., 99mTc) for single photon emission computed tomography (SPECT), or a paramagnetic molecule or nanoparticle (e.g., Gd3+ chelate or coated magnetite nanoparticle) for magnetic resonance imaging (MRI). In some molecules, the imaging agent is labeled with: a gadolinium chelate, an iron oxide particle, a super paramagnetic iron oxide particle, an ultra small paramagnetic particle, a manganese chelate or gallium containing agent. Examples of gadolinium chelates include, but are not limited to diethylene triamine pentaacetic acid (DTPA), 1,4,7,10-tetraazacyclododecane-1,4,7,10-tetraacetic acid (DOTA), and 1,4,7-triazacyclononane-N,N',N"-triacetic acid (NOTA). In some molecules, the imaging agent is a near-infrared fluorophore for near-infra red (near-IR) imaging, a luciferase (firefly, bacterial, or coelenterate) or other luminescent molecule for bioluminescence imaging, or a perfluorocarbon-filled self-assembled particle for ultrasound. In some molecules, the imaging agent is a nuclear probe. In some molecules, the imaging agent is a SPECT or PET radionuclide probe. In some molecules, the radionuclide probe is selected from: a technetium chelate, a copper chelate, a radioactive fluorine, a radioactive iodine, a indiuim chelate. Examples of Tc chelates include, but are not limited to HYNIC, DTPA, and DOTA. In some molecules, the imaging agent contains a radioactive moiety, for example a radioactive isotope such as $^{211}At$, $^{131}I$, $^{125}I$, $^{90}Y$, $^{186}Re$, $^{188}Re$, $^{153}Sm$, $^{212}Bi$, $^{32}P$, $^{64}Cu$ radioactive isotopes of Lu, and others.

All embodiments indicated herein for the polymers of formula (I) also apply to the conjugates of formula (II) for all the common structure they have. Also, the embodiments referred to the salts and stereoisomers indicated for the polymers of formula (I) apply to the conjugates of formula (II).

In the conjugates of formula (II), when s is 0, Z is directly attached to X or the PAA sarcosine repeating unit by a chemically feasible bond which is selected from the group consisting of an amide bond, a carbamate bond, an amine bond, a urea bond, an ether bond, and an ester bond.

More particularly, when s is 0, the sarcosine repeating unit PAA is of formula (XXI) and z' is 0, Z is directly attached to the PAA sarcosine repeating unit by a chemically feasible bond which is selected from the group consisting of an amide bond, and an ester bond, even more particularly, the bond between Z and the PAA sarcosine repeating unit may be formed between the -(O)- or -(N)-moiety of Z and the carbonyl group of the PAA sarcosine repeating unit.

When s is 0, the sarcosine repeating unit PAA is of formula (XXI) and z' is 1, Z is directly attached to X by a chemically feasible bond which is selected from the group consisting of an amide bond, a carbamate bond, an ester bond, and amine bond, an urea bond, and an ether bond, even more particularly, the bond between Z and X may be formed between the -(O)- or -(N)-moiety of X and a -CO-, -COO-, -CONH-, or $-CH_2-$ moiety of Z.

When s is 0, the sarcosine repeating unit PAA is of formula (XXII) and z' is 0, Z is directly attached to the PAA sarcosine repeating unit by a chemically feasible bond which is selected from the group consisting of an amide bond, a carbamate bond, a urea bond, and an amine bond, even more particularly, the bond between Z and the PAA may be formed between the -N- moiety of the PAA and a -CO-, -COO-, -CONH-, or $-CH_2-$ moiety of Z.

When s is 1 L' is attached to Z by a chemically feasible bond which is selected from the group consisting of an

amide bond, a carbamate bond, an amine bond, a urea bond, an ether bond, and an ester bond, and L' is attached to X or the PAA sarcosine repeating unit by a chemically feasible bond which is selected from the group consisting of an amide bond, a carbamate bond, an amine bond, a urea bond, an ether bond, and an ester bond.

More particularly, when s is 1, the sarcosine repeating unit PAA is of formula (XXI) and z' is 0, L' is directly attached to the PAA sarcosine repeating unit by a chemically feasible bond which is selected from the group consisting of an amide bond, and an ester bond, even more particularly, the bond between L' and the PAA sarcosine repeating unit may be formed between a -(O)- or -(N)-moiety of L' and the carbonyl group of the PAA sarcosine repeating unit.

More particularly, when s is 1, the sarcosine repeating unit PAA is of formula (XXI) and z' is 1, L' is attached to X by a chemically feasible bond which is selected from the group consisting of an amide bond, a carbamate bond, an ester bond, a urea bond, an amine bond, and an ether bond, more particularly, the bond between L' and X may be formed between the -(O)- or -(N)-moiety of X and a -CO-, -OCO-, -NHCO-, or $-CH_2-$ moiety of L'.

When s is 1, the sarcosine repeating unit PAA is of formula (XXII) and z' is 0, L' is directly attached to the PAA sarcosine repeating unit by a chemically feasible bond which is selected from the group consisting of an amide bond, a carbamate bond, a urea bond and an amine bond, more particularly, the bond between L' and the PAA may be formed between the -N- moiety of the PAA and a -CO-, -COO-, -CONH-, or $-CH_2-$ moiety of L'.

In one embodiment, optionally in combination with one or more features of the various embodiments described above or below, in the conjugate of formula (II), L' is a biradical chain which comprises from 1-50 moieties, more particularly from 2 to 30 or from 2 to 20 or from 2 to 15 moieties as defined above, provided that L' can be attached to the rest of the molecule by chemically feasible bonds.

According to a more particular embodiment, optionally in combination with any of the embodiments provided above or below, when in the conjugate of formula (II) the sarcosine repeating unit PAA is of formula (XXI), z' is 1, and s is 1, the linker L' is a biradical selected from the group consisting of $-(C_1-C_{12})$alkylene- optionally substituted with one or more substituents selected from the group consisting of halogen, -OH, $-NR_aR_b$, -SH, $-NHNH_2$, $-COOR_c$, $-CF_3$, and $-OCF_3$, wherein $R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl; $-(C_1-C_6)$alkyl-CO-; $-CO-(C_1-C_6)$alkyl-; and a biradical of formula (III), (IV), (V), (VI), (VII), (VIII), (IX), (X), (XI) (XII) (XIII), (XIV), (XV), (XVI), (XVII), (XVIII) or (XIX), as previously defined.

According to another more particular embodiment, optionally in combination with any of the embodiments provided above or below, when in the conjugate of formula (II) the sarcosine repeating unit PAA is of formula (XXII), z' is 0, and s is 1, the linker L' is a biradical selected from the group consisting of $-(C_1-C_{12})$alkylene-optionally substituted with one or more substituents selected from the group consisting of halogen, -OH, $-NR_aR_b$, -SH, $-NHNH_2$, $-COOR_c$, $-CF_3$, and $-OCF_3$, wherein $R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl; $-(C_1-C_6)$alkyl-CO-; $-CO-(C_1-C_6)$alkyl-; and a biradical of formula (III), (IV), (V), (VI), (VII), (VIII), (IX), (X), (XI) (XII) (XIII), (XIV), (XV), (XVI), (XVII), (XVIII) or (XIX), as previously defined.

According to a more particular embodiment, optionally in combination with any of the embodiments provided above or below, when in the conjugate of formula (II) the sarcosine repeating unit PAA is of formula (XXI), z' is 0, and s is 1, the linker L' is a biradical selected from the group consisting of $-X'-(C_1-C_{12})$alkylene- optionally substituted with one or more substituents selected from the group consisting of halogen, -OH, $-NR_aR_b$, -SH, $-NHNH_2$, $-COOR_c$, $-CF_3$, and $-OCF_3$, wherein $R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl; $-X'-(C_1-C_6)$alkyl-CO-; $-X'-CO-(C_1-C_6)$alkyl-; and a biradical of formula (III'), (IV'), (V'), (VI'), (VII), (VIII), (IX'), (X'), (XI'), (XII') (XIII'), (XIV'), (XV'), (XVI'), (XVII'), (XVIII'), or (XIX'), as previously defined.

In one embodiment, optionally in combination with one or more features of the various embodiments described above or below, the Z is a moiety has the formula (XX):

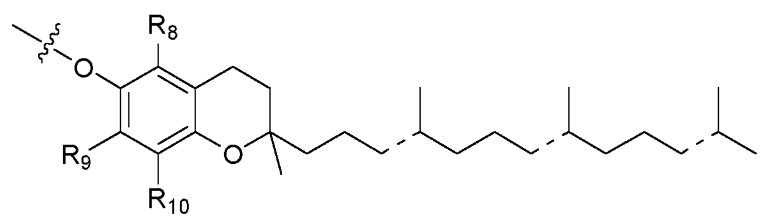

(XX)

wherein $R_8$, $R_9$ and $R_{10}$ are independently selected from the group consisting of hydrogen, fluorine, methyl, $-CH_2F$, $-CHF_2$, and $-CF_3$; and each of the dashed bonds ----- is independently a single bond or, alternatively, a double bond.

According to one embodiment, optionally in combination with one or more features of the various embodiments

described above or below, $R_8$, $R_9$ and $R_{10}$ are independently selected from the group consisting of hydrogen and methyl.

In another embodiment, optionally in combination with any of the embodiments provided above or below, in the conjugate of formula (II), Z is a radical of formula (XX), wherein each of the dashed bonds of the alkyl chain attached to the chroman ring is a single bond, and $R_8$, $R_9$ and $R_{10}$ are selected from the following meanings: (i) $R_8$, $R_9$ and $R_{10}$ are methyl, (ii) $R_8$, $R_{10}$ are methyl and $R_9$ is hydrogen, (iii) $R_9$ and $R_{10}$ are methyl and $R_8$ is hydrogen, and (iv) $R_{10}$ is methyl and $R_8$ and $R_9$ are hydrogen. More particularly, in the above embodiment $R_8$, $R_9$ and $R_{10}$ are methyl.

In a more particular embodiment of the previous one, $R_8$, $R_9$ and $R_{10}$ are methyl. More particularly, in the conjugate of formula (II), Z is a radical of formula (XXa):

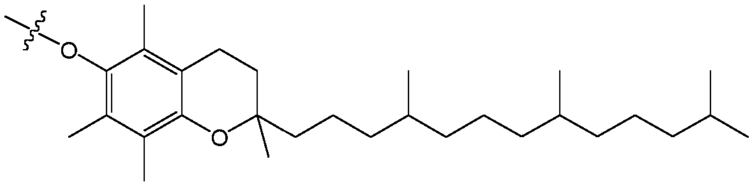

(XXa)

and even more particularly Z is a radical of formula (XXb):

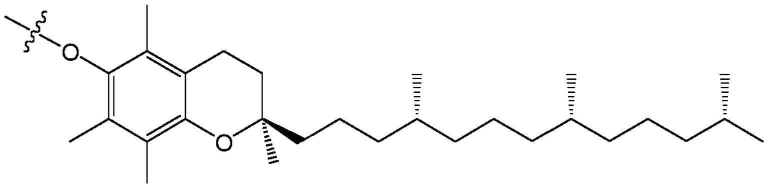

(XXb)

The conjugates of formula (II) may be prepared by processes well-known in the art. These processes also form part of the invention. For example, when the repeating sarcosine-based unit PAA has the formula (XXI), s is 0, and X is an amine or alcohol group, Z can be used as initiator in the ROP for the preparation of polysarcosine-based moiety as shown in the scheme below:

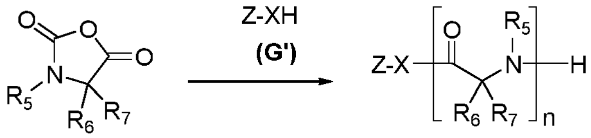


(F) (B')

This reaction can be carried out under the conditions previously mentioned for the preparation of polymers (B).

The obtained polysarcosine derivative (B') may be attached to the vitamin E derivative through a linker L' by any of the processes disclosed for the preparation of the polymers of formula (I).

When s is 1, and X is an amine or alcohol group, Z can be used as initiator in the ROP for the preparation of polysarcosine-based moiety as shown in the scheme below:

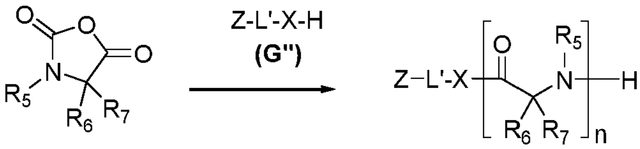


(F) (B")

This reaction can be carried out under the conditions previously mentioned for the preparation of polymers (B).

The obtained polysarcosine derivative (B") may be attached to the vitamin E derivative through a linker L by any of the processes disclosed for the preparation of the polymers of formula (I).

Conjugates of formula (II) wherein the repeating sarcosine-based unit PAA has the formula (XXII) may be prepared by procedures as the ones described above for the compounds of formula (Ib).

Self-assembled particles

Another aspect of the invention relates to a self-assembled particle comprising:

(a) the polymer as defined herein; or alternatively
(b) the conjugate of formula (II) as defined herein; or alternatively
(c) a combination of the polymer as defined herein and the conjugate of formula (II),

and optionally an agent encapsulated within the self-assembled nano/microparticles self-assembled particle which is selected from the group consisting of a nutraceutically active agent, a pharmaceutically active agent, a cell-targeting agent, a diagnostically active agent, and a cosmetically active agent.

As used herein, the term "self-assembled particles" refers to a small, enclosed structure of any shape, typically spherical and/or tubular. It intends to encompass any of a number of structures that are known in the art to be formed from amphiphilic polymers. Non-limiting examples of self-assembled particles include micelles (also being referred to interchangeably herein as micellar worms or simply "worms"), inverted micelles, planar bilayers, crystal nanoparticles, liposomes, microbubbles or lipid nanoparticles. The self-assembled nanoparticles and microparticles can also form gels.

The self-assembled particles may be self-assembled nanoparticles or self-assembled microparticles. A "nanoparticle", as defined herein, is any particle having a smallest end-to-end diameter of between 1 and 100 nm in size. A "microparticle", as defined herein, is typically any particle having a smallest end-to-end between 0.1 and 100 $\mu$m in size. Typically, in a composition comprising a plurality of particles, the relevant diameter is the number average diameter.

In the case a) above, the polymer forms the external shell of the self-assembled particle, and an agent is encapsulated or loaded in the inner core. In the case b) above, the conjugate of formula (II) forms the external shell of the self-assembled particle, and an agent is optionally encapsulated, i.e., loaded, in the inner core. In the case c) above, both the polymer as defined herein and the conjugate of formula (II) form the external shell of the self-assembled particle, and an agent is optionally encapsulated or loaded in the inner core or the shell surface.

In one embodiment, optionally in combination with one or more features of the various embodiments described above or below, the self-assembled particle is selected from the group consisting of a micelle, inverted micelle, planar bilayer, crystal nanoparticle, liposome, and lipid nanoparticle.

For the purposes of the invention, the term "micelle" refers to the arrangement of the polysarcosine vitamin E derivatives of the invention in a solvent in which the non-polar tails (hydrophobic part) face inward and the polar head portion face outward. Typically, the micelles show a spherical or tubular shape. In particular, the micelle is a core-shell structure comprising an inner core and an external shell.

According to one embodiment, optionally in combination with one or more features of the various embodiments described above or below, the invention relates to a micelle comprising:

(a) the polymer as defined herein; or alternatively,
(b) the conjugate of formula (II) as defined herein; or alternatively,
(c) a combination of the polymer as defined herein and the conjugate of formula (II);

and optionally an agent encapsulated within the micelle which is selected from the group consisting of a nutraceutically active agent, a pharmaceutically active agent, a cell-targeting agent, a diagnostically active agent, and a cosmetically active agent.

In one embodiment, optionally in combination with one or more features of the various embodiments described above or below, the micelle is formed in water.

In another embodiment, optionally in combination with one or more features of the various embodiments described above or below, the micelle is formed in an organic solvent. More particularly, the organic solvent is selected from the group consisting of acetone, tetrahydrofuran, methanol, ethanol, acetonitrile and mixtures thereof.

In one embodiment, optionally in combination with one or more features of the various embodiments described above or below, the micelles have a hydrodynamic diameter (Dh) in water, as measured by diffusion-ordered NMR spectroscopy (DOSY) or alternatively measured by Dynamic Light Scattering (DLS), from 5 to 500 nm.

According to another embodiment, optionally in combination with one or more features of the various embodiments described above or below, the micelles have a critical micelle concentration (CMC) in water at 25 °C, atmospheric pressure, from 0.01 to 10 mg/mL.

According to another embodiment, optionally in combination with one or more features of the various embodiments described above or below, the encapsulation efficiency of the polymer of formula (I) is from 0.1 to 100 wt%, more particularly from 15 to 60 wt%. The encapsulation efficiency is calculated by the following formula:

$$\text{Encapsulation efficiency (wt\%)} = \frac{\text{(total agent added – free non-entrapped agent) (by weight)}}{\text{total agent added (by weight)}}$$

The micelles may be prepared by different methods which are well-known for a person skilled in the art, such as for example direct formulation, co-solvent formulation, thin-film formulation, formulation in supercritical $CO_2$, and the like.

It also forms part of the invention a process for the preparation of the micelle as defined herein, which comprises either a) dissolving the polymer of the invention, the conjugate of formula (II) or combination thereof as defined herein, and optionally an agent to be encapsulated within the micelle in water, or alternatively b) dissolving the polymer of the invention, the conjugate of formula (II) or combination thereof as defined herein, and optionally an agent to be encapsulated within the micelle in an organic solvent to obtain a solution, add this solution to water and evaporate the organic solvent, or alternatively c) dissolving the polymer of the invention, the conjugate of formula (II) or combination thereof as defined herein, and optionally an agent to be encapsulated within the micelle in an organic solvent to obtain a solution, evaporate the organic solvent to obtain a film, and dissolve the obtained film with water. The present invention also relates to the micelle obtainable by the above process.

The term "obtainable" is used herein for defining the micelle by its preparation process. For the purposes of the invention, the expressions "obtainable", "obtained" and similar equivalent expressions are used interchangeably and, in any case, the expression "obtainable" encompasses the expression "obtained".

According to one embodiment, optionally in combination with one or more features of the various embodiments described above or below, the invention relates to a liposome comprising:

(a) the polymer as defined herein; or alternatively,
(b) the conjugate of formula (II) as defined herein; or alternatively,
(c) a combination of the polymer as defined herein and the conjugate of formula (II);

and optionally an agent encapsulated within the micelle which is selected from the group consisting of a nutraceutically active agent, a pharmaceutically active agent, a cell-targeting agent, a diagnostically active agent, and a cosmetically active agent.

According to one embodiment, optionally in combination with one or more features of the various embodiments described above or below, the invention relates to a lipid nanoparticle (LNP) comprising:

(a) the polymer as defined herein; or alternatively,
(b) the conjugate of formula (II) as defined herein; or alternatively,
(c) a combination of the polymer as defined herein and the conjugate of formula (II);

and optionally an agent encapsulated within the micelle which is selected from the group consisting of a nutraceutically active agent, a pharmaceutically active agent, a cell-targeting agent, a diagnostically active agent, and a cosmetically active agent.

For the purposes of the invention, the term "liposome" refers to artificially prepared self-assembled particles composed of concentric lipidic bi-layers enclosing one or more aqueous compartments. Non-limiting examples of liposomes include monolamellar liposome, multi-lamellar liposomes, multi-vesicular-liposomes and polymer-coated liposomes.

The term "lipid nanoparticle" refers to particles typically spherical on the order of nanometers (e.g., 1-1000 nm) that includes lipids and that is stable and dispersible in aqueous media.

According to one embodiment, optionally in combination with one or more features of the various embodiments described above or below, the invention relates to a crystal nanoparticle comprising:

(a) the polymer as defined herein; or alternatively,
(b) the conjugate of formula (II) as defined herein; or alternatively,
(c) a combination of the polymer as defined herein and the conjugate of formula (II);

and optionally an agent encapsulated within the micelle which is selected from the group consisting of a nutraceutically active agent, a pharmaceutically active agent, a cell-targeting agent, a diagnostically active agent, and a cosmetically active agent.

For the purposes of the invention, the term "crystal nanoparticle" refers to artificially prepared crystals composed of solid lipids enclosing one or more hydrophobic compartments.

The term "crystal nanoparticle" refers to particles of different shapes on the order of nanometers (e.g., 1-1000

nm) that includes lipids and that is stable and dispersible in aqueous media.

The liposomes and lipid nanoparticles disclosed herein may comprise any suitable lipid, including ionizable lipids, cationic lipids, zwitterionic lipids, neutral lipids, or anionic lipids.

Suitable ionizable lipids include, without limitation, (2S)-2,5- bis(3-aminopropylamino)- N-[2-(dioctadecylamino) acetyl]pentanamide (DOGS), N1-[2-((1S)-1-[(3-aminopropyl)amino]-4-[di(3-aminopropyl)amino]butylcarboxamido) ethyl]-3,4-di[oleyloxy]-benzamide (MVL5), 3β-[N-(N',N'-dimethylaminoethane)-carbamoyl]cholesterol (DC- Cholesterol), (1,2-distearyloxy-N,N-dimethyl-3-aminopropane (DSDMA), 1,2-dilinoleyloxy-N,N-dimethyl-3-aminopropane (DLin-DMA), 1,2-dilinolenyloxy-N,N-dimethyl-3-aminopropane (DLenDMA), 1,2-di-y-linolenyloxy-N,N-dimethylaminopropane (γ-DLenDMA), 2-dilinoleyl-4-dimethylaminomethyl-[1,3]-dioxolane (DLin-K-DMA), 2,2- dilinoleyl-4- dimethylaminoethyl-[1,3]-dioxolane (DLin- KC2-DMA, also known as DLin-C2K-DMA, XTC2, and C2K), 2,2-Dilinoleyl-4-(3-dimethylaminopropyl)-[1,3]-dioxolane (DLin-KC3-DMA), 2,2-Dilinoleyl-4-(4-dimethylaminopropyl)-[1,3]-dioxolane (DLin-KC4-DMA), 1,2-dilinoleny loxy-4-(2-dimethylaminoethyl)-1,3-dioxolane (DLen-C2K-DMA), 1,2-di- y-linolenyloxy-4-(2-dimethylaminoethyl)-1,3-dioxolane (y-DLen-C2K-DMA), (6Z,9Z,28Z,31Z)-Heptatriaconta-6,9,28,31-tetraen-19-yl 3-(dimethylamino)propanoate (DLin-M-C2-DMA, also known as MC2), (6Z,9Z,28Z,31Z)- heptatriaconta-6,9,28,31- tetraen-19- yl 4-(dimethylamino)butanoate (DLin-M-C3-DMA, also known as MC3) and 3-((6Z,9Z,28Z,31Z)-heptatriaconta-6,9,28,31-tetraen-19-yloxy)-N,N-dimethylpropan-1-amine (DLin-MP-DMA, also known as 1-B11).

Suitable cationic lipids include, without limitation, N,N-dioleyl-N,N-dimethylammonium chloride (DODAC), N,N-distearyl-N,N-dimethylammonium bromide (DDA13), N-(1-(2,3-dioleoyloxy)propyl)-N,N,N-trimethylammonium chloride (DOTAP), N-(1-(2,3-dioleyloxy)-propyl)-N,N,N-trimethylammonium chloride (DOTMA), N-[1-(2,3,-ditetradecyloxy)propyll-N,N-dimethyl-N-hydroxyethylammonium bromide (DMRIE), N-[1-(2,3,dioleyloxy)propyl]-N,N-dimethyl-N-hydroxy ethylammonium bromide (DORIE), 3β-[N-(N',N'-dimethylamino-ethane)-carbamoyl]cholesterol (DC-Chol), dimethyldioctadecylammonium (DDAB), 2,3-dioleyloxy- N-[2-(sperminecarboxamido)ethyl]- N,N- dimethyl-1- propanaminium trifluoroacetate (DOSPA), ethylphosphatidylcholine, (ePC), and N,N-dimethyl-2,3-dioleyloxy)propylamine (DODMA).

Examples of anionic lipids include, but are not limited to, phosphatidylglycerol, diacylphosphatidylserine, diacylphosphatidic acid, N-Succinyl phosphatidylethanolamine, N-glutaryl phosphatidylethanolamine cholesterol hemisuccinate (CHEMS), lysylphos-phatidylglycerol, N-dodecanoyl phosphatidyl ethanoloamine, cardiolipin, and combinations thereof.

Suitable neutral lipids may be uncharged or zwitterionic lipids and include, without limitation steroids, phospholipids, and combinations thereof.

Examples of steroids include, without limitation, cholesterol, progesterone, cortisone, aldosterone, estradiol, testosterone, and combinations thereof. Examples of phospholipids include, but are not limited to, phosphatidylcholine (PC), phosphatidic acid (PA), phosphatidylethanolamine (PE), phosphatidylglycerol (PG), phosphatidylserine (PS), and phosphatidylinositol (PI), dimyristoyl phosphatidyl choline (DMPC), distearoyl phosphatidyl choline (DSPC), dioleoyl phosphatidyl choline (DOPC), dipalmitoyl phosphatidyl choline (DPPC), dimyristoyl phosphatidyl glycerol (DMPG), distearoyl phosphatidyl glycerol (DSPG), dioleoyl phosphatidyl glycerol (DOPG), dipalmitoyl phosphatidyl glycerol (DPPG), dimyristoyl phosphatidyl serine (DMPS), distearoyl phosphatidyl serine (DSPS), dioleoyl phosphatidyl serine (DOPS), dipalmitoyl phosphatidyl serine (DPPS), dioleoyl phosphatidyl ethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyp-cyclohexane-1-carboxylate (DOPE-mal), dipalmitoyl phosphatidyl ethanolamine (DPPE), dimyristoylphosphoethanolamine (DMPE), distearoylphosphatidyl-ethanolamine (DSPE); lysophosphatidyl choline, lysophosphatidylethanolamine, 1,2-distearoyl-sn-glycero-3-phosphocholine (DAPC), dilauryloylphosphatidylcholine (DLPC), 1-myristoyl-2-palmitoyl phosphatidylcholine (MPPC), 1-palmitoyl-2-myristoyl phosphatidylcholine (PMPC), 1-palmitoyl-2-stearoyl phosphatidylcholine (PSPC), 1,2-diarachidoyl-sn-glycero-3-phosphocholine (DBPC), 1-stearoyl-2-palmitoyl phosphatidylcholine (SPPC), 1,2-dieicosenoyl-sn-glycero3-phosphocholine (DEPC),and combinations thereof.

The polymers of the invention as well as their conjugates may be used as stealth lipids in lipid nanoparticles.

According to one embodiment, optionally in combination with one or more features of the various embodiments described above or below, the invention relates to a lipid nanoparticle (LNP) or a liposome comprising the polymer, the conjugate of formula (II) or a combination of both, further comprises one or more of an ionisable lipid, a cationic lipid, a neutral lipid, and an anionic lipid. More particularly, the LNP further comprises one or more nucleic acids as previously defined.

Compositions

The present invention also relates to a composition comprising an effective amount (a) the polymer of the invention as defined herein, or alternatively, (b) the conjugate of formula (II) as defined herein, or alternatively, (c) a combination of the polymer of the invention and the conjugate of formula (II), or alternatively, (d) the self-assembled particles as defined herein, together with one or more acceptable excipients or carriers.

The term "effective amount" as used herein refers to the amount of each one of the components of the

composition of the present invention which provides a beneficial effect after its application.

In one embodiment, optionally in combination with one or more features of the various embodiments described above or below, the composition is a nutraceutical supplement comprising a nutraceutically effective amount of (a) the polymer of the invention as defined herein, or alternatively, (b) the conjugate of formula (II) as defined herein, or alternatively, (c) a combination of the polymer as defined herein and the conjugate of formula (II), or alternatively, (d) the self-assembled particles as defined herein, together with one or more nutraceutically acceptable excipients or carriers, wherein the encapsulated agent in the self-assembled particles, when present, is a nutraceutically active agent, and in the conjugate of formula (II), when present, Z is a nutraceutically active agent.

In the context of the present invention, the terms "nutraceutical supplement", "food supplement", "dietary supplement" or "nutritional supplement" as used herein interchangeably and refer to a preparation intended to supplement the diet and provide nutrients that may be missing or may not be consumed in sufficient quantity in a person's diet. The term "nutraceutically acceptable" excipients or carriers refers to components that may be consumed by humans without significant deleterious health consequences.

In another embodiment, optionally in combination with one or more features of the various embodiments described above or below, the composition is a pharmaceutical composition comprising a therapeutically effective amount of (a) the polymer as defined herein, or alternatively, (b) the conjugate of formula (II) as defined herein, or alternatively, (c) a combination of the polymer as defined herein and the conjugate of formula (II), or alternatively, (d) the self-assembled particles as defined herein, together with one or more pharmaceutically acceptable excipients or carriers, wherein the encapsulated agent in the self-assembled particles, when present, is a pharmaceutically active agent or a cell-targeting agent, and in the conjugate of formula (II), when present, Z is a pharmaceutically active agent or a cell-targeting agent.

The expression "therapeutically effective amount" as used herein, refers to the amount of a polymer that, when administered, is sufficient to prevent development of, or alleviate to some extent, one or more of the symptoms of the disease which is addressed. The specific dose of the polymer of the invention to obtain a therapeutic benefit may vary depending on the particular circumstances of the individual patient including, among others, the size, weight, age and sex of the patient, the nature and stage of the disease, the aggressiveness of the disease, and the route of administration.

For the purposes of the present invention, the term "pharmaceutically acceptable" excipients or carriers refers to components which are appropriate for use in pharmaceutical technology for the preparation of compositions for medical use. Each component should be "acceptable" in the sense of being compatible with the other ingredients of the composition. When used in contact with the tissue or organ of humans and animals should not have excessive toxicity, irritation, allergic response, immunogenicity, or other problems or complications, commensurate with a reasonable benefit/risk ratio.

In one embodiment, optionally in combination with one or more features of the various embodiments described above or below, the composition is a diagnostic composition comprising a diagnostically effective amount of (a) the conjugate of formula (II) as defined herein, or alternatively, (b) the self-assembled particles as defined herein, with the condition that when the self-assembled particles comprise the polymer of the invention in the absence of a conjugate of formula (II), an encapsulated agent is present within the self-assembled particles; together with one or more diagnostically acceptable excipients or carriers, wherein the encapsulated agent in the self-assembled particles, when present, is a diagnostically active agent, and in the conjugate of formula (II), when present, Z is a diagnostically active agent.

The term "diagnostic composition" refers to a composition suitable for use in diagnostic, particularly in imaging diagnostic technology. The term "diagnostically effective amount" as used herein, refers to the effective amount of a detection polymer that, when administered, is sufficient for the diagnosis of a disease or disorder; particularly as imaging diagnostic use as contrast imaging agent. The dose of the detection polymer administered will of course be determined by the particular circumstances surrounding the case, including the polymer administered, the route of administration, the particular condition being diagnosticated, and the similar considerations. The diagnostic composition of the present invention comprises one or more diagnostically acceptable excipients or carriers. The term "diagnostically acceptable" refers to that excipients or carriers suitable for use in the diagnosing technology for preparing compositions with diagnostic use; particularly by imaging diagnostic use. The detection of these diagnostic agents in the body of the patient can be carried out by the well-known techniques used such as in imaging diagnostic with magnetic resonance imaging (MRI) and X-ray.

In one embodiment, optionally in combination with one or more features of the various embodiments described above or below, the composition is a cosmetic composition comprising a cosmetically effective amount of (a) the polymer of formula (I) as defined herein, or alternatively, (b) the conjugate of formula (II) as defined herein, or alternatively, (c) a combination of the polymer of formula (I) and the conjugate of formula (II), or alternatively, (d) the self-assembled particles as defined herein, together with one or more cosmetically acceptable excipients or carriers; wherein the encapsulated agent in the self-assembled particles, when present, is a cosmetically active agent, and in the conjugate of formula (II), when present, Z is a cosmetically active agent.

The term "cosmetically effective amount" as used herein, refers to the effective amount of a cosmetic active agent

that, when administered, is intended to improve its appearance or to beautify, preserve, condition, cleanse, color or protect the skin, nails or hair without non-medical application. The term "cosmetically acceptable" or "dermatologically acceptable" excipients or carriers is used interchangeably in this document and refer to components which are appropriate for use in human skin contact without toxicity, incompatibility, instability, inappropriate allergic response, among others.

The compositions of the invention may be in solid or liquid form. Depending on the desired purpose, the skilled person will know the most appropriate formulation and excipients to be used. Examples of excipients or carriers include, without limitation, diluents, binders, glidants, disintegrants, lubricants colorants, mixtures thereof, and other components known in the state of the art. Any administration route may be used such as e.g. oral, topical, rectal or parenteral route (including subcutaneous, intraperitoneal, intradermal, intramuscular, intravenous route, etc.).

The nutraceutically active agents, pharmaceutically active agents, cell-targeting agents present in the self-assembled particles and compositions of the invention are as defined in the context of the conjugates of formula (II). Thus, all embodiments indicated for the conjugates of formula (II) regarding the active agents also apply to the self-assembled particles and compositions.

Uses

Another aspect of the invention relates to (i) the polymer of formula (I) as defined herein, or alternatively, (ii) the conjugate of formula (II) as defined herein, or alternatively,

(iii) the self-assembled particle as defined herein, or alternatively, (iv) the pharmaceutical composition as defined herein, for use in medicine, wherein the encapsulated agent in the self-assembled particles, when present, is a pharmaceutically active agent or a cell-targeting agent as defined herein, and in the conjugate of formula (II), when present, Z is a pharmaceutically active agent or a cell-targeting agent.

Non-limiting examples of diseases that may be treated and/or prevented by the derivatives of the present invention include neurodegenerative disorders, neurological diseases, cancer, infectious diseases, disorders related to aging, neuro-inflammation, demyelinating disorders, multiple sclerosis, ischemic disorders, ischemia-reperfusion damage, amyloidotic disease, cardiomyopathy, spinal cord injury, immune disorders, inflammatory disorders, rare diseases, wound healing, skin related diseases and lysosomal storage diseases.

Non-limiting examples of neurodegenerative diseases include Alzheimer's disease, Parkinson's disease, amyotrophic lateral sclerosis, cerebral ischaemia, post-encephalitic Parkinsonisms, dystonias, Tourette syndrome, periodic limb movement pathologies, restless legs syndrome, attention deficit hyperactivity disorders, Huntington's disease, progressive supranuclear palsy, Pick's disease, fronto-temporal dementia and neuromuscular diseases.

The term "disorder" as used herein is intended to be generally synonymous, and is used interchangeably with, the terms "disease," "syndrome," and "condition" (as in medical condition), in that all reflect an abnormal condition of the human or animal body or of one of its parts that impairs normal functioning, is typically manifested by distinguishing signs and symptoms.

For example, a conjugate of formula (II) wherein Z is an anticancer agent, or a self-assembled particle which comprises an anticancer agent encapsulated within the self-assembled particle, may be used for the treatment of cancer. Thus, in one embodiment, the invention relates to

(i) the conjugate of formula (II) as defined herein; or alternatively,
(ii) the self-assembled particle as defined herein, with the condition that when the self-assembled particle comprises the polymer of formula (I) in the absence of a conjugate of formula (II), an encapsulated agent is present within the self-assembled particle; or alternatively,
(iii) the pharmaceutical composition as defined herein;

for use in the treatment and/or prevention of cancer, wherein the encapsulated agent in the self-assembled particles, when present, is an anticancer agent, and in the conjugate of formula (II), when present, Z is anticancer agent.

Thus, this aspect of the invention relates to the use of (i) the conjugate of formula (II) as defined herein; or alternatively, (ii) the self-assembled particle as defined herein, with the condition that when the self-assembled particle comprises the polymer of formula (I) in the absence of a conjugate of formula (II), an encapsulated agent is present within the self-assembled particle; or alternatively,

(iii) the pharmaceutical composition as defined herein; for the manufacture of a medicament for the treatment and/or prevention of cancer, wherein the encapsulated agent in the self-assembled particles, when present, is an anticancer agent, and in the conjugate of formula (II), when present, Z is anticancer agent.

It may also be formulated as a method for the treatment and/or prevention of cancer, comprising administering in a subject in need thereof, including a human, an effective amount of (i) the conjugate of formula (II) as defined herein; or alternatively, (ii) the self-assembled particle as defined herein, with the condition that when the self-assembled particle comprises the polymer of formula (I) in the absence of a conjugate of formula (II), an encapsulated agent is present within

the self-assembled particle; or alternatively, (iii) the pharmaceutical composition as defined herein; wherein the encapsulated agent in the self-assembled particles, when present, is an anticancer agent, and in the conjugate of formula (II), when present, Z is anticancer agent.

In the context of the present invention, the terms "treat", "treating" and "treatment", as used herein, refers to ameliorating symptoms associated with a disease or disorder, including preventing or delaying the onset of the disease or disorder symptoms, and/or lessening the severity or frequency of symptoms of the disease or disorder.

Another aspect of the invention relates to (i) the conjugate of formula (II) as defined herein, or alternatively, (ii) the self-assembled particle as defined herein, with the condition that when the self-assembled particle comprises the polymer of formula (I) in the absence of a conjugate of formula (II), an encapsulated agent is present within the self-assembled particle; or alternatively, (iii) the diagnostic composition as defined herein, for use in diagnostics, wherein the encapsulated agent in the self-assembled particles, when present, is a diagnostically active agent, and in the conjugate of formula (II), when present, Z is a diagnostically active agent.

This aspect of the invention relates to the use of (i) the conjugate of formula (II), or alternatively, (ii) the self-assembled particle as defined herein, with the condition that when the self-assembled particle comprises the polymer of formula (I) in the absence of a conjugate of formula (II), an encapsulated agent is present within the self-assembled particle; or alternatively, (iii) the diagnostic composition as defined herein in diagnostics, wherein the encapsulated agent in the self-assembled particles, when present, is a diagnostically active agent, and in the conjugate of formula (II), when present, Z is a diagnostically active agent. It may also be formulated as a method for the diagnostic of a disease or condition, comprising administering (i) the conjugate of formula (II), or alternatively, (ii) the self-assembled particle as defined herein, with the condition that when the self-assembled particle comprises the polymer of formula (I) in the absence of a conjugate of formula (II), an encapsulated agent is present within the self-assembled particle; or alternatively, (iii) the diagnostic composition as defined herein, in a subject in need thereof, including a human, wherein the encapsulated agent in the self-assembled particles, when present, is a diagnostically active agent, and in the conjugate of formula (II), when present, Z is a diagnostically active agent.

The detection of these imaging agents can be carried out by well-known techniques such as imaging diagnostic techniques. Examples of imaging diagnostic techniques suitable for the present disclosure include, but not limited to, are magnetic resonance imaging (MRI), X-ray, positron emission tomography (PET), single-photon emission computed tomography (SPECT), fluorescence microscopy, and in vivo fluorescence.

Another aspect of the invention relates to the use of the polymer of formula (I) as defined herein as a carrier, as a solubilizer, as absorption and permeation enhancer, as emulsifier or as surface stabilizer. When used as a carrier, the polymer of formula (I) may be in the form of self-assembled particles. Thus, it also forms part of the invention the use of the self-assembled particles which comprise the polymer of formula (I) as carriers, solubilizers, absorption and permeation enhancers, emulsifiers or as surface stabilizers.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

## Examples

*Analytical methods*

*NMR spectroscopy*

NMR spectra were recorded at 27 °C (300 K) on a 300 UltrashieldTM from Bruker (Billerica MA, USA). Data were processed with the software Topspin (Bruker GmbH, Karlsruhe, Germany). Samples were prepared at a concentration of 20 - 10 mg/mL approx. in the required solvent.

*Diffusion Experiments*

Pulsed-field gradient NMR spectroscopy was used to measure translational diffusion by fitting the integrals or intensities of the NMR signals to the Stejskal-Tanner equation (Fieber W. et al., Macromolecules 2007, 40(15), 5372-5378): $I= I_0 \exp[-D\gamma^2 g^2 \delta^2(\Delta-\delta/3)]$ where I is the observed intensity, $I_0$ the reference intensity (unattenuated signal intensity), D the diffusion coefficient, $\gamma$ the gyromagnetic ratio of the observed nucleus, g the gradient strength, $\delta$ the length of the gradient, and $\Delta$ the diffusion time. Two-dimensional diffusion-ordered NMR spectroscopy (DOSY) was performed with a stimulated echo sequence using bipolar gradient pulses. The lengths of delays were held constant at $\Delta$= 100 ms, and

32 spectra of 64 scans each were acquired with the strength of the diffusion gradient varying between 5 % and 95 %. The lengths of the diffusion gradient and the stimulated echo were optimized for each sample. Typical values were $\delta$ = 5ms for the analysis of physical mixture species and cross-linked materials at 15 mg/mL. Moreover, the hydrodynamic radius (Rh) in nm through diffusion coefficient was obtained employing the Stokes-Einstein equation. $Rh=(KB \cdot T)/(6\pi \cdot \eta \cdot D)$ where KB is the Boltzman constant ($1.3806488 \cdot 10^{-23}$ $m^2 \cdot Kg/s^2 \cdot K$), T is the temperature (K) and $\eta$ is the dynamic viscosity ($1.095 \cdot 10^3$ Kg/m·s for $D_2O$ at 298.15 K and 0.1 MPa) (Duro-Castano A. et al., Advanced Materials 2017, 29(39), 12).

*Size Exclusion Chromatography (SEC)*

For SEC measurements in dimethylformamide (DMF) containing 0.1 % (w/w) of lithium bromide as an additive, a GPC max (Malvern Instruments) autosampler was used with a flow rate of 0.7 mL/min at 60 °C as an integrated instrument, TSKgel Alpha-4000 from Tosoh. Viscoteck TDATM 302 was used as an integrated detection system (Refractive index). The system was calibrated with polymethyl methacrylate (PMMA) (Mw = 65 kDa; PDI = 1.05) obtained from PSS. For this Mw determination an integrated triple detection system was used [Refractive Index, Light Scattering (two angles: 7 and 90 °) and Differential Pressure Viscosimeter]. Additional SEC detector of UV-Vis was used to measure the alpha-tocopherol absorption at 230 nm.

*Size Distribution*

Dynamic Light Scattering (DLS) measurements were performed using a Malvern Zetasizer NanoZS instrument, equipped with a 532 nm laser at a fixed scattering angle of 173 °. Polymer solutions were prepared under different conditions (MilliQ or KCl 1 mM at different concentrations and temperatures), solutions were sonicated for 10 min and allowed to age for the required time, filtered through a 0.45 $\mu$m cellulose membrane filter and measured. Size distribution was measured (diameter, nm) for each polymer per triplicate with n > 3 measurements, automatic optimization of beam focusing and attenuation was applied for each sample.

*Z-Potential measurements*

Z-potential measurements were performed at 20 °C using a Malvern ZetasizerNanoZS instrument, equipped with a 532 nm laser using disposable folded capillary cells, provided by Malvern Instruments Ltd. (Worcestershire, UK). Polymer solutions were prepared in 1 mM KCl in MilliQ water. The solutions were filtered through a 0.45 $\mu$m cellulose membrane filter. Z-potential was measured for each sample per triplicate with n > 3 measurements.

**EXAMPLE 1A**

*Preparation of polysarcosine-vitamin E derivatives*

*Coupling reagent for aqueous conjugations: 4-(4,6-Dimethoxy-1,3,5-triazin-2-yl)-4-methylmorpholinium chloride (DMTMMCl)*

2-Chloro-4,6-dimethoxy-1,3,5-triazine (CDMT, 50 g, 0.28 mol, 1 eq.) was dissolved in tetrahydrofuran (THF, 150 mL) at r.t. for 30 min. Then, N-methylmorpholine (NMM, 28.8 g, 0.28 mol, 1 eq.) was added slowly into reaction and a white solid was precipitated. The reaction was left 30 min and the product (DMTMMCl) was filtered under vacuum and washed 3 x 100 mL of THF. The white product was dried under vacuum and air current for 16 hours. Yield: 80 - 90%. $^1H$ NMR (300 MHz, $D_2O$) $\delta$ 4.70 (d, J = 11.4 Hz, 2H), 4.22 (d, J = 8.7 Hz, 8H), 4.06 - 3.87 (m, 4H), 3.63 (s, 3H).

*Coupling reagent for organic conjugations: 4-(4,6-Dimethoxy-1,3,5-triazin-2-yl)-4-methylmorpholinium tetrafluoroborate ($DMTMMBF_4$)*

DMTMMCl (25 g, 0.09 mol, 1 eq.) was dissolved in water (50 mL) at r.t. for 10 min. In parallel, sodium tetrafluoroborate ($NaBF_4$, 9.84 g, 0.09 mol, 1 eq.) also was dissolved in water (20 mL). Then, $NaBF_4$ solution was added drop by drop into the DMTMMCl solution and the $DMTMMBF_4$ precipitated. The white product ($DMTMMBF_4$) was filtered at vacuum and washed 3 x 20 mL of water. To remove the water, the wet product was freeze-dried. Yield: 80 - 90%. $^1H$ NMR (300 MHz, $D_2O$) $\delta$ 4.58 - 4.44 (m, 2H), 4.11 - 3.89 (m, 8H), 3.89 - 3.63 (m, 4H), 3.49 - 3.33 (m, 3H). $^{19}F$ NMR (282 MHz, $D_2O$) $\delta$ -150.52 (d, J = 14.3 Hz).

*1.1. Isopropyl-poly(sarcosine) derivatives (iPr-PSar6, iPr-PSar12, iPr-PSar20)*

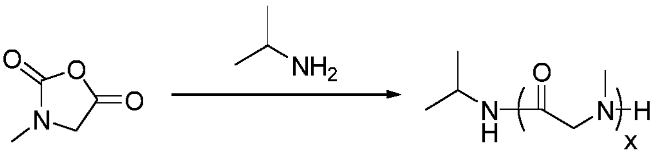

*Scheme 1*

Sarcosine-N-carboxyanhydride (3-methyl-2,5-oxazolidinedione, NCA-Sar (5 g, 43.47mmol) was added to a round bottom flask with a stirrer bar, a stopper and purged with 3 cycles of vacuum/$N_2$, and dissolved in anhydrous DMF (20 mL). Then, the isopropyl amine (DP6: 746.51 μL, 513.5 mg, 8.7 mmol, DP12: 311.05 μL, 213.9 mg, 4.5 mmol, DP20: 186.63 μL, 128.39 mg, 2.17 mmol) was dissolved in DMF (4 mL) and added to the reaction mixture under nitrogen atmosphere. Once the initiator was added, the round bottom flask was immediately connected to an air-lock allowing the $CO_2$ escape. The mixture was stirred at 10 °C until the total consumption of NCA-Sar confirmed by Fourier transform infrared (FTIR) spectroscopy (disappearance of NCA signals at 1853 and 1786 $cm^{-1}$). Upon completion, the reaction mixture became slightly yellow. The reaction mixture was poured into diethyl ether (1:10 to DMF:diethyl ether) to precipitate the product. The precipitate was isolated by centrifugation (3750 rpm, 4 min), washed two times with diethyl ether and dried under vacuum. For further purification, the obtained solid was redissolved in methanol at 500 mg/mL and then the solution was poured again into diethyl ether (1:10 to methanol:diethyl ether), washed two times with diethyl ether and dried under vacuum at least for 2 hours. Finally, the solid is redissolved in distilled water, freezed and lyophilized. $iPrPSar_n$ was isolated as an off-white solid. Yield: 70-90% $^1H$ NMR (MeOD): δ = 1.16 (m, 6H, $CH_3$), 2.40 (broad s, 3H, terminal N-CH3), 2.89-3.11 (broad m, 65H, polymer N-CH3), 3.50 (broad s, 2H, terminal $-CH_2-$) & 3.88-4.42 (broad m, 43H, $-CH_2-$).

The obtained products were analyzed in terms of Mn distribution and DP by SEC and $^1H$ NMR. Results are shown in table 1 below:

Table 1

| Polymer | DP (theoretical) | DP by NMR in MeOD[b] | Mn (Da) by SEC[a] | DP by SEC[a] | PDI by SEC[a] |
|---|---|---|---|---|---|
| iPrPSar(6) | 6 | 6 | 688 | 9 | 1.049 |
| iPrPSar(12) | 12 | 15 | 1324 | 18 | 1.037 |
| iPrPSar(20) | 20 | 22 | 2103 | 29 | 1.019 |
| *[a]Absolute Mw was determined by SEC-RI-MALS in DMF containing LiBr at 0.1%, [b]1H NMR in MeOD at 10 mg/mL.* | | | | | |

*1.2. a-tocopherol-succinate-poly(sarcosine) (iPrPSar(6)-succ-VitE, iPrPSar(12)-succ-VitE, iPrPSar(20)-succ-VitE)*

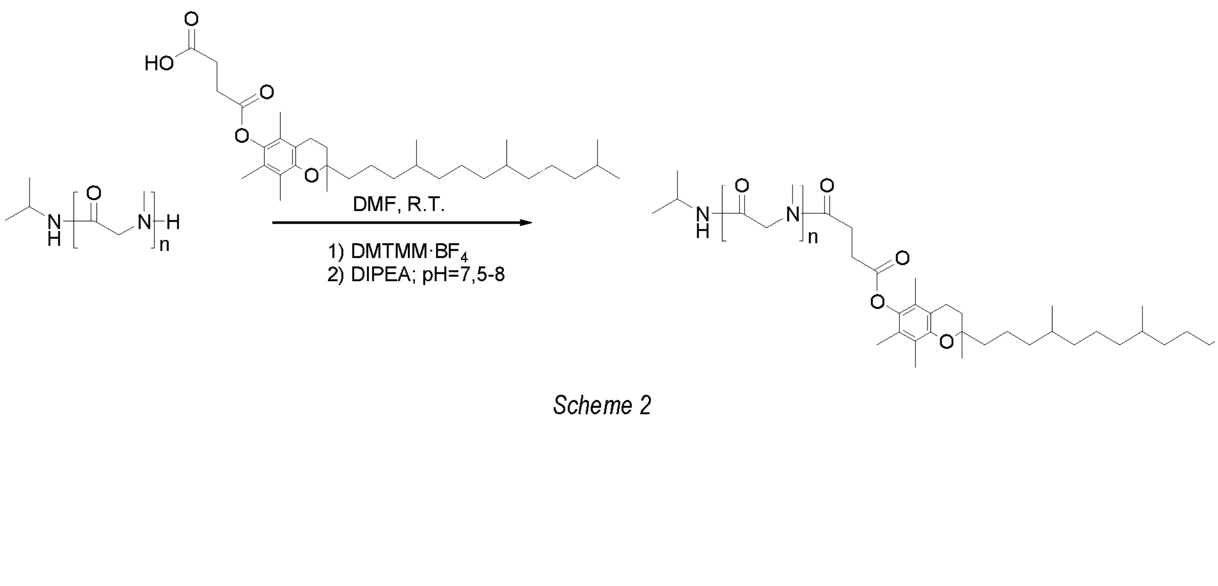


*Scheme 2*

Tocopherol succinate (DP6: 4.925 g, 9.28 mmol; DP12: 2.621 g, 4.94 mmol; DP20: 2.239 g, 4.22 mmol) was added to a round bottom flask with a stirrer bar, a stopper and purged with 3 cycles of vacuum/$N_2$, dissolved in anhydrous DMF (mL) and covered to avoid the direct light contact. Then, DMTMM·$BF_4$ coupling agent was added to a round bottom flask with a stirrer bar, a stopper and purged with 3 cycles of vacuum/$N_2$, and dissolved in anhydrous DMF (mL). DMTMM·BF4 solution was added over tocopherol succinate solution under inert atmosphere and let to react at r.t. for 30 min with stirring. A few drops of N,N-diisopropylethylamine (DIPEA) were added until pH rising to 6-7. Afterwards, iPrPSar(6) (3 g, 6.19 mmol), iPrPSar(12) (3 g, 3.29 mmol) or iPrPSar(20) (4 g, 2.81 mmol) was added to a round bottom flask with stirrer bar, a stopper and purged with 3 cycles of vacuum/$N_2$, and dissolved in anhydrous DMF (mL). After the 30-minutes, the iPrPSar solution was added over the reaction mixture under inert atmosphere. pH adjustment to 8-9 was performed with DIPEA addition. The mixture was stirred at r.t. for 48 hours at r.t avoiding direct light contact. The amphiphile polymer was poured into diethyl ether (1:10 DMF:diethyl ether) to precipitate the product. The precipitate was isolated by centrifugation (3750 rpm, 4 min) washed two times with diethyl ether and dried under vacuum. For further purification, the obtained solid was redissolved in methanol at 500 mg/mL and then the solution was poured again into diethyl ether (1:10 to methanol:diethyl ether), washed two times with diethyl ether and dried under vacuum at least for 2 hours. Finally, the solid was dissolved in distilled water and diafiltrated in a tangential flow filtration system with the addition of some drops of HCl for total DIPEA purification. The solution was freezed and lyophilized to obtain an off-white solid. Yield: 70-90% $^1H$ NMR (MeOD): $\delta = 0.90$ (m, 12H, CH-$CH_3$), 1.04-1.63 (broad m, 30H), 1.83 (m, 2H, -$CH_2$-$CH_2$-CCO), 1.98 (s, 3H, $CH_3$-), 2.02 (s, $CH_3$-), 2.09 (s, $CH_3$-), 2.63 (t, 2H, -$CH_2$-$CH_2$-C=C), 2.86-3.15 (broad s, 71H, N-$CH_3$), 3.92-4.55 (broad s, 46H).

The obtained product analyzed in terms of Mn distribution and DP by SEC, and $^1H$ NMR. Results are shown in table 2 below:

Table 2

| Polymer | DP (theoretical) | DP by NMR in MeOD[b] | Mn (Da) by SEC[a] | DP by SEC[a] | PDI by SEC[a] |
|---|---|---|---|---|---|
| **iPrPSar(6)-succ-VitE** | 6 | 7 | 1259 | 9 | 1.049 |
| **iPrPSar(12)-succ-VitE** | 12 | 14 | 1802 | 17 | 1.035 |
| **iPrPSar(20)-succ-VitE** | 20 | 22 | 2442 | 26 | 1.019 |
| *[a]Absolute Mn was determined by SEC-RI-MALS in DMF containing LiBr at 0.1%, [b]1H NMR in MeOD at 10 mg/mL.* | | | | | |

**EXAMPLE 1B**

*1.1. Functionalized-poly(sarcosine) derivatives (TLR-7, 8-PSar20)*

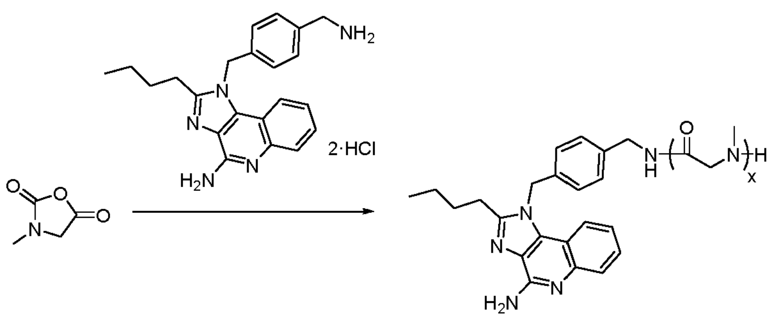


*Scheme 3*

Sarcosine-N-carboxyanhydride (3-methyl-2,5-oxazolidinedione, NCA-Sar (0.5 g, 4.34 mmol) was added to a round bottom flask with a stirrer bar, a stopper and purged with 3 cycles of vacuum/$N_2$, and dissolved in anhydrous DMSO (3 mL). Then, the 1-[[4-(aminomethyl)phenyl]methyl]-2-butyl-imidazo[4,5-C]quinolin-4-amine (DP20: 93.99 mg, 0.22 mmol) was dissolved in DMSO (1 mL), treated with N,N-diisopropylethylamine (75.67 μL, 56.14 mg, 0.43 mmol) and added to the reaction mixture under nitrogen atmosphere. Once the initiator was added, the round bottom flask was immediately connected to an air-lock allowing the $CO_2$ escape. The mixture was stirred at room temperature until the total consumption of NCA-Sar confirmed by Fourier transform infrared (FTIR) spectroscopy (disappearance of NCA signals at 1853 and 1786 $cm^{-1}$). Upon completion, the reaction mixture became slightly yellow. The reaction mixture was poured into

tetrahydrofuran (1:10 to DMSO:tetrahydrofuran) to precipitate the product. The precipitate was isolated by centrifugation (3750 rpm, 4 min), washed two times with tetrahydrofuran and dried under vacuum. For further purification, the obtained solid was redissolved in methanol at 200 mg/mL and then the solution was poured again into diethyl ether (1:10 to methanol:diethyl ether), washed two times with diethyl ether and dried under vacuum at least for 2 hours. Finally, the solid is redissolved in distilled water, freezed and lyophilized. TLR-7,8PSar$_{20}$ was isolated as an off-white solid. Yield: 70-90%. $^1$H NMR (MeOD): δ = 0.95 (t, 3H, $CH_3$-), 1.46 (m, 2H, -$CH_2$-), 1.84 (m, 2H, -$CH_2$-*)*, 2.72 (broad t, 5H, terminal N-$CH_3$ & -$CH_2$-), 2.82-3.13 (broad m, 71 H, terminal *N*-$CH_3$), 3.89 (broad s, 2H, terminal -$CH_2$-), 3.98-4.50 (broad m, 50H, -$CH_2$- & -$CH_2$-), 5.93 (s, 2H, -$CH_2$-*)*, 7.05 (broad d, 2H, H-), 7.30 (broad m, 3H, H-), 7.60 (t, 1H, H-) 7.75 (d, 1H, H-) & 7.95 (d, 1H, H-).

The obtained product was analyzed in terms of Mn distribution and DP by SEC and $^1$H NMR. Results are shown in table 3 below:

Table 3

| Polymer | DP (theoretical) | DP by NMR in MeOD[b] | Mn (Da) by SEC[a] | DP by SEC[a] | PDI by SEC[a] |
|---|---|---|---|---|---|
| TLR-7,8PSar(20) | 20 | 25 | 2578 | 31 | 1.016 |
| [a]*Absolute Mw was determined by SEC-RI-MALS in DMF containing LiBr at 0.1%,* [b]*1H NMR in MeOD at 10 mg/mL.* | | | | | |

*1.2. α-tocopherol-succinate-functionalized-poly(sarcosine) (TLR-7,8-PSar(20)-succ-tocopherol)*

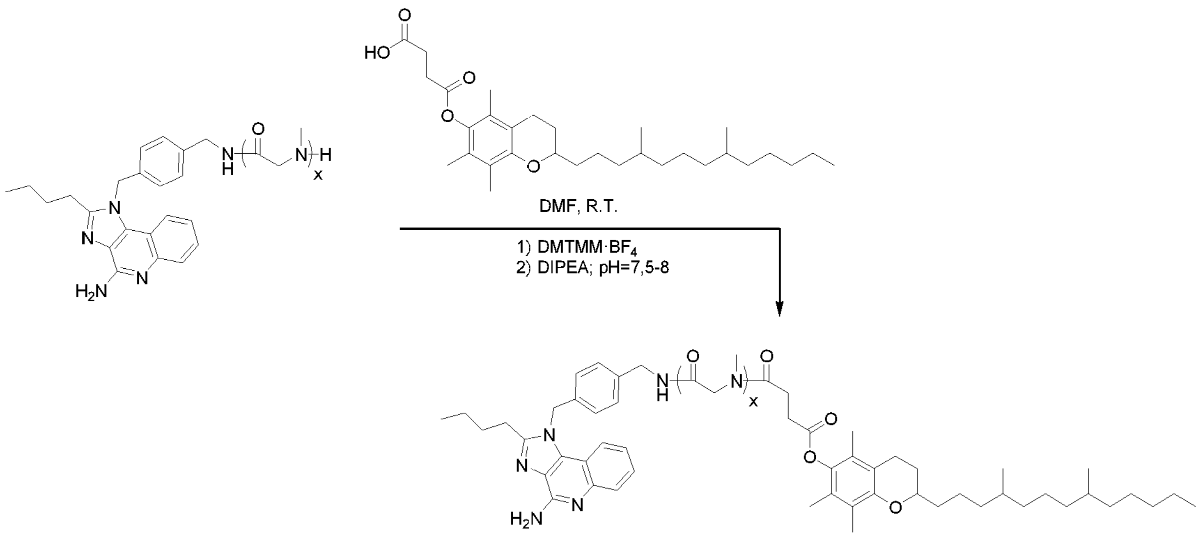


Tocopherol succinate (67.22 mg, 0.13 mmol) was added to a round bottom flask with a stirrer bar, a stopper and purged with 3 cycles of vacuum/$N_2$, dissolved in anhydrous DMF (1 mL) and covered to avoid the direct light contact. Then, DMTMM·$BF_4$(41.55 mg, 0.13 mmol) coupling agent was added to a round bottom flask with a stirrer bar, a stopper and purged with 3 cycles of vacuum/$N_2$, and dissolved in anhydrous DMF (1 mL). DMTMM·BF4 solution was added over tocopherol succinate solution under inert atmosphere and let to react at r.t. for 30 min with stirring. A few drops of N,N-diisopropylethylamine (DIPEA) were added until pH rising to 6-7. Afterwards, TLR-7,8-PSar(20) (0.15 g, 0.08 mmol) was added to a round bottom flask with a stirrer bar, a stopper and purged with 3 cycles of vacuum/$N_2$, and dissolved in anhydrous DMF (2 mL). After the 30-minutes, the iPrPSar solution was added over the reaction mixture under inert atmosphere. pH adjustment to 8-9 was performed with DIPEA addition. The mixture was stirred at r.t. for 48 hours at r.t avoiding direct light contact. The amphiphile polymer was poured into diethyl ether (1:10 DMF:diethyl ether) to precipitate the product. The precipitate was isolated by centrifugation (3750 rpm, 4 min) washed two times with diethyl ether and dried under vacuum. For further purification, the obtained solid was redissolved in methanol at 500 mg/mL and then the solution was poured again into diethyl ether (1:10 to methanol:diethyl ether), washed two times with diethyl ether and dried under vacuum at least for 2 hours. Finally, the solid was dissolved in distilled water and purified in a ultrafiltration unit with the addition of some drops of HCl for total DIPEA removal. The solution was freezed and lyophilized to obtain an off-white solid.

Yield: 70-90% $^1$H NMR (MeOD): δ = 0.87(m, 12H, CH-$CH_3$), 0.94 (t, 3H, $CH_3$-), 1.02-1.60 (broad m, 32H), 1.80 (m, 4H, -$CH_2$-*)*, 1.98 (s, 3H, $CH_3$-), 2.02 (s, 3H, $CH_3$-), 2.09 (s, 3H, $CH_3$-), 2.59 (broad s, 2H, -$CH_2$-), 2.76-3.15 (broad m, 84 H, terminal *N*-$CH_3$), 3.80-4.57 (broad m, 57H, -$CH_2$- & -$CH_2$-), 5.89 (s, 2H, -$CH_2$-), 7.03 (broad d, 2H, H-), 7.27 (broad m,

3H, H-), 7.55 (t, 1H, H-) 7.70 (d, 1H, H-) & 7.91 (d, 1H, H-).

The obtained product analyzed in terms of Mn distribution and DP by SEC, and $^1$H NMR. Results are shown in table 4 below:

Table 4

| Compound | DP (theorical) | DP by NMR in MeOD[b] | Mn (Da) by SEC[a] | DP by SEC[a] | PDI by SEC[a] |
|---|---|---|---|---|---|
| **TLR-7,8-PSar(20)-succ-tocopherol** | 20 | 28 | 3598 | 38 | 1.063 |
| *[a]Absolute Mn was determined by SEC-RI-MALS in DMF containing LiBr at 0.1%, [b]1H NMR in MeOD at 10 mg/mL.* | | | | | |

**EXAMPLE 1C**

*1.1. Preparation of VitE-Gly-OtBu:*

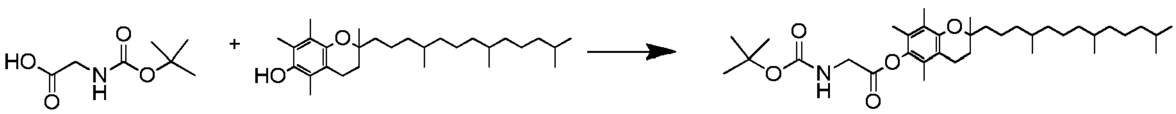

α-tocopherol (2000 mg, 4.64 mmol), Boc-Gly-OH (813 mg, 4.64 mmol) and DMAP (58.48 mg, 0.48 mmol) were added to a round bottom flask with a stirrer bar, a stopper and purged with 3 cycles of vacuum/$N_2$, dissolved in anhydrous dichloromethane (16 mL). Then, DCC (958.09 mg, 4.64 mmol) was dissolved in 4 mL of anhydrous dichloromethane and added over the mixture solution. The solution turned from yellow transparent solution into white cloudy appearance. Reaction mixture was left stirring at RT for 16 h.

The resulting solution was left for 45 min at -20°C, and later it was filtered off and the dichloromethane was removed by evaporation. Afterwards, the oil obtained was purified by silica gel column chromatography with hexane:ethyl acetate (95:5). The product Boc-Gly-VitE fractions were collected, concentrated and finally dried under vacuum.

Yield: 90 %

$^1$H NMR (MeOD): δ = 0.90 (m, 12H, CH-*$CH_3$*), 1.04-1.63 (broad m, 24H), 1.45 (s, 9H, *$(CH_3)_3$-C-*), 1.83 (m, 2H, -$CH_2$-*$CH_2$*-CCO), 1.98 (s, 3H, $CH_3$-), 2.02 (s, 3H, $CH_3$-), 2.09 (s, 3H, $CH_3$-), 2.63 (t, 2H, -$CH_2$-*$CH_2$*-C=C), 4.08 (s, 2H, NH-*$CH_2$*-CCO).

*1.2. Deprotection of VitE-Gly:*

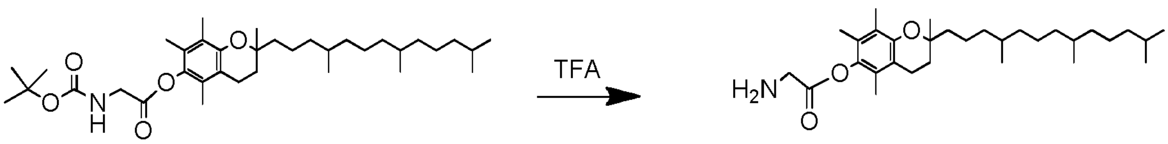


Boc-Gly-VitE (3000 mg, 5.1 mmol), was dissolved in 15.5 mL of dichoromethane and then 5.1 mL of dichoromethane:TFA 1:1. The mixture reaction was left stirring for 30 min at 4°C, and after, 1 h at RT.

Afterwards, solvents are removed by evaporation to obtain a brownish oil left for 24 h at -20°C. This oil was dissolved in MeOH at a concentration of 250 mg/mL and precipitated in 150 mL $H_2O$. Finally, the product was lyophilized obtaining a white powder.

Yield: 56 %

$^1$H NMR (MeOD): δ = $^1$H NMR (MeOD): δ = 0.90 (m, 12H, CH-*$CH_3$*), 1.04-1.63 (broad m, 24H), 1.83 (m, 2H, - $CH_2$-*$CH_2$*-CCO), 1.98 (s, 3H, $CH_3$-), 2.02 (s, 3H, $CH_3$-), 2.09 (s, 3H, $CH_3$-), 2.63 (t, 2H, -$CH_2$-*$CH_2$*-C=C), 4.14 (s, 2H, NH-*$CH_2$*-CCO).

*1.3. ROP polymerization to obtain VitE-Gly-PSar(x):*

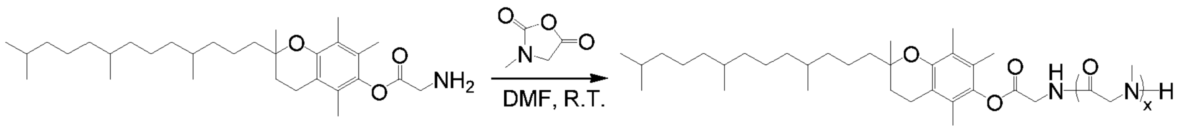


VitE-Gly-$NH_2$ (For DP x=20: 529.7 mg; x=50: 423.8 mg) was purged with 3 cycles of vacuum/$N_2$, and dissolved in anhydrous DMF (For DP x=20: 3 mL; x=50: 2 mL).

Sarcosine-N-carboxyanhydride (3-methyl-2,5-oxazolidinedione, NCA-Sar (For DP x=20: 2500 mg, 21.72 mmol; x=50: 5000 mg, 43.44 mmol) was added to a round bottom flask with a stirrer bar, a stopper and purged with 3 cycles of vacuum/$N_2$, and dissolved in anhydrous DMF (For DP x=20: 7 mL; x=50: 18 mL). Then, the corresponding initiator VitE-Gly-$NH_2$ was added to the reaction mixture under nitrogen atmosphere at 10 °C. Once the initiator was added, the round bottom flask was immediately connected to an air-lock allowing the $CO_2$ escape. The mixture was stirred at 10 °C until the total consumption of NCA-Sar confirmed by Fourier transform infrared (FTIR) spectroscopy (disappearance of NCA signals at 1853 and 1786 $cm^{-1}$). Upon completion, the reaction mixture became slightly yellow. The amphiphile polymer was poured into diethyl ether (1:10 DMF:diethyl ether) to precipitate the product and for further purification, the obtained solid was redissolved in methanol at 500 mg/mL and then the solution was poured again into diethyl ether (1:10 to methanol:diethyl ether). The solid was dried under vacuum and lyophilized, obtaining a white powder as a product.

*VitE-Gly-PSar(20):*

$^1$H NMR (MeOD): δ = 0.90 (m, 12H, CH-$CH_3$), 1.04-1.63 (broad m, 25H), 1.83 (m, 2H, -$CH_2$-$CH_2$-CCO), 1.98 (s, 3H, $CH_3$-), 2.02 (s, 3H, $CH_3$-), 2.09 (s, 3H, $CH_3$-), 2.63 (t, 2H, -$CH_2$-$CH_2$-C=C), 2.86-3.15 (broad s, 81H, N-$CH_3$), 3.92-4.55 (broad s, 57H).

Yield: 75%

*VitE-Gly-PSar(50):*

$^1$H NMR (MeOD): δ = 0.90 (m, 12H, CH-$CH_3$), 1.04-1.63 (broad m, 25H), 1.83 (m, 2H, -$CH_2$-$CH_2$-CCO), 1.98 (s, 3H, $CH_3$-), 2.02 (s, $CH_3$-), 2.09 (s, $CH_3$-), 2.63 (t, 2H, -$CH_2$-$CH_2$-C=C), 2.86-3.15 (broad s, 214H, N-$CH_3$), 3.92-4.55 (broad s, 144H).

Yield: 75%

The obtained product analyzed in terms of Mn distribution and DP by SEC, and $^1$H NMR. Results are shown in table 5 below:

Table 5

| Compound | DP (theorical) | DP by NMR in MeOD[b] | Mn (Da) by GPC[a] | DP by GPC[a] | PDI by SEC[a] | dn/dc |
|---|---|---|---|---|---|---|
| **Tocopherol-Gly-PSar(27)** | 20 | 28 | 2495 | 27 | 1.04 | 0.168 |
| **Tocopherol-Gly-PSar(62)** | 50 | 71 | 4974 | 62 | 1.03 | 0.168 |
| *[a]Absolute Mn was determined by GPC-RI-MALS in MeOH:Guanidinium 85:15, [b]1H NMR in MeOD at 10 mg/mL.* | | | | | | |

CMC determination

The critical micellar concentration of iPrPSar(x)-VitE polymers was determined employing the fluorescence dye Nile Red. Nile Red is a hydrophobic and solvatochromic dye with poor solubility and fluorescence in aqueous media. The excitation-mediated emission is observed when the Nile Red is associated with a hydrophobic domain, so if the concentration of polymer in water exceeds CMC and the polymeric micelles are constituted, Nile Red will emit fluorescence.

A stock solution of Nile Red at the concentration of 500 μM was prepared in acetone. Then 10 μL of the prepared stock solution was added to microcentrifuge tubes, which was allowed to evaporate the acetone in the dark. The 500-μL polymer solutions of various concentrations (range 1x$10^{-4}$ to 2 mg·$mL^{-1}$) were added into those microcentrifuge tubes and

allowed to age overnight.

The analysis of the samples was performed in a JASCO Spectrofluorometer FP-8300 equipped with a Xe lamp. The samples were excited at 550 nm and the emission spectra between 575-740 nm were recorded. The max. emission intensity recorded was plotted against the polymer concentration logarithm. The CMC was determined as the intersection point of the two main lineal curves adjusted plotted, data is added in Table 6.

Table 6

| Polymer | Equations Log(x) vs RFU | R^2 | Intersection (CMC in mg/mL) |
|---|---|---|---|
| iPrPSar6-succ-VitE | y=76.644x + 264.13 | 0.9981 | 0.190 |
| | y= 4055.8x + 6859.4 | 0.9942 | |
| iPrPSar12-succ-VitE | y=27.801x + 93.291 | 0.8785 | 0.237 |
| | y= 2117x + 3098 | 0.9407 | |
| iPrPSar20-succ-VitE | y=29.466x + 116.43 | 0.8332 | 0.250 |
| | y= 1655.2x + 2368.9 | 0.9943 | |

*Solubilizer and encapsulation properties*

Solubilizer properties and encapsulation capability of iPrPSar-VitE polymers were tested and proven employing Carbamazepine (logP= 2.67) as hydrophobic drug through solid suspension encapsulation in aqueous micelle formulation.

iPrPSar(X)-succ-VitE polymers were solubilized in $ddH_2O$ at 10 $mg \cdot mL^{-1}$. Then, solid Carbamazepine was added over aqueous polymer solution in a 15%(w/w) ratio to get a saturated solution. The micellar solution was let to stir 24 h at room temperature avoiding the direct light contact. Afterwards, the solution was centrifuged at 13300 rpm for 2 min. The supernatant was isolated and filtrated by Nylon 0.22 μm. The resultant solution was diluted 100 times in a $MeOH/H_2O$ 1/1 mixture and the carbamazepine content was determined by UV spectroscopy at 285 nm.

Table 7

| Polymer | [Carbamazepine] ($mg \cdot L^{-1}$) in ppm | mg Carbamazepine/g polymer | Encap. Efficiency (%) |
|---|---|---|---|
| iPrPSar(6)-succ-VitE | 586 | 58.92 | 37.5 |
| iPrPSar(12)-succ-VitE | 474 | 49.50 | 29.4 |
| i PrPSar(20)-succ-VitE | 356 | 35.55 | 21.6 |
| TPGS (1kDa) | 356 | 35.63 | 21.8 |

The polymers of the invention showed comparable or even better encapsulation efficiency with respect to TPGS.

*Polymeric micelle formulations*

In order to study the physico-chemical properties such as size, electrostatic charge, diffusion coefficient and morphology, empty and Dil-loaded micelles of polysarcosine- vitamin E derivatives were prepared. Dil dye fluorophore (1,1'-dioctadecyl-3,3,3',3'-tetramethylindocarbocyanine perchlorate, Dil C18, ThermoFisher) was used as a hydrophobic active agent model at 1% in w/w. The micelles prepared from polysarcosine-vitamin E derivatives were compared to TPGS micelles. TPGS as used in these experiments was commercially available (PMC Isochem) and had the following characteristics shown in table 8 below:

Table 8

| Theoretical Mw (kDa) | Mw (kDa) by SEC[a] | PDI by SEC[a] | DP by Mw SEC[a] | DP by NMR in MeOD[b] |
|---|---|---|---|---|
| 1.531 | 1.484 | 1.18 | 22 | 22 |
| [a]*Absolute Mw was determined by SEC-RI-MALS in* DMF *containing LiBr at 0.1%;* [b]$^1$*H NMR in MeOD at 10 mg/mL.* | | | | |

Micelles were formulated by 3 different approaches (direct, thin-film and co-solvent) adapted from Gaucher et al as previously described (Gaucher G. et al., Journal of Controlled Release 2005, 109(1-3), 169-188).

*Direct formulation general methodology*

The block copolymer, iPrPSar(20)-succ-VitE as prepared in example 1 (TPSS) or TPGS, (10 mg/mL) was dissolved in distilled water directly with continuous stirring at r.t. until solution homogenization. The formulation was equilibrated at r.t. for 24 hours. Formulations were equilibrated at r.t. for 24 hours, followed by centrifugation at 1000 rpm for 10 minutes. Both for TPGS and TPSS, no sediment in the centrifugation step was found. The solution was freeze-dried directly obtaining a powder solid. For the preparation of fluorescently labeled micelles, the same procedure was followed with the difference that 1 % w/w of Dil loading was added directly together with the block copolymer in the distilled water.

*Co-solvent formulation general methodology*

The block copolymer, iPrPSar(20)-succ-VitE as prepared in example 1 (TPSS) or TPGS, (100 mg/mL) was dissolved in MeOH and added dropwise into distilled water with continuous stirring at r.t. until the evaporation of the organic solvent. Formulations were equilibrated at r.t. for 24 hours, followed by centrifugation at 1000 rpm for 10 minutes. Both for TPGS and TPSS, no sediment in the centrifugation step was found. The solution was freeze-dried directly obtaining a powder solid. For the preparation of fluorescently labeled micelles, the same procedure was followed with the difference that 1 % w/w of Dil loading was aimed by dissolving the block copolypeptides and the dye in THF/MeOH and following the same procedures described above.

*Thin-film formulation general methodology*

The block copolymer, iPrPSar(20)-succ-VitE as prepared in example 1 (TPSS) or TPGS (100 mg/mL) was dissolved in MeOH in a round bottom flask with the adequate size. Then, MeOH was removed slowly in the rotatory evaporator forming a thin-film layer in the round bottom flask. The product was dissolved in distilled water (10 mg/mL). The solutions were equilibrated at r.t. for 24 hours, followed by centrifugation at 1000 rpm for 10 minutes. Both for TPGS and TPSS, no sediment in the centrifugation step was found. The solution was freeze-dried directly obtaining a powder solid. For the preparation of fluorescently labeled micelles, 1 % w/w of Dil loading was prepared by dissolving the block copolypeptides and the dye in THF/MeOH and following the same procedures described above.

Properties of the empty and Dil-loaded micelles such as size by DLS and DOSY NMR, electrostatic charge by Z potential measurement, diffusion coefficient by DOSY NMR and morphology by TEM were analyzed. Regarding Dil C18 loading and encapsulation efficiency, since no sediments appeared in the centrifugation step, it could be assumed as indirect method that Dil C18 encapsulation was quantitative for micelles prepared by all three different routes.

*Size by DLS and electrostatic charge by Z potential*

The obtained micelles with and without Dil were studied by DLS in terms of size and electrostatic charge at different concentrations. Results are shown in table 9 below:

Table 9

| Micelle | Micelle Formulation Methodology | Conc. mg/mL | Size, I[a] nm | Size, N[b] nm | PDI | Z potential mV |
|---|---|---|---|---|---|---|
| **TPGS** | Direct | 10 | 14.1 | 7.0 | 0.13 | -0.56 |
| | | 1 | 18.3 | 9.1 | 0.27 | -2.08 |
| **TPGS + Dil** | Direct | 10 | 13.6 | 8.6 | 0.13 | - |
| | | 1 | 13.9 | 9.5 | 0.25 | - |
| | Co-Solvent | 10 | 12.1 | 7.3 | 0.03 | - |
| | | 1 | 13.4 | 6.9 | 0.15 | - |
| | Thin-Film | 10 [c] | 13.7/405.5 | 6.8 | 0.46 | - |
| | | 1 [c] | 12.4/315.1 | 7.7 | 0.46 | - |

(continued)

| Micelle | Micelle Formulation | Conc. | Size, I[a] | Size, N[b] | PDI | Z potential |
|---|---|---|---|---|---|---|
| | Methodology | mg/mL | nm | nm | | mV |
| **TPSS** | Direct | 10 | 14.2 | 7.0 | 0.03 | -0.05 |
| | | 1 | 17.8 | 8.9 | 0.14 | -0.50 |
| **TPSS + Dil** | Direct | 10 [c] | 15.5/195.3 | 10.9 | 0.50 | - |
| | | 1[c] | 14.9/154.4 | 10.9 | 0.49 | - |
| | Co-Solvent | 10 | 10.7 | 6.6 | 0.03 | - |
| | | 1 | 12.6 | 7.8 | 0.07 | - |
| | Thin-Film | 10 | 12.6 | 7.8 | 0.03 | - |
| | | 1 | 13.6 | 8.9 | 0.03 | - |

*[a]Hydrodynamic diameter by Intensity. [b]Hydrodynamic diameter by Number. [c]Bimodal size distribution by intensity.*

All micelles were studied at 10 and 1 mg/mL to determine if the concentration can induce aggregates, and this was not observed. Empty micelles were formulated using the direct method since TPGS and TPSS are completely water soluble. Micelle formulation methodologies with or without Dil seem not to affect the hydrodynamic diameters (Dh) by Intensity or Number, since all values are between 6 and 19 nm. Importantly, TPGS+Dil prepared by *thin-film* and TPSS + Dil by the *direct* method resulted in bimodal Dh distributions by Intensity calculations. Regarding the Z potential, the electrostatic charge determined was neutral as expected for TPGS and TPSS at both concentrations studied. Therefore, there is not a significant difference in the micelle Dh of TPGS compared with TPSS.

*Size by Diffusion Coefficient obtained by DOSY NMR*

Diffusion Ordered Spectroscopy by NMR (DOSY NMR) is a powerful tool to study binary systems and hybrid materials. Applying an electromagnetic field gradient the Diffusion Coefficient (DC) of molecules can be calculated (Avram, L. et al., Chemical Society Reviews 2015, 44(2), 586-602, Groves P., Polymer Chemistry 2017, 8(44), 6700-6708). Besides, employing the Stejskal-Tanner equation the profile of intensity decay can be obtained for different moieties in complex molecules. Results for TPGS and TPSS of DC and Dh in $D_2O$ are shown in table 10 below:

Table 10

| *Deuterated Solvent* | *$D_2O$ (10 mg/mL)* | |
|---|---|---|
| ***Product*** | ***DC by DOSY ($m^2/s$)*** | ***Dh (nm) by DOSY*** |
| ***Vit E Succinate*** | - | - |
| ***TPGS*** | *$2.77 \cdot 10^{-11}$* | 14.49 |
| ***TPSS*** | *$2.76 \cdot 10^{-11}$* | 14.54 |

*Values for DC were obtained by NMR software MestReNova. Values for Dh were achieved employing the Stokes-Einstein equation [28].*

Dh values obtained in deuterated water (14.49 nm for TPGS and 14.54 nm for TPSS) are in good agreement with Dh determined by DLS using the intensity method being 14.1 nm for TPGS empty micelle at 10 mg/mL and 14.2 nm for TPSS in the same conditions in good agreement with previously obtained data by different techniques. Concerning face-to-face comparison between both micelles, DC and Dh values are very close between them and there are no appreciable differences in terms of DC and Dh.

*Lipid nanoparticles (LNPs) formulation*

In the following example the oligonucleotides used was a pDNA purchased from PlasmidFactory, with reference PF461 (pCMV-luc)), containing 6233 bp expressing luciferase. Any other olugonucleotide with the characteristics above indicated could be used to carry out the experiments below. The ionizable lipid (6Z,9Z,28Z,31Z)-heptatriacont-6,9,28,31-tetraene-19-yl 4-(dimethylamino)butanoate (DLin-MC3-DMA or simply MC3) was purchased from Nanosoft Polymers. Shielding lipid 1,2-Dimyristoyl-rac-glycero-3-methoxypolyethylene glycol-2000 (DMG-PEG2000) was purchased from Avanti Polar Lipids. Both polymers were used to form benchmark LNPs. Structural lipids used to form LNPs were: 1,2-distearoyl-sn-glycero-3-phosphocholine (DSPC, from Avanti Polar Lipids, 850365P) and Cholesterol (Chol, Sigma-Aldrich, C8667).

A microfluidic HerringboneMixer from Darwing Microfluidics was used for LNPs formulation of Table 11. The reactor presents 2 inlet-channels (one for the DNA in aqueous phase and the other for the lipid mixture in ethanol) and 1 outlet-channel. The specifications of the mixer are the following:

- Slide format: 25 x 75 m
- Channel depth: 0.08 mm
- Channel width: 0.1 to 0.5 mm
- Volume: 3.3 μL
- Volume Mixer: 0.47 μL
- Length Mixer: 28.7 mm
- Material: Glass
- Connectors: 1/4"-28 Fittings

In addition, two programmable pumps control the fluid flow rates of the syringes (NE-1000 Programmable Single Syringe Pump, Syringe Pump, USA). The system accepts infusion rates from 0.73 μL/h (1 mL syringe) to 2100 mL/h (60 mL syringe).

This methodology provides reproducibility to the formation of LNPs as well as the possibility of scaling up the process.

Benchmark LNPs of Table 11 were formulated as follows:

- Lipids were dissolved in ethanol at 50/10/38.5/1.5 molar ratio (MC3/DSPC/Cholesterol/DMG-PEG)
- pDNA was diluted in acetate buffer 25 mM at pH 4
- Amine to phosphate ratio (N/P) was 4
- Flow ratio was 3:1 (ethanol:$H_2O$)
- Total flow was 2 mL/min
- Tubing internal diameter was 0.5 mm

After formulation, LNPs were diluted in 10 mM Phosphate buffer saline (PBS) pH 7.4 and concentrated with centrifugal concentrators (Vivaspin™ 500, Sigma) to remove ethanol. The formulation containing the polymer of the invention was prepared analogously with the difference that a polymer of formula (I) was used instead of DMG-PEG and the lipid ratio was different (shown below Table 11).

Benchmark LNPs of Table 12 were formulated with NanoScaler (KNAUER) as follows:

- Lipids were dissolved in ethanol at 46.3/9.4/42.7/1.6 molar ratio (ALC-0315/DSPC/Cholesterol/ALC-0159).
- mRNA was diluted in acetate buffer 25 mM at pH 4
- Amine to phosphate ratio (N/P) was 6.2
- Flow ratio was 1:3 (ethanol:$H_2O$)
- Total flow rate for LNPs was 4.5 mL/min (3mL/min undiluted LNPs + 1.5mL/min in -line dilution with PBS)

After formulation, LNPs were further diluted in 10 mM Phosphate buffer saline (PBS) pH 7.4 to decrease ethanol concentration and purified with centrifugal concentrators (Vivaspin™ 500, Sigma) to remove ethanol. The formulation containing the polymer of the invention was prepared analogously with the difference that a polymer of formula (I) was used instead of shielding lipid ALC-0159 and the lipid ratio was different (shown below Table 12).

<u>LNPs Characterization</u>

<u>Size and polydispersity</u>

Size and polydispersity (PDI) of the formulated LNPs containing mRNA or pDNA at different N/P ratios (positively-chargeable polymer amine (N = nitrogen) groups to negatively-charged nucleic acid phosphate (P)), different molar ratios and shielding polymers were performed using a Malvern ZetasizerNanoZS instrument, equipped with a 532 nm laser at a fixed scattering angle of 173°. 50μl of the samples were measured using a quartz glass high performance cuvette (Hellma Analytics). Size distribution was measured (diameter, nm) with n ≥ 3 measurements.

Benchmark LNPs containing MC3 as ionizable lipid, DMG-PEG as shielding lipid and pDNA as cargo, were formulated at 50/10/38.5/1.5 molar ratios for MC3/DSPC/Cholesterol/DMG-PEG obtaining the following results:

Table 10

| N | Q total (mL/min) | N/P | PDI | Z-ave (nm) |
|---|---|---|---|---|
| 1 | 2 | 4 | 0.125 | 114.7±1.8 |
| 2 | 2 | 4 | 0.227 | 105.9±0.6 |
| 3 | 2 | 4 | 0.166 | 103.8±1.5 |
| 4 | 2 | 4 | 0.228 | 96.6±0.4 |

The presence of DMG-PEG at 1.5% is enough to stabilize the LNPs obtaining a good size and PDI and reproducible formulations.

Table 11 shows the results obtained of iPrPSar(20)-succ-VitE formulated at molar ratio of 5% containing pDNA as cargo.

Table 11

| Q total (mL/min) | % MC3 | % DSPC | % Chol | % iPrPSar(20)-succ-VitE | N/P | PDI | Z-ave (nm) |
|---|---|---|---|---|---|---|---|
| 2 | 50 | 10 | 35 | 5 | 4 | 0.295 | 122.3 ±1.2 |

Results suggest that at 5% of **iPrPSar(20)-succ-VitE,** LNPs can be obtained showing low PDI and appropriate size.

Benchmark LNPs containing ALC-0315 as ionizable lipid, ALC-0159 as shielding lipid and mRNA as cargo, were formulated at 46.3/9.4/42.7/1.6 molar ratios for ALC-0315/DSPC/Cholesterol/ALC-0159 and the results obtained using iPrPSar(x)-succ-VitE formulated at molar ratio of 5% containing mRNA as cargo. Results are shown in Table 12 below:

Table 12

| LNP | Q total (mL/min) | % AL-C-03-15 | % DSPC | % Chol | Shielding lipid | % Shielding lipid | N/P | PDI | Z-ave (nm) | EE% |
|---|---|---|---|---|---|---|---|---|---|---|
| 1 | 3 | 46.3 | 9.4 | 42.7 | ALC-0159 | 1.6 | 6.2 | 0.16 | 74.7 ±1.4 | >90% |
| 2 | 3 | 46.3 | 9.4 | 39.3 | iPrPSar(10)-succ-VitE | 5 | 6.2 | 0.25 | 167.0 ±4.5 | >80% |
| 3 | 3 | 46.3 | 9.4 | 39.3 | iPrPSar(20)-succ-VitE | 5 | 6.2 | 0.17 | 82.6 ±0.6 | >90% |
| 4 | 3 | 46.3 | 9.4 | 39.3 | iPrPSar(25)-succ-VitE | 5 | 6.2 | 0.17 | 53.2 ±0.8 | >90% |

Results suggest that at 5% of different **iPrPSar(x)-succ-VitE,** LNPs can be obtained showing low PDI and appropriate size and Encapsulation Efficiency (EE%) > 90%.

*Biological evaluation methods*

*Cell Viability*

To carry out the *in vitro* cytotoxicity studies two cell lines were used: human immortalized non-tumorigenic keratinocytes (HaCaT cells), supplied by CLS Cell Lines Service (Germany), and human fibroblasts supplied by Hospital

La Fe (Valencia, Spain). High glucose Dulbecco's Modified Eagle Medium (DMEM Glutamax, Fisher, Spain) was used for keratinocyte culture and DMEM (Gibco, Spain) for fibroblast culture; both were supplemented with 2% penicillin/streptomycin and 50 mL of fetal bovine serum (FBS) in a humidified incubator (Hucoa-Erlöss S.A., Spain) at 5% $CO_2$ and 37 °C.

For the cell viability assays, 50 μL of cells were seeded in 96-well plates at a concentration of 4.000 cells/well for HaCaT cells and 2.000 cells/well for human fibroblasts. After 24 h, 50 μL of each treatment was added, reaching a final volume of 100 μL. Cells were incubated with samples or controls for 72 h before performing MTS assay. For this assay, 20 μL of phenazine methosulfate (PMS) and 3-(4,5-dimethylthiazol-2-yl)-5-(3-carboxymethoxyphenyl)-2-(4-sulfophenyl)-2H-tetrazolium, inner salt (MTS) (1:20) solution were added. After 3 h of incubation, absorbance values were captured at 490 nm using a Victor$^2$Wallac™ plate reader (Perkin Elmer, Spain). The absorbance values were represented as the percentage of cell viability relative to the 100% cell viability of untreated control cells. TPGS and iPrPSar(20)-succ-VitE (TPSS) in the form of micelles prepared by the direct formulation methodology, as well as free Vitamin E were tested. The concentrations of TPGS, iPrPSar(20)-succ-tocopherol (TPSS) and the free Vitamin E were in a range from 0.1 to 2.57 μg/mL equivalents of Vitamin E.

FIG. 1 shows the *in vitro* cytotoxicity studies, in two cell lines: HaCaT cells and human fibroblasts. In the range of concentrations of TPGS, TPSS and the free Vitamin E were in a range from 0.1 to 2.57 μg/mL equivalents of Vitamin E, results show no cytotoxic effect in the cell lines studied for none of the samples and in none of the cell lines tested.

*Skin permeation studies*

To demonstrate the property of the of the invention as skin penetration enhancers skin permeation studies by Franz diffusion cells using human skin were performed. In this case, the penetration capacity of both amphiphilic micelles based on vitamin E derivatives were studied.

Breast skin samples were obtained from healthy women undergoing plastic surgery after written informed consent (kindly donated by Hospital la Fe, Valencia, Spain). Immediately after excision, the subcutaneous fatty tissue was carefully removed using a scalpel. The skin was cut into 4 $cm^2$ pieces, wrapped in aluminum foil and stored at -20 °C until use. To reduce inter-individual variability and afford a better comparison of results, skin from only one donor was used in all experiments. Before starting each experiment, the skin was allowed to equilibrate at room temperature (r.t.).

Permeation studies employed the modified Franz diffusion cells (Logan Instruments Corp., EE. UU) with a diffusional area of 0.95 $cm^2$. The skin was fixed carefully between the donor and the receptor chamber, so that stratum corneum was placed upwards. The receptor chamber was filled with 8 mL of 0.01 M phosphate-buffered saline (PBS) pH = 7.4 and stirred with a magnetic stirring bar at 600 rpm with a thermostated temperature of 37 °C. After an equilibration time of 30 min, 100 μL of the formulations (TPGS-Dil and TPSS-Dil as prepared by direct formulation methodology) using a fixed concentration (10 mg/mL) were introduced into the donor chamber using a standard pipette. The donor chamber was then sealed with Parafilm® and aluminum foil, and the experiments ran for 8 h.

After the permeation study, skin samples were washed twice with 0.1% Phosphate-buffered Saline with Bovine Serum Albumin (PBS-BSA) and kept in 4% paraformaldehyde (PFA) for 24 h at r.t. After that, the samples were washed with 30% sucrose in PBS solution and retained for 24 h at 4°C. Finally, skin samples were washed twice with PBS and preserved in a cryopreservation solution (40% 0.1 M Phosphate Buffer PB, 30% ethylene glycol and 30% glycerol) at 4°C until use.

Finally, the samples were placed in the optimum cutting temperature (OCT) inclusion medium, and slides of 5 μm were generated via cryostat (version CM1850 UV, Leica, Germany) and samples were analyzed by confocal microscopy.

Images were captured with an inverted DM IRE2 microscope equipped with a A-blue 40x oil immersion objective and handled with a TCS SP2 system, equipped with an Acoustic Optical Beam Splitter (AOBS). Dil C18(3) was excited at 549 nm, and DAPI at 405 nm. Images were captured at an 8-bit greyscale and processed with LCS software (version 2.5.1347a, Leica, Germany) containing multicolor, macro and 3D components. Control tissue that follows the same incubation time with MilliQ water was also analyzed to establish the autofluorescence. Dil intensity was quantified five times per sample using Image J software.

Confocal microscopy (not shown) and pixels quantification by ImageJ (FIG. 2) was carried out. It was observed that the Dil accumulation in the epidermis layer was higher when it was applied in the TPSS micelle than TPGS. By pixels quantification using the ImageJ it was observed how both systems are accumulated mainly in the stratum corneum (SC), but the TPSS micelle showed higher accumulation in the epidermis layer than the TPGS Dil micelle. In any case, any Dil was found in the receptor chamber. These preliminary results demonstrate the use of the PSar-based materials for skin delivery applications since the TPSS showed higher accumulation in the deeper layer than the PEGylated material.

*Immunogenicity assay*

Activation of the complement cascade was analyzed by determining SC5b-9 (Protein S-bound terminal complement complex c5b-9) levels using an immunoassay kit MicroVue SC5b-9 Plus. Sera of three healthy individuals

was used for further testing. The concentration employed 0.2 mg/mL VitE equivalents. Hepes Buffered Saline (HBS) was used as a negative control and Zymosan (0.25 mg/mL) as a positive control. All samples were incubated by mixing 1 part polymer suspension with 4 parts human serum for 1 h at 37 °C under constant agitation. Following the incubation samples were diluted 100-fold (700-fold for Zymosan) with manufacturers dilution buffer and the assay was conducted as described in the manufacturers protocol. Table 13 shows the SC5b-9 amounts and standard error mean (SEM):

Table 13

| Polymer | μg SC5b-9/mL | SEM |
|---|---|---|
| i PrPSar(20)-succ-VitE | 2354.8 | 694.26 |
| TPGS (1kDa) | 458.3 | 132.57 |
| Zymosan (+) | 24354.2 | 3524.90 |
| HBS (-) | 1956.0 | 481.70 |

iPrPSar(20)-succ-VitE shows 10x less immunogenic response than the positive control zymosan. And comparable results to negative control HBS. Therefore, iPrPSar(20)-succ-VitE has no immunogenic response in these conditions.

**Citation List**

- Fieber W. et al., NMR diffusion and relaxation studies of the encapsulation of fragrances by amphiphilic multiarm star block copolymers, Macromolecules 2007, 40(15), 5372-5378.
- Duro-Castano A. et al., "Extraordinary" Soft-Assembled ChargE Polypeptides as a Strategy for Nanocarrier Design, Advanced Materials 2017, 29(39), 12.
- Gaucher G., et al., Block copolymer micelles: preparation, characterization and application in drug delivery, Journal of Controlled Release 2005, 109(1-3), 169-188.
- Avram, L. et al., Diffusion NMR of molecular cages and capsules, Chemical Society Reviews 2015, 44(2), 586-602.
- Groves P., Diffusion ordered spectroscopy (DOSY) as applied to polymers, Polymer Chemistry 2017, 8(44), 6700-6708.


**Claims**

1. A polymer comprising a repeating sarcosine-based unit which is covalently attached directly or through a linker to a vitamin E-based moiety, or a pharmaceutically, nutraceutically, or cosmetically acceptable salt thereof, or any stereoisomer or mixtures of stereoisomers, either of the polymer or of any of its salts; wherein the repeating sarcosine-based unit has the formula (XXIII)

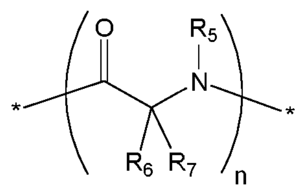

(XXIII)

wherein $R_5$ is selected from the group consisting of methyl, $-CH_2F$, $-CHF_2$, and $-CF_3$; $R_6$ and $R_7$ are independently selected from the group consisting of hydrogen and fluorine; and n is an integer from 5 to 500; and the vitamin E-based moiety has the formula (XXIV):

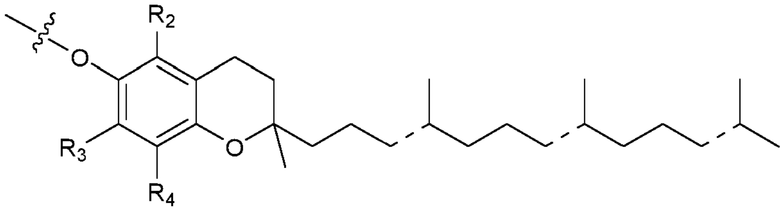

(XXIV)

wherein $R_2$, $R_3$ and $R_4$ are independently selected from the group consisting of hydrogen, fluorine, methyl, - $CH_2F$, $-CHF_2$, and $-CF_3$; and each of the dashed bonds ----- is independently a single bond or, alternatively, a double bond.

**2.** The polymer according to claim 1, which has the formula (I),

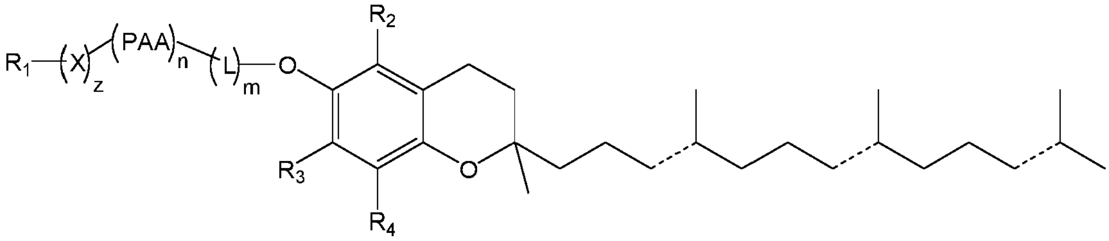

(I)

wherein:

PAA is a repeating sarcosine-based unit selected from formula (XXI) and formula (XXII):

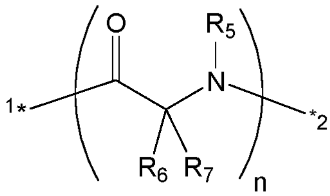

(XXI)

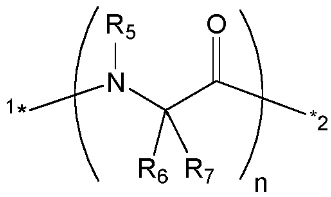

(XXII)

wherein "*1" denotes the attaching point of the repeating sarcosine-based unit PAA to X or $R_1$, and "*2" denotes the attaching point of the repeating sarcosine-based unit PAA to the linker L or the oxygen atom of the vitamin E-based moiety;

m is 0 or 1, with the condition that when the repeating sarcosine-based unit PAA has the formula (XXI), then m is 1;

z is 0 or 1, with the condition that when the repeating sarcosine-based unit PAA has the formula (XXI), then z is 1, and when the repeating sarcosine-based unit PAA has the formula (XXII), then z is 0;

$R_1$ is selected from the group consisting of $-(C_1-C_{12})$alkyl, $-(C_1-C_{12})$alkylphenyl,$-(C_1-C_6)$alkyl-O-$(C_1-C_6)$alkyl, -CO-$(C_1-C_{12})$alkyl, -CO-$(C_1-C_{12})$alkylphenyl, and -CO-$(C_1-C_6)$alkyl-O-$(C_1-C_6)$alkyl; wherein $R_1$ is optionally substituted with one or more substituents selected from the group consisting of halogen, -OH, $-NR_aR_b$, -SH, $-NHNH_2$, $-COOR_c$, $-CF_3$, and $-OCF_3$;

$R_2$, $R_3$ and $R_4$ are independently selected from the group consisting of hydrogen, fluorine, methyl, $-CH_2F$, $-CHF_2$, and $-CF_3$;

$R_5$ is selected from the group consisting of methyl, $-CH_2F$, $-CHF_2$, and $-CF_3$;

$R_6$ and $R_7$ are independently selected from the group consisting of hydrogen and fluorine;

n is an integer from 5 to 500;

X is -O- or -NH-; and

L is a biradical chain which comprises one or more moieties selected from the group consisting of -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -(CO)O-,

$-C(=CH_2)-$, $-C(=NH)-$, -CONH-, -NHCO-, -NH(CO)NH-, -S-, -S-S-, -SO-, $-SO_2-$, $-SO_2NH_2-$ and -phenylene-, wherein L is optionally substituted with one or more substituents selected from the group consisting of halogen, -OH, $-NR_aR_b$, -SH, $-NHNH_2$, $-COOR_c$, $-CF_3$, $-OCF_3$,

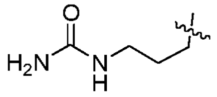 ; 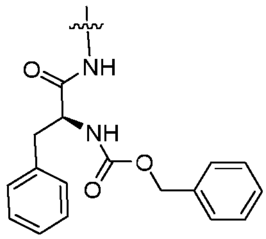 ; 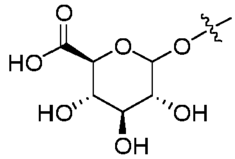

wherein when L comprises a $-CH_2-$ moiety, the two hydrogen atoms attached to the carbon atom are optionally replaced by the ring:

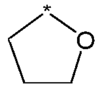

when PAA is a repeating sarcosine-based unit of formula (XXI), L is attached to the nitrogen atom of the PAA by a chemically feasible bond which is selected from the group consisting of an amide bond, a carbamate bond, a urea bond, and an amine bond, and when PAA is a repeating sarcosine-based unit of formula (XXII) and m is 1, L is attached to the carbonyl group of the PAA by a chemically feasible bond which is selected from the group consisting of an amide bond and an ester bond;
L is attached to the oxygen atom of the vitamin E-based moiety by a chemically feasible bond which is selected from the group consisting of an ester bond, a carbamate bond and an ether bond; and
each of the dashed bonds ----- is independently a single bond or, alternatively, a double bond; and
$R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl.

**3.** The polymer according to any of claims 1 to 2, wherein n is an integer from 5 to 50.

**4.** The polymer according to claims 1 to 3, wherein $R_5$ is methyl, and $R_6$ and $R_7$ are hydrogen.

**5.** The polymer according to any of claims 1 to 4, wherein $R_2$, $R_3$ and $R_4$ are methyl, and each of the dashed bonds ----- is a single bond.

**6.** The polymer according to any of claims 2 to 5, wherein the repeating sarcosine-based unit PAA has the formula (XXI), z is 1, and m is 1.

**7.** The polymer according to claim 6, wherein z is 1 and X is -NH-.

**8.** The polymer according to any of claims 2 to 5, wherein the repeating sarcosine-based unit PAA has the formula (XXII), and z is 0.

**9.** A conjugate of formula (II), or a pharmaceutically, nutraceutically, or cosmetically acceptable salt thereof, or any stereoisomer or mixtures of stereoisomers, either of the conjugate of formula (II) or of any of its salts

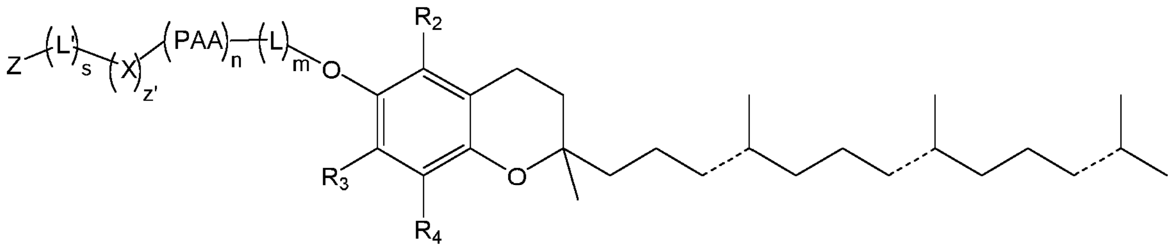

(II)

wherein:

m, PAA, X, n, L, $R_2$, $R_3$, $R_4$, and the dashed bonds are as defined in any of claims 1-8;
s is 0 or 1;
z' is 0 or 1, with the condition that when the repeating sarcosine-based unit PAA has the formula (XXII), then z' is 0;
Z is an agent selected from the group consisting of a nutraceutically active agent, a pharmaceutically active agent, a cell-targeting agent, a diagnostically active agent, and a cosmetically active agent;
L' is a biradical chain which comprises one or more moieties selected from the group consisting of -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -COO-, $-C(=CH_2)-$, -C(=NH)-, -CONH-, -NHCO-, -NH(CO)NH-, -S-, -S-S-, -SO-, $-SO_2-$, $-SO_2NH_2-$ and -phenylene-, wherein L' is optionally substituted with one or more substituents selected from the group consisting of halogen, -OH, $-NR_aR_b$, -SH, $-NHNH_2$, $-COOR_c$, $-CF_3$, $-OCF_3$,

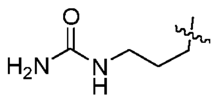 ; 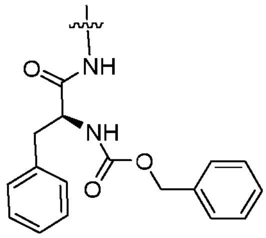 ; 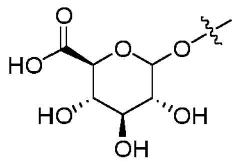

and $R_a$, $R_b$ and $R_c$ are radicals independently selected from the group consisting of H, -phenyl, $-(C_1-C_6)$alkyl, $-(C_2-C_6)$alkenyl, $-(C_1-C_6)$alkylphenyl, and -phenyl$(C_1-C_6)$alkyl; and wherein when L' comprises a $-CH_2-$moiety, the two hydrogen atoms attached to the carbon atom are optionally replaced by the ring:

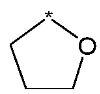

when s is 0, Z is directly attached to X or the PAA sarcosine repeating unit by a chemically feasible bond which is selected from the group consisting of an amide bond, a carbamate bond, an amine bond, a urea bond, an ether bond, and an ester bond;
when s is 1, L' is attached to Z by a chemically feasible bond which is selected from the group consisting of an amide bond, a carbamate bond, an amine bond, a urea bond, an ether bond, and an ester bond, and L' is attached to X or the PAA sarcosine repeating unit by a chemically feasible bond which is selected from the group consisting of an amide bond, a carbamate bond, an amine bond, a urea bond, an ether bond, and an ester bond.

**10.** A self-assembled particle comprising

(a) the polymer as defined in any of the claims 1-8; or alternatively
(b) the conjugate of formula (II) as defined in claim 9; or alternatively
(c) a combination of the polymer as defined in any of the claims 1-8, and the conjugate of formula (II) as defined in claim 9,

and optionally an agent encapsulated within the self-assembled particle which is selected from the group consisting of a nutraceutically active agent, a pharmaceutically active agent, a cell-targeting agent, a diagnostically active agent,

and a cosmetically active agent.

**11.** The self-assembled nanoparticle according to claim 10, which is selected from the group consisting of a micelle, inverted micelle, planar bilayer, crystal nanoparticle, liposome, microbubble and lipid nanoparticle.

**12.** A composition comprising an effective amount of

(a) the polymer as defined in any of the claims 1-8, or alternatively,
(b) the conjugate of formula (II) as defined in claim 9, or alternatively,
(c) a combination of the polymer as defined in any of the claims 1-8 and the conjugate of formula (II) as defined in claim 9, or alternatively,
(d) the self-assembled particles as defined in claim 10,

together with one or more acceptable excipients or carriers.

**13.** The polymer as defined in any of the claims 1-8, or alternatively, the conjugate of formula (II) as defined in claim 9, or alternatively, the self-assembled particle as defined in claim 10, or alternatively, the pharmaceutical composition as defined in claim 12, for use in medicine, wherein the encapsulated agent in the self-assembled particle, when present, is a pharmaceutically active agent or a cell-targeting agent, and in the conjugate of formula (II), when present, Z is a pharmaceutically active agent or a cell-targeting agent..

**14.** The conjugate of formula (II) as defined in claim 9; or alternatively, the self-assembled particle as defined in claim 10, with the condition that when the self-assembled particle comprises the polymer as defined in any of the claims 1-8 in the absence of a conjugate of formula (II), an encapsulated agent is present within the self-assembled particles; or alternatively, the diagnostic composition as defined in claim 12, for use in diagnostics, wherein the encapsulated agent in the self-assembled particles, when present, is a diagnostically active agent, and in the conjugate of formula (II), when present, Z is a diagnostically active agent.

**15.** Use of the polymer as defined in any of the claims 1-8, as a carrier, as a solubilizer, as absorption and permeation enhancer, as emulsifier or as surface stabilizer.

## Patentansprüche

**1.** Ein Polymer umfassend eine sich wiederholende auf Sarcosin basierende Einheit, die direkt oder über einen Linker kovalent an eine auf Vitamin E basierende Einheit gebunden ist, oder ein pharmazeutisch, nutrazeutisch oder kosmetisch akzeptables Salz davon, oder ein beliebiges Stereoisomer oder Mischungen von Stereoisomeren, entweder des Polymers oder eines seiner Salze; wobei die sich wiederholende auf Sarcosin basierende Einheit die Formel (XXIII) hat

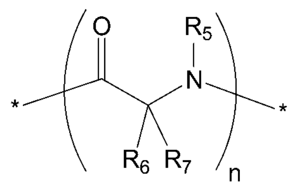

(XXIII)

wobei $R_5$ ausgewählt ist aus der Gruppe bestehend aus Methyl, $-CH_2F$, $-CHF_2$ und $-CF_3$; $R_6$ und $R_7$ unabhängig voneinander ausgewählt sind aus der Gruppe bestehend aus Wasserstoff und Fluor; und n eine ganze Zahl von 5 bis 500 ist; und die auf Vitamin E basierende Einheit die Formel (XXIV) hat:

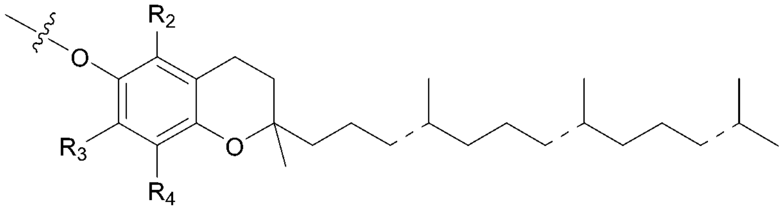

(XXIV)

wobei $R_2$, $R_3$ und $R_4$ unabhängig voneinander aus der Gruppe ausgewählt sind, die aus Wasserstoff, Fluor, Methyl, $-CH_2F$, $-CHF_2$ und $-CF_3$ besteht; und jede der gestrichelten Bindungen ----- unabhängig voneinander eine Einfachbindung oder ersatzweise eine Doppelbindung ist.

**2.** Das Polymer nach Anspruch 1, welches die Formel (I) hat,

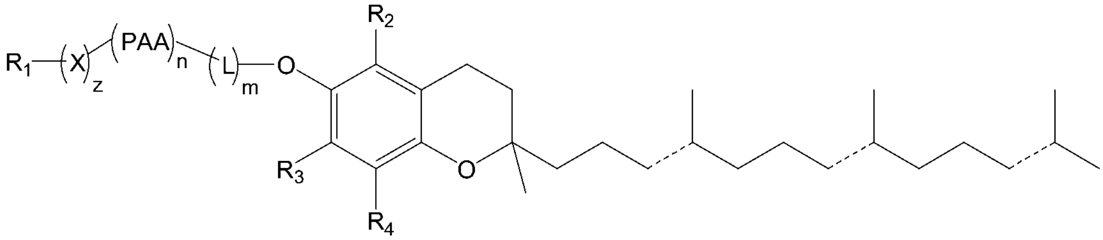

(I)

wobei:

PAA eine sich wiederholende Einheit auf Sarcosin-Basis ist, die ausgewählt ist aus Formel (XXI) und Formel (XXII):

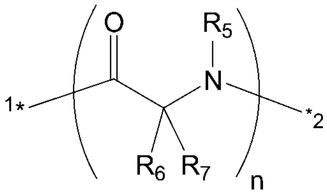

(XXI)

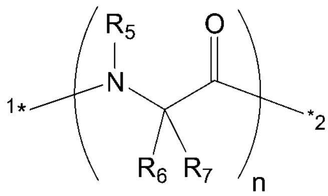

(XXII)

wobei "*1" den Anknüpfungspunkt der sich wiederholenden auf Sarcosin basierenden Einheit PAA an X oder $R_1$ bezeichnet und "*2" den Anknüpfungspunkt der sich wiederholenden auf Sarcosin basierenden Einheit PAA an den Linker L oder das Sauerstoffatom der auf Vitamin E basierenden Einheit bezeichnet;

m = 0 oder 1 ist, mit der Bedingung, dass, wenn die sich wiederholende auf Sarcosin basierende Einheit PAA die Formel (XXI) hat, dann m = 1 ist;

z = 0 oder 1 ist, mit der Bedingung, dass, wenn die sich wiederholende auf Sarcosin basierende Einheit PAA die Formel (XXI) hat, dann z = 1 ist, und wenn die sich wiederholende auf Sarcosin basierende Einheit PAA die Formel (XXII) hat, dann z = 0 ist;

$R_1$ ausgewählt ist aus der Gruppe bestehend aus $-(C_1-C_{12})$Alkyl, $-(C_1-C_{12})$Alkylphenyl, $-(C_1-C_6)$Alkyl-O-$(C_1-C_6)$alkyl, -CO-$(C_1-C_{12})$Alkyl, -CO-$(C_1-C_{12})$Alkylphenyl, und -CO-$(C_1-C_6)$Alkyl-O-$(C_1-C_6)$alkyl; wobei $R_1$ wahlweise mit einem oder mehreren Substituenten substituiert ist, die ausgewählt sind aus der Gruppe bestehend aus Halogen, -OH, $-NR_aR_b$, -SH, $-NHNH_2$, $-COOR_c$, $-CF_3$, und $-OCF_3$;

$R_2$, $R_3$ und $R_4$ unabhängig voneinander aus der Gruppe ausgewählt sind, die aus Wasserstoff, Fluor, Methyl, $-CH_2F$, $-CHF_2$ und $-CF_3$ besteht;

$R_5$ ausgewählt ist aus der Gruppe bestehend aus Methyl, $-CH_2F$, $-CHF_2$ und $-CF_3$;

$R_6$ und $R_7$ unabhängig voneinander ausgewählt sind aus der Gruppe bestehend aus Wasserstoff und Fluor;

n eine ganze Zahl von 5 bis 500 ist;

X = -O- oder -NH- ist; und

L eine biradikalische Kette ist, die eine oder mehrere Einheiten umfasst, die ausgewählt sind aus der Gruppe bestehend aus -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -(CO)O-, $-C(=CH_2)-$, -C(=NH)-, - CONH-, -NHCO-, -NH(CO)NH-, -S-, -S-S-, -SO-, $-SO_2-$, $-SO_2NH_2-$ und -Phenylen-, wobei L wahlweise mit einem oder mehreren Substituenten ausgewählt aus der Gruppe bestehend aus Halogen, -OH, $-NR_aR_b$, -SH, $-NHNH_2$, $-COOR_c$, $-CF_3$, $-OCF_3$ substituiert ist,

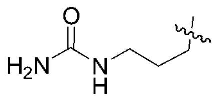 ; 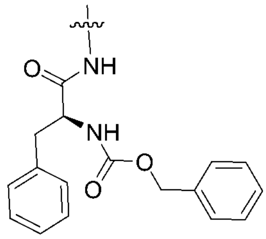 ; 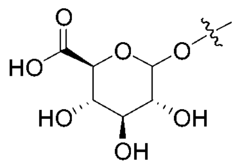

wobei, wenn L eine $-CH_2$-Einheit umfasst, die zwei Wasserstoffatome, die an das Kohlenstoffatom gebunden sind, wahlweise durch den Ring ersetzt sind:

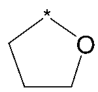

wenn PAA eine sich wiederholende Sarcosin basierende Einheit der Formel (XXI) ist, L an das Stickstoffatom der PAA durch eine chemisch plausible Bindung gebunden ist, welche aus der Gruppe ausgewählt ist, die aus einer Amidbindung, einer Carbamatbindung, einer Harnstoffbindung und einer Aminbindung besteht, und wenn PAA eine sich wiederholende Sarcosin basierende Einheit der Formel (XXII) ist und m = 1 ist, L an die Carbonylgruppe der PAA durch eine chemisch plausible Bindung gebunden ist, welche aus der Gruppe ausgewählt ist, die aus einer Amidbindung und einer Esterbindung besteht;

L durch eine chemisch plausible Bindung an das Sauerstoffatom der auf Vitamin E basierenden Einheit gebunden ist, welche aus der Gruppe ausgewählt ist, die aus einer Esterbindung, einer Carbamatbindung und einer Etherbindung besteht; und

jede der gestrichelten Bindungen ----- unabhängig voneinander eine Einfachbindung oder ersatzweise eine Doppelbindung ist; und

$R_a$, $R_b$ und $R_c$ unabhängig voneinander Reste sind, die ausgewählt sind aus der Gruppe bestehend aus H, -Phenyl, $-(C_1-C_6)$-Alkyl, $-(C_2-C_6)$-Alkenyl, $-(C_1-C_6)$-Alkylphenyl, und -Phenyl$(C_1-C_6)$alkyl.

**3.** Das Polymer nach einem der Ansprüche 1 bis 2, wobei n eine ganze Zahl von 5 bis 50 ist.

**4.** Das Polymer nach den Ansprüchen 1 bis 3, wobei $R_5$ Methyl ist und $R_6$ und $R_7$ Wasserstoff sind.

**5.** Das Polymer nach einem der Ansprüche 1 bis 4, wobei $R_2$, $R_3$ und $R_4$ Methyl sind und jede der gestrichelten Bindungen ----- eine Einfachbindung ist.

**6.** Das Polymer nach einem der Ansprüche 2 bis 5, wobei die sich wiederholende auf Sarcosin basierende Einheit PAA die Formel (XXI) hat, z = 1 und m = 1 ist.

**7.** Das Polymer nach Anspruch 6, wobei z = 1 und X = -NH- ist.

**8.** Das Polymer nach einem der Ansprüche 2 bis 5, wobei die sich wiederholende auf Sarcosin basierende Einheit PAA die Formel (XXII) hat und z = 0 ist.

**9.** Ein Konjugat der Formel (II), oder ein pharmazeutisch, nutrazeutisch oder kosmetisch akzeptables Salz davon, oder ein Stereoisomer oder Mischungen von Stereoisomeren, entweder des Konjugats der Formel (II) oder eines seiner Salze

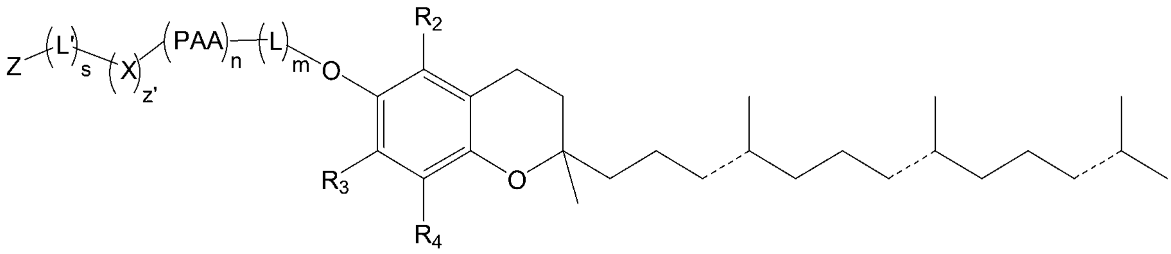

(II)

wobei:

m, PAA, X, n, L, $R_2$, $R_3$, $R_4$ und die gestrichelten Bindungen wie in einem der Ansprüche 1 bis 8 definiert sind;
s = 0 oder 1 ist;
z' = 0 oder 1 ist, mit der Bedingung, dass, wenn die sich wiederholende auf Sarcosin basierende Einheit PAA die Formel (XXII) hat, dann z' = 0 ist;
Z ein Stoff ist, das ausgewählt ist aus der Gruppe bestehend aus einem nutrazeutisch wirksamen Stoff, einem pharmazeutisch wirksamen Stoff, einem zellzielenden Stoff, einem diagnostisch wirksamen Stoff und einem kosmetisch wirksamen Stoff;
L' eine biradikalische Kette ist, die eine oder mehrere Einheiten umfasst, die ausgewählt sind aus der Gruppe bestehend aus -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -COO-, $-C(=CH_2)-$, -C(=NH)-, - CONH-, -NHCO-, -NH(CO)NH-, -S-, -S-S-, -SO-, $-SO_2-$, $-SO_2NH_2-$ und -Phenylen-, wobei L' wahlweise mit einem oder mehreren Substituenten ausgewählt aus der Gruppe bestehend aus Halogen, -OH, $-NR_aR_b$, -SH, $-NHNH_2$, $-COOR_c$, $-CF_3$, $-OCF_3$ substituiert ist,

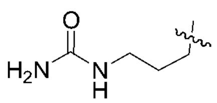 ; 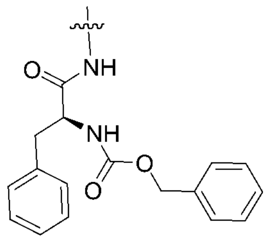 ; 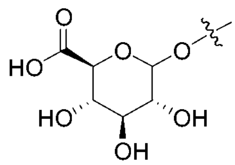

und $R_a$, $R_b$ und $R_c$ Reste sind, die unabhängig voneinander ausgewählt sind aus der Gruppe bestehend aus H, -Phenyl, $-(C_1-C_6)$Alkyl, $-(C_2-C_6)$Alkenyl, $-(C_1-C_6)$Alkylphenyl, und -Phenyl$(C_1-C_6)$alkyl; und wobei, wenn L' eine $-CH_2-$Einheit umfasst, die beiden an das Kohlenstoffatom gebundenen Wasserstoffatome wahlweise durch den Ring ersetzt sind:

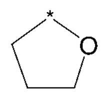

wenn s = 0 ist, Z direkt an X oder die sich wiederholende Sarcosin-Einheit PAA durch eine chemisch plausible Bindung gebunden ist, die ausgewählt ist aus der Gruppe bestehend aus einer Amidbindung, einer Carbamatbindung, einer Aminbindung, einer Harnstoffbindung, einer Etherbindung und einer Esterbindung;
wenn s = 1 ist, L' an Z durch eine chemisch plausible Bindung gebunden ist, die aus der Gruppe ausgewählt ist, die aus einer Amidbindung, einer Carbamatbindung, einer Aminbindung, einer Harnstoffbindung, einer Etherbindung und einer Esterbindung besteht, und L' an X oder die sich wiederholende Sarcosin-Einheit PAA durch eine chemisch plausible Bindung gebunden ist, die aus der Gruppe ausgewählt ist, die aus einer Amidbindung, einer Carbamatbindung, einer Aminbindung, einer Harnstoffbindung, einer Etherbindung und einer Esterbindung besteht.

**10.** Ein selbstorganisierendes Partikel, umfassend

(a) das Polymer wie in einem der Ansprüche 1 bis 8 definiert; oder ersatzweise
(b) das Konjugat der Formel (II) wie in Anspruch 9 definiert; oder ersatzweise

(c) eine Kombination des Polymers wie in einem der Ansprüche 1 bis 8 definiert, und des Konjugats der Formel (II) wie in Anspruch 9 definiert,

und wahlweise einen in dem selbstorganisierenden Partikel eingekapselten Stoff, der ausgewählt ist aus der Gruppe bestehend aus einem nutrazeutisch wirksamen Stoff, einem pharmazeutisch wirksamen Stoff, einem zellzielenden Stoff, einem diagnostisch wirksamen Stoff und einem kosmetisch wirksamen Stoff.

**11.** Das selbstorganisierende Nanopartikel nach Anspruch 10, das ausgewählt ist aus der Gruppe bestehend aus einer Mizelle, einer inversen Mizelle, einer planaren Doppelschicht, einem Kristallnanopartikel, einem Liposom, einer Mikroblase und einem Lipidnanopartikel.

**12.** Eine Zusammensetzung umfassend eine wirksame Menge von

(a) dem Polymer wie in einem der Ansprüche 1 bis 8 definiert, oder ersatzweise,
(b) dem Konjugat der Formel (II) wie in Anspruch 9 definiert, oder ersatzweise,
(c) einer Kombination des Polymers wie in einem der Ansprüche 1 bis 8 definiert und des Konjugats der Formel (II) wie in Anspruch 9 definiert, oder ersatzweise,
(d) dem selbstorganisierenden Partikel wie in Anspruch 10 definiert,

zusammen mit einem oder mehreren akzeptablen Hilfsstoffen oder Trägersubstanzen.

**13.** Das Polymer wie in einem der Ansprüche 1 bis 8 definiert, oder ersatzweise das Konjugat der Formel (II) wie in Anspruch 9 definiert, oder ersatzweise das selbstorganisierende Partikel wie in Anspruch 10 definiert, oder ersatzweise die pharmazeutische Zusammensetzung wie in Anspruch 12 definiert, zur Verwendung in der Medizin, wobei der eingekapselte Stoff in dem selbstorganisierenden Partikel, falls vorhanden, ein pharmazeutisch wirksamer Stoff oder ein zellzielender Stoff ist, und in dem Konjugat der Formel (II), falls vorhanden, Z ein pharmazeutisch wirksame Stoff oder ein zellzielender Stoff ist.

**14.** Das Konjugat der Formel (II) wie in Anspruch 9 definiert; oder ersatzweise das selbstorganisierende Partikel wie in Anspruch 10 definiert, mit der Bedingung, dass, wenn das selbstorganisierende Partikel das Polymer wie in einem der Ansprüche 1 bis 8 definiert in Abwesenheit eines Konjugats der Formel (II) umfasst, ein eingekapselter Stoff in den selbstorganisierenden Partikeln vorhanden ist; oder ersatzweise die diagnostische Zusammensetzung wie in Anspruch 12 definiert zur Verwendung in der Diagnostik, wobei der eingekapselte Stoff in den selbstorganisierenden Partikeln, falls vorhanden, ein diagnostisch aktiver Stoff ist, und in dem Konjugat der Formel (II), falls vorhanden, Z ein diagnostisch aktiver Stoff ist.

**15.** Verwendung des Polymers wie in einem der Ansprüche 1 bis 8 definiert, als Träger, als Lösungsvermittler, als Absorptions- und Permeationsverstärker, als Emulgator oder als Oberflächenstabilisator.

## Revendications

**1.** Un polymère comprenant un motif de répétition à base de sarcosine qui est lié par covalence directement ou par l'intermédiaire d'un lieur à un fragment à base de vitamine E, ou un sel pharmaceutiquement, nutraceutiquement ou cosmétiquement acceptable de celui-ci, ou tout stéréoisomère ou mélange de stéréoisomères, soit du polymère, soit de l'un de ses sels ; dans lequel le motif de répétition à base de sarcosine a la formule (XXIII)

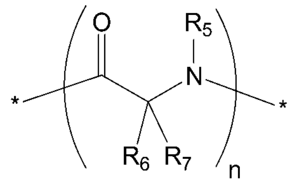

(XXIII)

dans laquelle $R_5$ est choisi dans le groupe constitué par méthyle, $-CH_2F$, $-CHF_2$ et $-CF_3$ ; $R_6$ et $R_7$ sont choisis indépendamment dans le groupe constitué par hydrogène et fluor ; et n est un nombre entier allant de 5 à 500 ; et le

fragment à base de vitamine E a la formule (XXIV) :

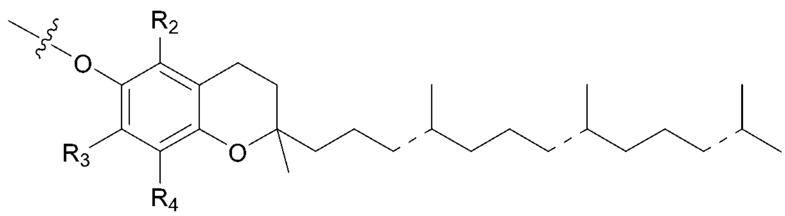

(XXIV)

dans lequel $R_2$, $R_3$ et $R_4$ sont indépendamment choisis dans le groupe constitué par l'hydrogène, le fluor, le méthyle, $-CH_2F$, $-CHF_2$ et $-CF_3$ ; et chacune des liaisons en pointillés ----- est indépendamment une liaison simple ou, en variante, une liaison double.

**2.** Le polymère selon la revendication 1, qui a la formule (I),

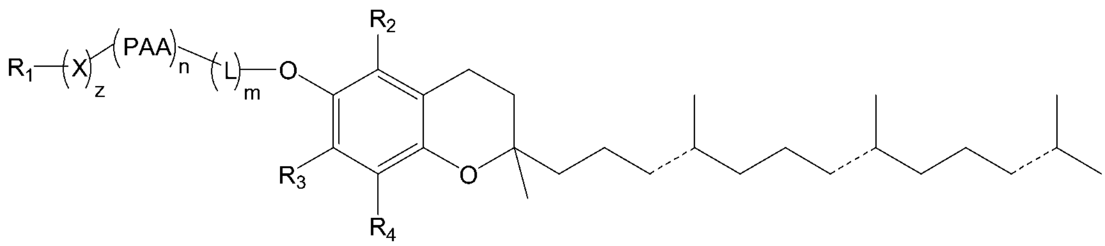

(I)

dans laquelle :

PAA est un motif de répétition à base de sarcosine choisi parmi la formule (XXI) et la formule (XXII) :

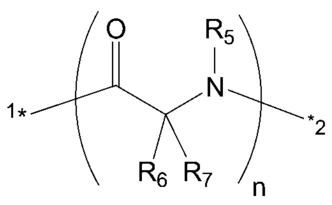

(XXI)

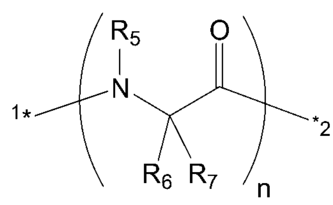

(XXII)

dans lequel « $*^1$ » désigne le point de fixation du motif de répétition à base de sarcosine PAA à X ou $R_1$, et « $*^2$ » désigne le point de fixation du motif de répétition à base de sarcosine PAA au lieur L ou à l'atome d'oxygène du fragment à base de vitamine E ;
m est 0 ou 1, à la condition que lorsque le motif de répétition à base de sarcosine PAA a la formule (XXI), alors m est 1 ;
z est 0 ou 1, à la condition que lorsque le motif de répétition à base de sarcosine PAA a la formule (XXI), alors z est 1, et lorsque le motif de répétition à base de sarcosine PAA a la formule (XXII), alors z est 0 ;
$R_4$ est choisi dans le groupe constitué par alkyle en $-(C_1-C_{12})$, alkylphényle en $-(C_1-C_{12})$, alkyle en $-(C_1-C_6)-O-(C_1-C_6)$alkyle, alkyle en $-CO-(C_1-C_{12})$, alkylphényle en $-CO-(C_1-C_{12})$ et alkyle en $-CO-(C_1-C_6)-O-(C_1-C_6)$alkyle ; où $R_1$ est facultativement substitué par un ou plusieurs substituants choisis dans le groupe constitué par halogène, -OH, $-NR_aR_b$, -SH, $-NHNH_2$, $-COOR_c$, $-CF_3$, et $-OCF_3$;
$R_2$, $R_3$ et $R_4$ sont indépendamment choisis dans le groupe constitué par l'hydrogène, le fluor, le méthyle, $-CH_2F$, $-CHF_2$ et $-CF_3$ ;
$R_5$ est choisi dans le groupe constitué par méthyle, $-CH_2F$, $-CHF_2$ et $-CF_3$ ;
$R_6$ et $R_7$ sont indépendamment choisis dans le groupe constitué par l'hydrogène et le fluor ; n est un nombre entier

allant de 5 à 500 ;

X est -O- ou -NH- ; et

L est une chaîne biradicalaire qui comprend une ou plusieurs fractions choisies dans le groupe constitué par -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -(CO)O-, $-C(=CH_2)-$, -C(=NH)-, -CONH-, - NHCO-, -NH(CO)NH-, -S-, -S-S-, -SO-, $-SO_2-$, $-SO_2NH_2-$ et -phénylène-, où L est facultativement substitué par un ou plusieurs substituants choisis dans le groupe constitué par halogène, -OH, $-NR_aR_b$, -SH, $-NHNH_2$, $-COOR_c$, $-CF_3$, $-OCF_3$,

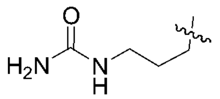 ; 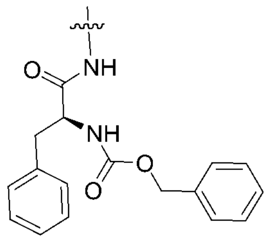 ; 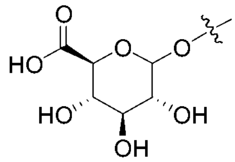

dans lequel lorsque L comprend un fragment $-CH_2-$, les deux atomes d'hydrogène attachés à l'atome de carbone sont facultativement remplacés par le cycle :

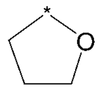

lorsque le PAA est un motif de répétition à base de sarcosine de formule (XXI), L est attaché à l'atome d'azote du PAA par une liaison chimiquement réalisable qui est choisie dans le groupe constitué par une liaison amide, une liaison carbamate, une liaison urée et une liaison amine, et lorsque le PAA est un motif de répétition à base de sarcosine de formule (XXII) et que m est égal à 1, L est attaché au groupe carbonyle du PAA par une liaison chimiquement réalisable qui est choisie dans le groupe constitué par une liaison amide et une liaison ester ;

L est lié à l'atome d'oxygène du fragment à base de vitamine E par une liaison chimiquement réalisable qui est choisie dans le groupe constitué par une liaison ester, une liaison carbamate et une liaison éther ; et

chacune des liaisons en pointillés ----- est indépendamment une liaison simple ou, en variante, une liaison double ; et

$R_a$, $R_b$ et $R_c$ sont des radicaux choisis indépendamment dans le groupe constitué par H, -phényle, -alkyle en $(C_1-C_6)$, alcényle en $-(C_2-C_6)$, alkylphényle en $-(C_1-C_6)$ et -phénylalkyle en $(C_1-C_6)$.

**3.** Le polymère selon l'une quelconque des revendications 1 à 2, dans lequel n est un nombre entier allant de 5 à 50.

**4.** Le polymère selon les revendications 1 à 3, dans lequel $R_5$ est le méthyle, et $R_6$ et $R_7$ sont l'hydrogène.

**5.** Le polymère selon l'une quelconque des revendications 1 à 4, dans lequel $R_2$, $R_3$ et $R_4$ sont des groupes méthyle, et chacune des liaisons en pointillés ----- est une liaison simple.

**6.** Le polymère selon l'une quelconque des revendications 2 à 5, dans lequel le motif de répétition à base de sarcosine PAA a la formule (XXI), z est 1 et m est 1.

**7.** Le polymère selon la revendication 6, dans lequel z est 1 et X est -NH-.

**8.** Le polymère selon l'une quelconque des revendications 2 à 5, dans lequel le motif de répétition à base de sarcosine PAA a la formule (XXII), et z est 0.

**9.** Un conjugué de formule (II), ou un sel pharmaceutiquement, nutraceutiquement ou cosmétiquement acceptable de celui-ci, ou tout stéréoisomère ou mélange de stéréoisomères, soit du conjugué de formule (II), soit de l'un de ses sels

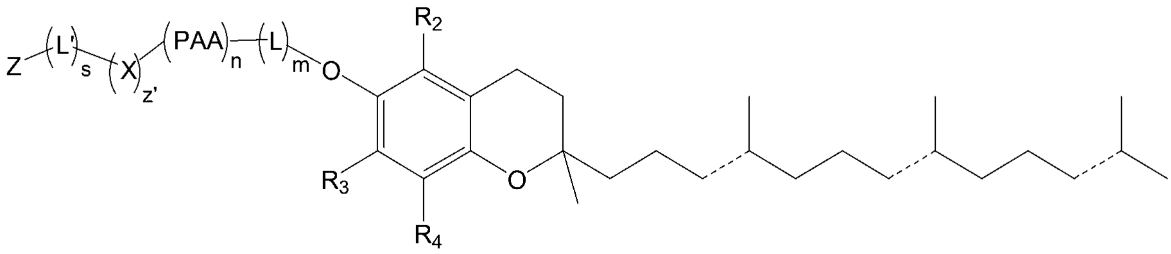

(II)

dans laquelle :

m, PAA, X, n, L, $R_2$, $R_3$, $R_4$ et les liaisons en pointillés sont tels que définis dans l'une quelconque des revendications 1 à 8 ;
s est 0 ou 1 ;
z' est 0 ou 1, à la condition que lorsque le motif de répétition à base de sarcosine PAA a la formule (XXII), alors z' est 0 ;
Z est un agent choisi dans le groupe constitué par un agent nutraceutiquement actif, un agent pharmaceutiquement actif, un agent de ciblage cellulaire, un agent diagnostiquement actif et un agent cosmétiquement actif ;
L' est une chaîne biradicalaire qui comprend un ou plusieurs fragments choisis dans le groupe constitué par -CH=CH-, -C=C-, $-CH_2-$, -N=CH-, -CH=NH-, -NH-, -NH-NH-, -NH-N=CH-, -O-, $-O(CH_2)O-$, -CO-, -O(CO)-, -COO-,
$-C(=CH_2)-$, -C(=NH)-, -CONH-, -NHCO-, -NH(CO)NH-, -S-, -S-S-, -SO-, $-SO_2-$, $-SO_2NH_2-$ et -phénylène-, où L' est facultativement substitué par un ou plusieurs substituants choisis dans le groupe constitué par
halogène, -OH, $-NR_aR_b$, -SH, $-NHNH_2$, $-COOR_c$, $-CF_3$, $-OCF_3$,

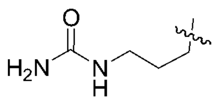 ; 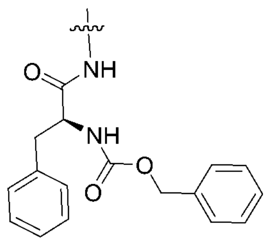 ; 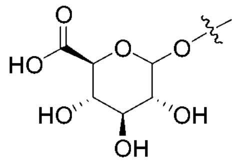

et $R_a$, $R_b$ et $R_c$ sont des radicaux choisis indépendamment dans le groupe constitué par H, -phényle, alkyle en $-(C_1-C_6)$, alcényle en $-(C_2-C_6)$, alkylphenyle en $-(C_1-C_6)$,
et -phénylalkyle en $(C_1-C_6)$ ; et dans lequel lorsque L' comprend un fragment $-CH_2-$, les deux atomes d'hydrogène attachés à l'atome de carbone sont facultativement remplacés par le cycle :

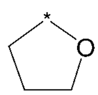

lorsque s est égal à 0, Z est directement attaché à X ou au motif de répétition de sarcosine PAA par une liaison chimiquement réalisable qui est choisie dans le groupe constitué par une liaison amide, une liaison carbamate, une liaison amine, une liaison urée, une liaison éther et une liaison ester ;
lorsque s est 1, L' est attaché à Z par une liaison chimiquement réalisable qui est choisie dans le groupe constitué par une liaison amide, une liaison carbamate, une liaison amine, une liaison urée, une liaison éther et une liaison ester, et L' est attaché à X ou au motif de répétition de sarcosine PAA par une liaison chimiquement réalisable qui est choisie dans le groupe constitué par une liaison amide, une liaison carbamate, une liaison amine, une liaison urée, une liaison éther et une liaison ester.

**10.** Une particule auto-assemblée comprenant

(a) le polymère tel que défini dans l'une quelconque des revendications 1 à 8 ; ou en variante
(b) le conjugué de formule (II) tel que défini dans la revendication 9 ; ou en variante

(c) une combinaison du polymère tel que défini dans l'une quelconque des revendications 1 à 8, et du conjugué de formule (II) tel que défini dans la revendication 9,

et facultativement un agent encapsulé dans la particule auto-assemblée qui est choisi dans le groupe constitué par un agent nutraceutiquement actif, un agent pharmaceutiquement actif, un agent de ciblage cellulaire, un agent diagnostiquement actif et un agent cosmétiquement actif.

**11.** La nanoparticule auto-assemblée selon la revendication 10, qui est choisie dans le groupe constitué par une micelle, une micelle inversée, une bicouche plane, une nanoparticule cristalline, un liposome, une microbulle et une nanoparticule lipidique.

**12.** Une composition comprenant une quantité efficace

(a) du polymère tel que défini dans l'une quelconque des revendications 1 à 8, ou en variante,
(b) du conjugué de formule (II) tel que défini dans la revendication 9, ou en variante,
(c) d'une combinaison du polymère tel que défini dans l'une quelconque des revendications 1 à 8 et du conjugué de formule (II) tel que défini dans la revendication 9, ou en variante,
(d) des particules auto-assemblées telles que définies dans la revendication 10,

avec un ou plusieurs excipients ou véhicules acceptables.

**13.** Le polymère tel que défini dans l'une quelconque des revendications 1 à 8, ou en variante, le conjugué de formule (II) tel que défini dans la revendication 9, ou en variante, la particule auto-assemblée telle que définie dans la revendication 10, ou en variante, la composition pharmaceutique telle que définie dans la revendication 12, pour l'utilisation en médecine, où l'agent encapsulé dans la particule auto-assemblée, lorsqu'il est présent, est un agent pharmaceutiquement actif ou un agent de ciblage cellulaire, et dans le conjugué de formule (II), lorsqu'il est présent, Z est un agent pharmaceutiquement actif ou un agent de ciblage cellulaire.

**14.** Le conjugué de formule (II) tel que défini dans la revendication 9 ; ou en variante, la particule auto-assemblée telle que définie dans la revendication 10, avec la condition que lorsque la particule auto-assemblée comprend le polymère tel que défini dans l'une quelconque des revendications 1 à 8 en l'absence d'un conjugué de formule (II), un agent encapsulé est présent dans les particules auto-assemblées ; ou en variante, la composition de diagnostic telle que définie dans la revendication 12, pour l'utilisation dans des diagnostics, où l'agent encapsulé dans les particules auto-assemblées, lorsqu'il est présent, est un agent diagnostiquement actif, et dans le conjugué de formule (II), lorsqu'il est présent, Z est un agent diagnostiquement actif.

**15.** Utilisation du polymère tel que défini dans l'une quelconque des revendications 1 à 8, en tant que véhicule, en tant qu'agent solubilisant, en tant qu'amplificateur de l'absorption et de la perméation, en tant qu'émulsifiant ou en tant que stabilisant de surface.

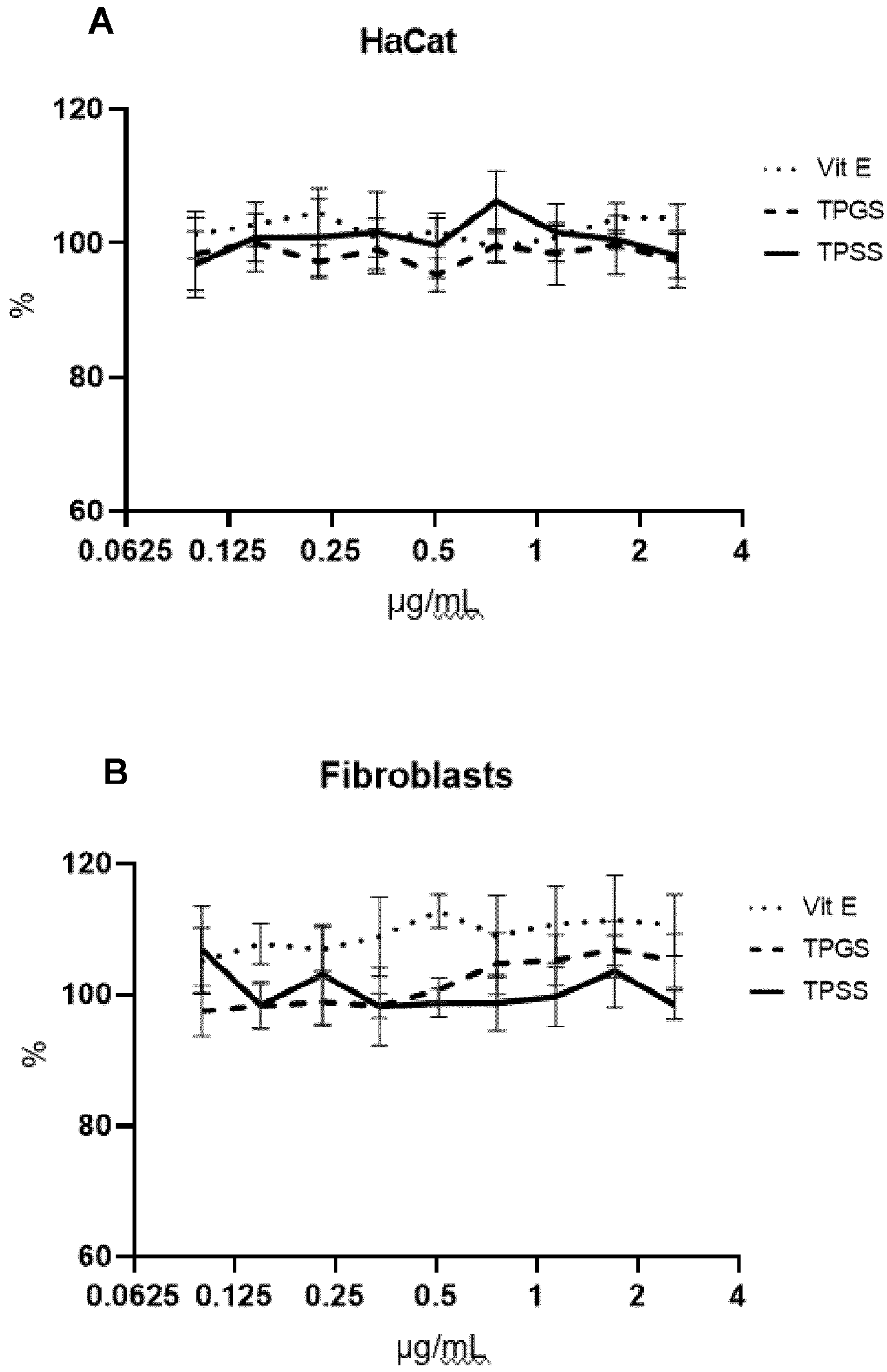
A
HaCat
120
100
80
60
%
0.0625
0.125
0.25
0.5
1
2
4
µg/mL
Vit E
TPGS
TPSS
B
Fibroblasts
120
100
80
60
%
0.0625
0.125
0.25
0.5
1
2
4
µg/mL
Vit E
TPGS
TPSS

FIG. 1

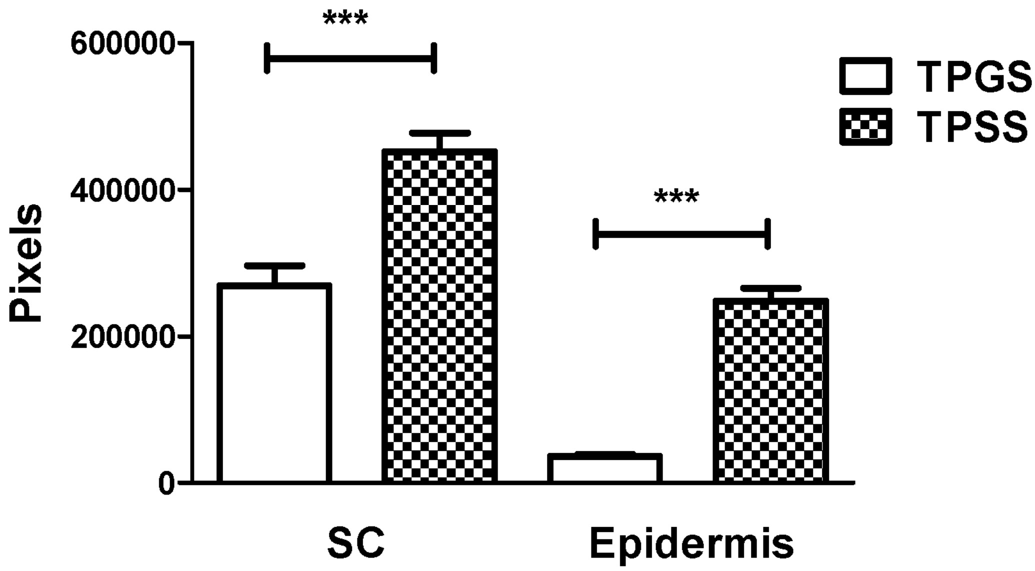


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 21383149


### Non-patent literature cited in the description


- **BIRKE et al.** *Progress in Polymer Science*, 2018, vol. 81, 163-208
- **FIEBER W. et al.** *Macromolecules*, 2007, vol. 40 (15), 5372-5378
- **DURO-CASTANO A. et al.** *Advanced Materials*, 2017, vol. 29 (39), 12
- **GAUCHER G. et al.** *Journal of Controlled Release*, 2005, vol. 109 (1-3), 169-188
- **AVRAM, L. et al.** *Chemical Society Reviews*, 2015, vol. 44 (2), 586-602
- **GROVES P.** *Polymer Chemistry*, 2017, vol. 8 (44), 6700-6708
- **FIEBER W. et al.** NMR diffusion and relaxation studies of the encapsulation of fragrances by amphiphilic multiarm star block copolymers. *Macromolecules*, 2007, vol. 40 (15), 5372-5378
- **DURO-CASTANO A. et al.** ''Extraordinary'' Soft-Assembled ChargE Polypeptides as a Strategy for Nanocarrier Design. *Advanced Materials*, 2017, vol. 29 (39), 12
- **GAUCHER G. et al.** Block copolymer micelles: preparation, characterization and application in drug delivery. *Journal of Controlled Release*, 2005, vol. 109 (1-3), 169-188
- **AVRAM, L. et al.** Diffusion NMR of molecular cages and capsules. *Chemical Society Reviews*, 2015, vol. 44 (2), 586-602
- **GROVES P.** Diffusion ordered spectroscopy (DOSY) as applied to polymers. *Polymer Chemistry*, 2017, vol. 8 (44), 6700-6708